# EUROPEAN PATENT APPLICATION

(11) **EP 1 790 661 A2**
(43) Date of publication of application: **30.05.2007**
(21) Application number: 06256018.0
(22) Date of filing: 24.11.2006
(51) Int. Cl.: C07K 14/395, C12N 15/81, C12N 5/10, C12N 1/15, C12N 1/21

(54) **A highly efficient secretory signal peptide and a protein expression system using the peptide thereof**

(30) Priority: 24.11.2005 JP 2005339383; 01.11.2006 JP 2006297923
(71) Applicant: National Institute of Advanced Industrial Science and Technology, Tokyo 100-8921 (JP)
(72) Inventor: Sahara, Takehiko, c/o Hokkaido Center, National Institute of, Sapporo-shi, Hokkaido 062-8517 (JP); Gouda, Takako, c/o Hokkaido Center, National Institute of, Sapporo-shi, Hokkaido 062-8517 (JP); Tochigi, Yuki, c/o Hokkaido Center, National Institute of, Sapporo-shi, Hokkaido 062-8517 (JP); Ohgiya, Satoru, c/o Hokkaido Center, National Institute of, Sapporo-shi, Hokkaido 062-8517 (JP)
(74) Representative: Maschio, Antonio

(57) **Abstract**

This invention is directed to the identification of secretory signal peptides exhibiting higher secretion efficiency than conventional secretory signal peptides. Secretory signal peptides exhibiting higher secretion ability than the secretory signal peptides used in conventional membrane and secretory protein expression systems are identified and isolated from membrane proteins and secretory proteins existing in the Saccharomyces cerevisiae genome.

## Description

### Technical Field

The present invention relates to secretory signal peptides exhibiting higher secretion efficiency than- conventional secretory signal peptides-, for example.

### Background Art

Up to the present, a variety of proteins have been expressed and produced with the use of a variety of hosts, such as E. coli, yeast, insect cell, or animal cell. Among such protein production systems, the E. coli host is one of the most commonly used hosts. When proteins derived from animals such as humans are produced in E. coli hosts, however, these proteins are often insolubilized, and- such insolubilized proteins are expressed as denatured proteins or cannot be expressed in some cases.

In contrast, protein production systems involving the use- of cultured cell hosts, such as insect or animal cell hosts, are capable of producing proteins derived from animals such as humans as properly folded proteins with high probability. These protein production systems utilizing cultured cell hosts, however, suffer from the following drawbacks: (1) high culture cost; (2) slow growth; and (3) difficulty of enlarging the culture scale.

When yeast, i.e., a unicellular eukaryote, is used as a host cell of a protein production system, proteins derived from animals such as humans can be expressed as properly folded proteins with relatively high probability, as with cases involving the use of cultured cells. As with cases of microorganisms such as E. coli, culture utilizing a yeast host can be carried out in a less expensive manner, in terms of culture equipment, culture duration, and medium cost, than culture utilizing cultured cells. Since budding yeast, Saccharomyces cerevisiae, is used in the production of alcohol beverages such as beer or wine or fermented foods such as bread, the safety of yeast as an organism has been confirmed. Also, sequencing of the yeast genome was completed earlier than that of other eukaryotes, and yeast has an excellent molecular biological background, i.e., well-organized genomic information is available-.

Thus, yeast had been extensively utilized as a host for production system of endogenous/exogenous proteins, with the utilization of recombinant DNA techniques.

As described above, expression and production of a variety of proteins, including human-derived proteins, have been heretofore attempted. Compared with the intracellular expression of soluble proteins, it is generally known that expression of membrane proteins and secretory proteins is difficult. This is because many membrane proteins and secretory proteins are required to mature as functional proteins by various types of modifications in a protein intracellular transportation system through endoplasmic reticulum/Golgi bodies from -cytoplasm.

In the research for development of new drugs, functional/structural analysis of human membrane proteins and secretory proteins is important. Such analysis requires highly efficient expression systems for membrane proteins and secretory proteins. In the case of the extracellular expression of secretory proteins, purification of expressed proteins can be carried out more easily than in the case of intracellular proteins, and degradation caused by intracellular proteases can be inhibited. Because of such advantages, extracellular expression of secretory proteins is regarded important in the industrial production of proteins. In addition to the aforementioned advantages, yeast advantageously has an intracellular protein transportation system similar to that of animal cells. Thus, expression of membrane proteins and secretory proteins derived from animals is relatively easier than that involving the use of prokaryotic hosts.

In general, membrane proteins or secretory proteins comprise at their N termini secretory signal peptides. It is reported that substitution of the secretory signal peptides of the membrane proteins or secretory proteins with secretory signal peptides exhibiting high secretion efficiency, including those derived from hosts, can enhance the expression efficiency and the success rate of expression of such membrane proteins or secretory proteins in a protein expression system. In order to improve the efficiency of membrane and secretory protein expression systems, it is critical to improve the secretion efficiency of secretory signal peptides. Examples of signal peptides of membrane proteins and secretory proteins derived from Saccharomyces cerevisiae or yeast viruses that are used in conventional membrane and secretory protein expression systems in yeast include secretory signal peptides derived from α-factor (Non-Patent Document 1), α-factor receptor (Non-Patent Document 2), preprotoxin, SUC2 proteins and PHO5 proteins (Non-Patent Document 3), BGL2 proteins (Non-Patent Document 4), and AGA2 proteins (Non-Patent Document 5). However, only a few secretory signal peptides have been us-ed- for the expression of membrane proteins and secretory proteins in the past. Accordingly, it is unknown whether or not such secretory signal peptides used so far -exhibit the highest level of efficiency.

Computer programs that predict the sequences of secretory signal peptides have been provided. Examples of such computer programs include SOSUI signal Beta (http://sosui.proteome.bio.tuat.ac.jp/~sosui/proteome/sosuisignal/sosuisignal_s ubmit.html), SignalP (http://www.cbs.dtu.dk/services/SignalP/), PSORT (http://psort.nibb.ac.jp/), and Phobius (http://phobius.cgb.ki.se/). Use of these computer programs enables the prediction of the presence of the sequences of the secretory signal peptides in membrane proteins and secretory proteins based on the genomic information. At present, however, it is difficult to predict the efficiency of each secretory signal peptide with the use of these computer programs. Specifically, it is impossible to predict whether or not a predicted secretory signal peptide can actually be used to express proteins such as membrane proteins and secretory proteins and whether or not such peptide could be used for efficient mass-production of proteins.

In the past, the present inventors developed an expression system in yeast at low temperature by analyzing the response mechanism of Saccharomyces cerevisiae at low temperature (Patent Documents 1 and 2 and Non-Patent Document 6). The present inventors conducted an experiment concerning the expression of human proteins with the use of this expression system. As a result, they found that this expression system could more effectively inhibit the insolubilization of expressed proteins and the degradation caused by proteases at low temperature and would yield higher expression efficiency with the use of low-temperature-inducible promoters that are induced by low-temperature stimuli, compared with the conventional expression systems involving budding yeast at moderate temperature. In the present expression system, however, the- expression efficiency of membrane proteins and secretory proteins were lower than that of the soluble proteins in yeast cells.

Patent Document 1: WO 2004/003197;

Patent Document 2: JP Patent Publication (Kokai) No. 2005-160357 A;

Non-Patent Document 1: M. K. Ramjee, J. R. Petithory, J. McElver, S. C. Weber, and J. F. Kirsch., A novel yeast expression/secretion system for the recombinant plant thiol endoprotease propapain, Protein Engineering, 1996, vol. 9, pp. 1055-1061;

Non-Patent Document 2: V. Sarramegna, F. Talmont, P. Demange, and A. Milon, Heterologous expression of G-protein-coupled receptors: comparison of expression systems from the standpoint of large-scale production and purification, Cellular and Molecular Life Sciences, 2003, vol. 60, pp. 1529-1546;

Non-Patent Document 3: A. Eiden-Plach, T. Zagorc, T. Heintel, Y. Carius, F. Breinig, and MJ. Schmitt, Viral preprotoxin signal sequence allows efficient secretion of green fluorescent protein by Candida glabrata, Pichia pastoris, Saccharomyces cerevisiae, and Schizosaccharomyces pombe., Applied and Environmental Microbiology, 2004, vol. 70, pp. 961-966;

Non-Patent Document 4: T. Achstetter, M. Nguyen-Juilleret, A. Findeli, M. Merkamm, and Y. Lemoine, A new signal peptide useful for secretion of heterologous proteins from yeast and its application for synthesis of hirudin., Gene, 1992, vol. 110, pp. 25-31;

Non-Patent Document 5: D. Huang and EV. Shusta, Secretion and surface display of green fluorescent protein using the yeast Saccharomyces cerevisiae., Biotechnology Progress, 2005, vol. 21, pp. 349-357;

Non-Patent Document 6: T. Sahara, T. Goda, and S. Ohgiya, Comprehensive expression analysis of time-dependent genetic response in yeast cells to low temperature., Journal of Biological Chemistry, 2002, vol. 277, pp .50015-50021

### Disclosure of the Invention

As described above, the expression of membrane proteins and secretory proteins requires the use of secretory signal peptides exhibiting high secretion efficiency.

Accordingly, it is an object of the present invention to identify and provide secretory signal peptides exhibiting higher efficiency of transportation to cell organelles, including cell membranes, endoplasmic reticulum, and Golgi bodies, and higher efficiency of extracellular secretion, than conventional secretory signal peptides, for example.

The present inventors have conducted concentrated studies in order to attain the above object. As a result, they succeeded in discovering secretory signal peptides exhibiting a higher ability for transportation to cell organelles, including cell membranes, endoplasmic reticulum, and Golgi bodies, and a higher ability for extracellular secretion under moderate and low temperature conditions, than secretory signal peptides used in conventional membrane and secretory protein expression systems, from membrane proteins and secretory proteins existing in the Saccharomyces cerevisiae genomes, with the use of low-temperature-inducible promoters (Patent Document 1 and Non-Patent Document 6) and a reporter assay system involving the use of secretory luciferase as a reporter protein (International Application Number: PCT/JP2006/311597, claiming the priority right of JP Patent Application No. 2005-169768). This has led to the completion of the present invention.

The present invention includes the following.
(1) DNA encoding the following secretory signal peptide (a) or (b):
   (a) a secretory signal peptide consisting of the amino acid sequence as shown in any one of SEQ ID NO: 2, 4, 6, 8, 10, 12, 14, 16, and 18; or
   (b) a secretory signal peptide consisting of an amino acid sequence derived from the amino acid sequence of the secretory signal peptide (a) by deletion, substitution, or addition of one or several amino acids and having secretory signal activity at 30°C.
(2) DNA according to (1), wherein said DNA is any one of the following DNA (a) to (c) encoding a secretory signal peptide:
   (a) DNA consisting of the nucleotide sequence as shown in any one of SEQ ID NO: 1, 3, 5, 7, 9, 11, 13, 15, and 17;
   (b) DNA consisting of a nucleotide sequence derived from DNA (a) by deletion, substitution, or addition of one or several nucleotides and encoding a secretory signal peptide having secretory signal activity at 30°C; and
   (c) DNA hybridizing under stringent conditions with DNA consisting of a nucleotide sequence complementary to DNA (a) and encoding a secretory signal peptide having secretory signal activity at 30°C.
(3) A secretory signal peptide encoded by DNA according to (1) or (2).
(4) An expression vector comprising DNA according to (1) or (2) and a foreign gene.
(5) A transformant transformed by the expression vector according to (4).
(6) The transformant according to (5), wherein the host is yeast.
(7) The transformant according to (6), wherein the yeast is Saccharomyces cerevisiae.
(8) A method for producing a protein, wherein the transformant according to any one of (5) to (7) is cultured at 20°C to 42°C.
(9) DNA encoding the following secretory signal peptide (a) or (b):
   (a) a secretory signal peptide consisting of the amino acid sequence as shown in any one of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82-, 84, 86, 88, 90, 92, 94, 96, 98, 100, and 102; or
   (b) a secretory signal peptide consisting of an amino acid sequence derived from the amino acid sequence of the secretory signal peptide (a) by deletion, substitution, or addition of one or several amino acids and having secretory signal activity at 15°C.
(10) DNA according to (9), wherein said DNA is any one of the following DNA (a) to (c) encoding a secretory signal peptide:
   (a) DNA consisting of the nucleotide sequence as shown in any one of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, and 101;
   (b) DNA consisting of a nucleotide sequence derived from DNA (a) by deletion, substitution, or addition of one or several nucleotides and encoding a secretory signal peptide having secretory signal activity at 15°C; and
   (c) DNA hybridizing under stringent conditions with DNA consisting of a nucleotide sequence complementary to DNA (a) and encoding a secretory signal peptide having secretory signal activity at 15°C.
(11) A secretory signal peptide encoded by DNA according to (9) or (10).
(12) An expression vector comprising DNA according to (9) or (10) and a foreign gene.
(13) A transformant transformed by the expression vector according to (12).
(14) The transformant according to (13), wherein the host is yeast.
(15) The transformant according to (14), wherein the yeast is Saccharomyces cerevisiae.
(16) A method for producing a protein, wherein the transformant according to any one of (13) to (15) is cultured at 0°C to 20°C.

This specification includes part or all of the contents as disclosed in the specification and/or -drawings of each of Japanese Patent Applications Nos. 2005-339383 and 2006-297923, which are priority documents of the present application.

### Preferred Embodiments of the Invention

Hereafter, the present invention is described in detail.

DNA encoding a secretory signal peptide according to the present invention can be identified by identifying a gene encoding a protein containing the secretory signal peptide derived from Saccharomyces cerevisiae. At the outset, 1,037 genes included in the categories of the plasma membrane, the integral membrane, the cell periphery, the cell wall, the extracellular, the endoplasmic reticulum (ER), and Golgi from the subcellular localization table of MIPS CYGD (http://mips.gsf.de/genre/proj/yeast/), which is the Saccharomyces cerevisiae (budding yeast) genomic database, are selected. Based on the amino acid sequences encoded by the nucleotide sequences of the genes selected from the database, the transmembrane sites and the secretory signal peptides are then predicted using the prediction programs for transmembrane domains and the pred-iction programs for the secretory signal peptides, TMHMM 2.0 (http://www.cbs.dtu.dk/services/TMHMM/), Phobius (http://phobius.cgb.ki.se/), SOSUI signal Beta (http://sosui.proteome.bio.tuat.ac.jp/~sosui/proteome/sosuisignal/sosuisignal_s ubmi-t.html), and SignalP 3.0 (http://www.cbs.dtu.dk/services/SignalP/). Based on the results of this analysis, the gene regions encoding secretory signal peptides are extracted from the aforementioned database via several prediction programs, concerning the genes, for which the secretory signal peptides have been predicted.

Further, a reporter gene containing a DNA fragment in which a secretory luciferase gene lacking the original secretory signal peptide has been ligated downstream of the gene region encoding the predicted secretory signal peptide is prepared, and reporter assay (International Application Number: PCT/JP2006/311597, claiming the priority right of JP Patent Application No. 2005-169768) is carried out using the resulting reporter gene to identify the secretory signal peptide having secretion ability that is more than twice, at moderate temperature (30°C) and low temperature (15°C), that of the secretory signal peptide (the α-factor-derived secretory signal peptide (Non-Patent Document 1)) used in conventional membrane and secretory protein expression systems, with the use of extracellular luciferase activity as an indicator.

As a result, 9 genes containing novel gene regions encoding secretory signal peptides having high secretion ability at moderate temperature were identified. The identified genes are shown in Table 1.

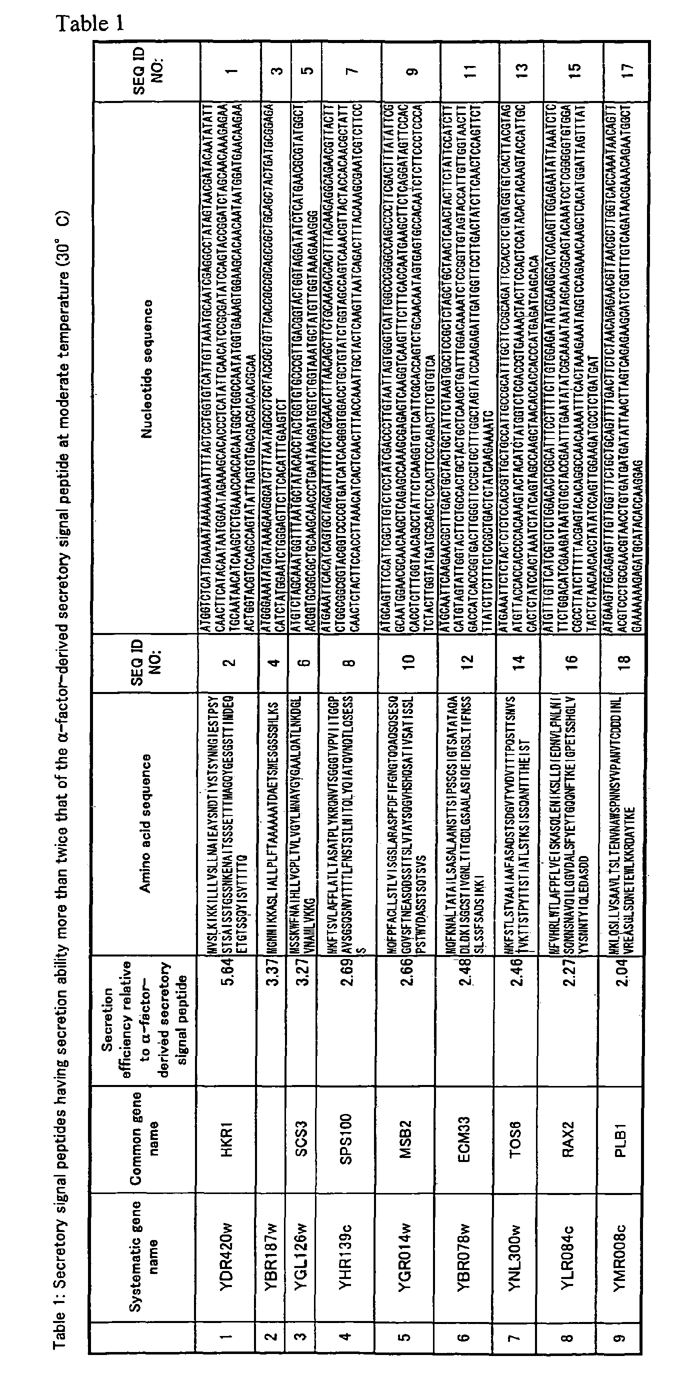

Table 1 shows the systematic and common names of genes from which the gene regions encoding secretory signal peptides are derived, relative secretion efficiency in relation to α-factor-derived secretory signal peptide, the amino acid sequences of the secretory signal peptides, and the nucleotide sequences of the gene regions encoding secretory signal peptides, concerning secretory signal peptides having secretion ability that is more than twice that of α-factor-derived secretory signal peptide at moderate temperature (30°C). SEQ ID NOs: of the amino acid sequences and those of the nucleotide sequences are indicated in the rightmost column.

Also, 51 genes containing novel gene regions encoding secretory signal peptides having high secretion ability at low temperature are shown in Table 2.

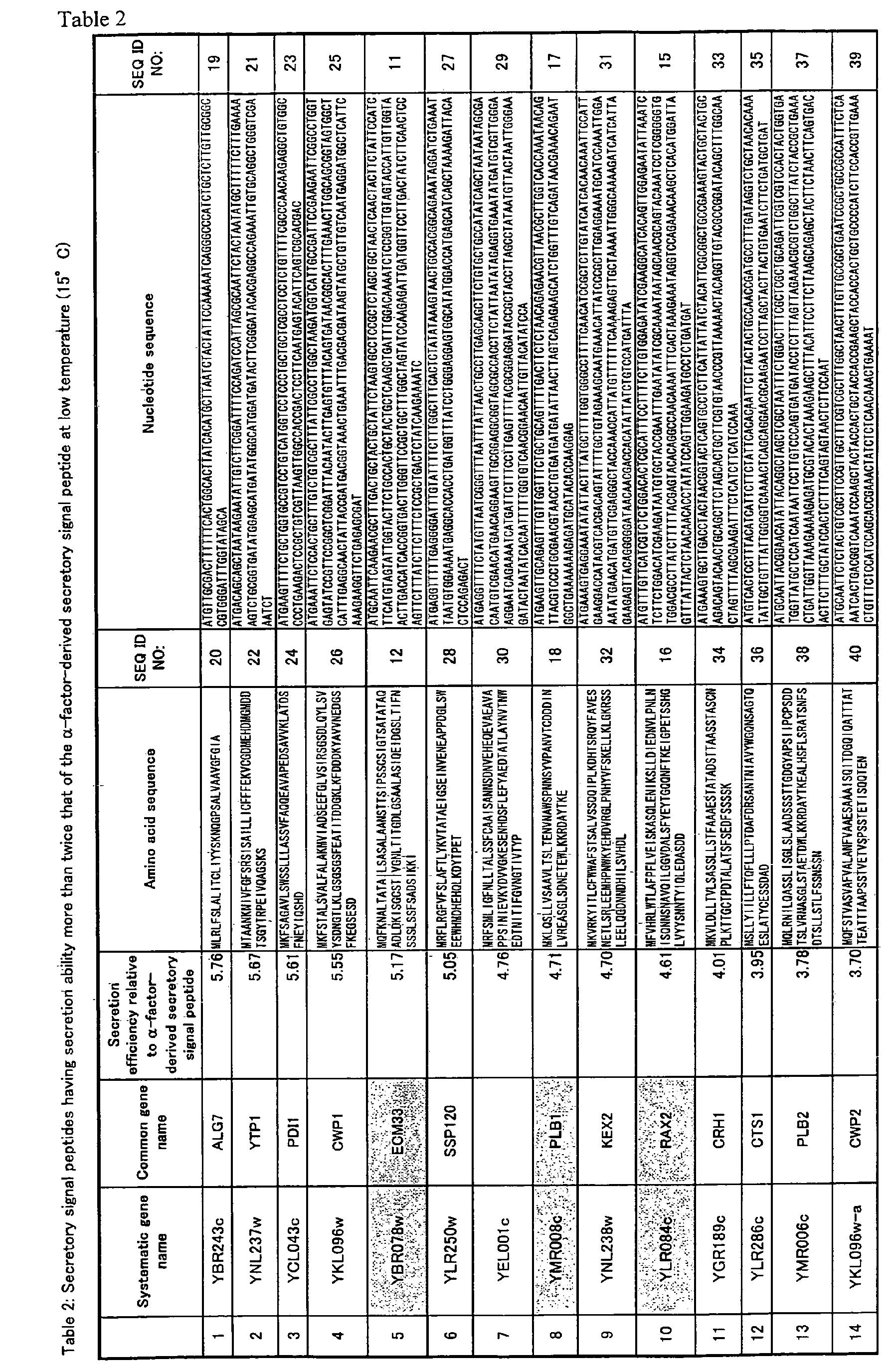

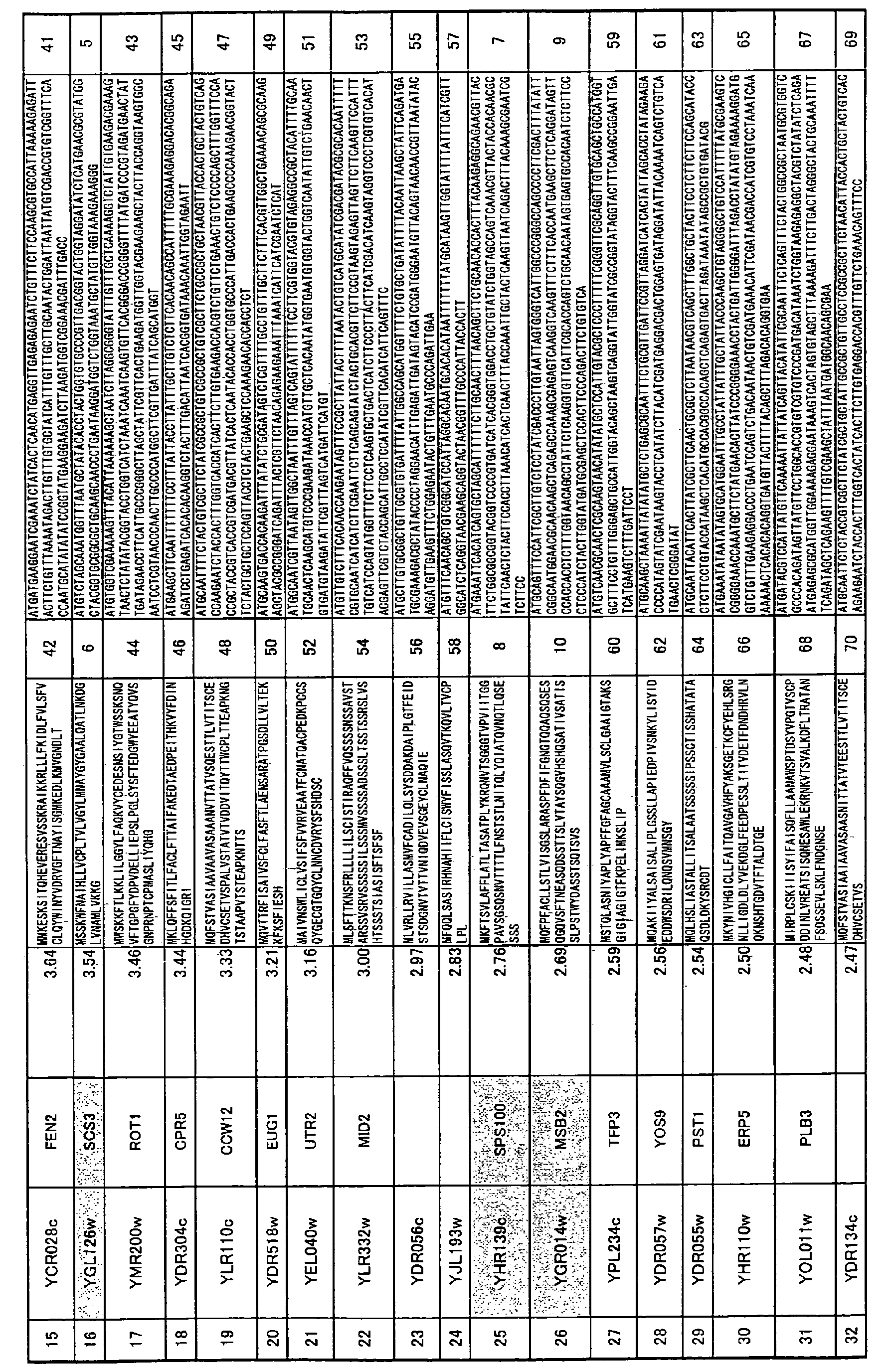

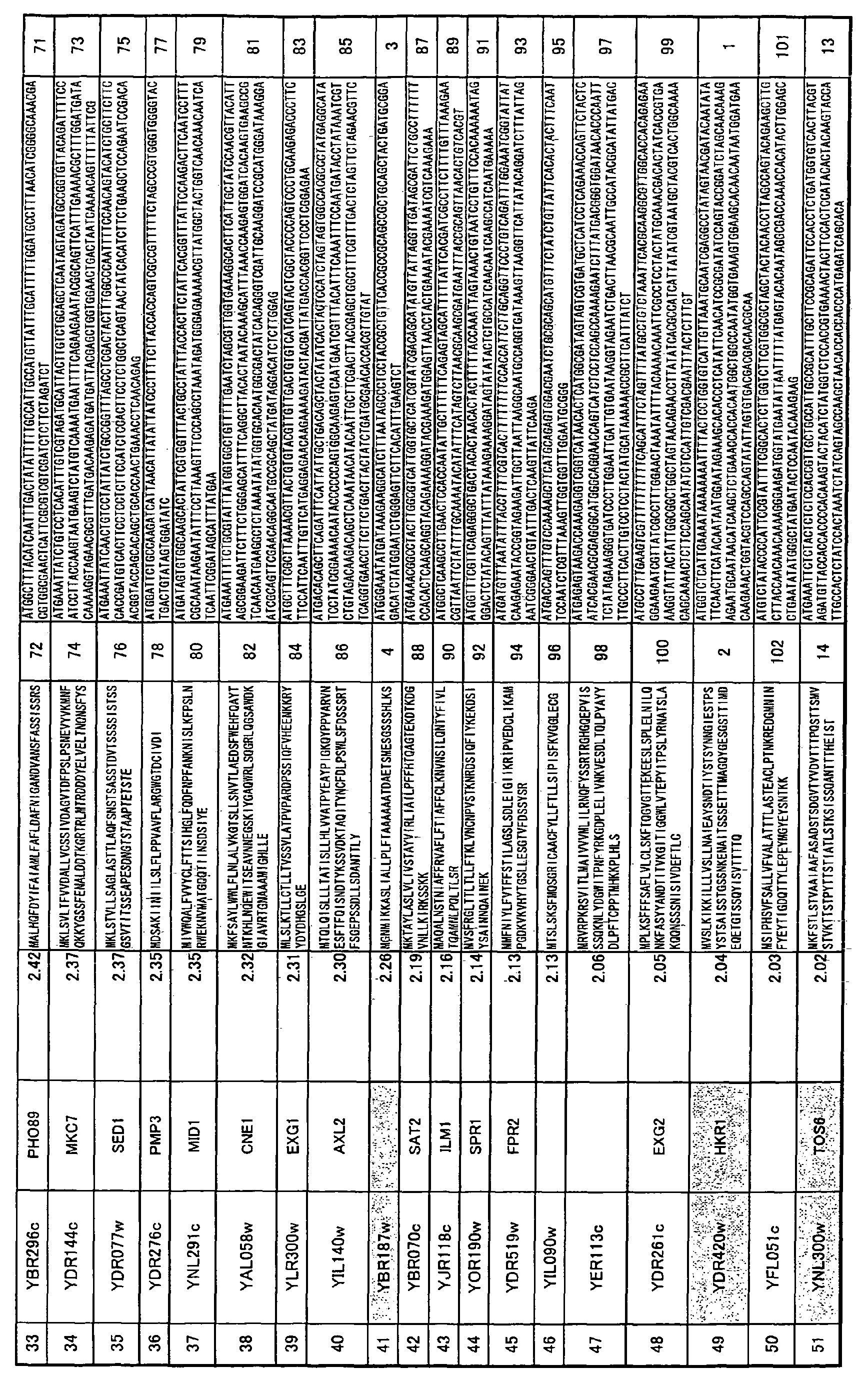

Table 2 shows the systematic and common names of genes from which the gene regions encoding secretory signal peptides are derived, relative secretion efficiency in relation to α-factor-derived secretory signal peptides, the amino acid sequences of the secretory signal peptides, and the nucleotide sequences of the gene regions encoding secretory signal peptides, concerning secretory signal peptides having secretion ability that is more than twice that of α-factor-derived secretory signal peptide at low temperature (15°C). SEQ ID NOs: of the amino acid sequences and the nucleotide sequences are indicated in the rightmost column. The systematic and common names- of genes (or gene regions encoding secretory signal peptides) identical to genes (or gene regions encoding secretory signal peptides) shown in Table 1 are shaded.

Hereafter, a secretory signal peptide (or a gene region encoding a secretory signal peptide) shown in Table 1 is referred to as a "secretory signal peptide at moderate temperature (or a gene region encoding a secretory signal peptide at moderate temperature)," and a secretory signal peptide (or a gene region encoding a secretory signal peptide) shown in Table 2 is referred to as a "secretory signal peptide at low temperature (or a gene region encoding a secretory signal peptide at low temperature)." Also, a "secretory signal peptide at moderate temperature (or a gene region encoding a secretory signal peptide at moderate temperature)" and a secretory signal peptide at low temperature (or -a gene region encoding a secretory signal peptide at low temperature) are collectively referred to as "secretory signal peptides (or gene regions encoding secretory signal peptides)."

DNA according to the present invention encodes a secretory signal peptide consisting of the amino acid sequence of a secretory signal peptide shown in Table 1 or 2. DNA according to the present invention can be easily obtained by preparing a primer based on the amino acid sequence of a secretory signal peptide shown in Table 1 or 2 and conducting PCR using the genomic DNA extracted from Saccharomyces cerevisiae as a template.

DNA according to the present invention may be DNA encoding a secretory signal peptide consisting of an amino acid sequence derived from the amino acid sequence of a secretory signal peptide shown in Table 1 or 2 by deletion, substitution, or addition of one or several amino acids (e.g., 1 to 10 or 1 to 5 amino acids), and having secretory signal activity at 30°C regarding the amino acid sequence derived from the amino acid sequence of the secretory signal peptide at moderate temperature shown in Table 1, or having secretory signal activity at 15°C regarding the amino acid sequence derived from the amino acid sequence of the secretory signal peptide at low temperature shown in Table 2.

The term "secretory signal activity" used herein refers to the ability for extracellular protein secretion of secretory signal peptides expressed as fusion proteins with proteins, such as membrane proteins and secretory proteins, or the ability for transporting proteins to intracellular organelles, including cell walls, cell membranes, endoplasmic reticulums, and Golgi bodies. Secretory signal activity of the secretory signal peptide encoded by DNA according to the present invention can be evaluated by, for example, utilizing a reporter gene, such as the luciferase gene. For example, an expression vector, wherein a reporter gene is ligated, -as a fusion protein, to a site downstream of DNA according to the present invention, is constructed. Subsequently, an adequate host (e.g., yeast) is transformed using the resulting expression vector. The resulting transformant is cultured at 30°C or 15°C, the amount of the reporter protein secreted extracellularly or the amount thereof transported to intracellular organelles, including cell walls, cell membranes, endoplasmic reticulums, and Golgi bodies, is quantified to result in the activity level of the reporter protein or to result in the protein level via an immunological method (e.g., Western blotting, ELISA, or flow cytometry). Thus, secretory signal activity of the secretory signal peptide encoded by DNA according to the present invention can be evaluated. Alternatively, the ability for transportation to the intracellular organelles as secretory signal activity can be evaluated by inspecting the localization of the fusion protein encoded by a foreign gene ligated downstream of DNA according to the present invention in intracellular organelles via immunostaining using an antibody or other means.

Further, DNA according to the present invention may be DNA consisting of a nucleotide sequence of a gene region encoding a secretory signal peptide shown in Table 1 or 2, or DNA cosisting of a nucleotide sequence derived from the nucleotide sequence of a gene region encoding a secretory signal peptide shown in Table 1 or 2 by deletion, substitution, or addition of 1 or several (e.g., 1 to 10 or 1 to 5) nucleotides and encoding a secretory signal peptide having secretory signal activity at 30°C regarding the nucleotide sequence derived from the nucleotide sequence of the gene region encoding a secretory signal peptide at moderate temperature shown in Table 1, or encoding a secretory signal peptide having secretory signal activity at 15°C regarding the nucleotide sequence derived from the nucleotide sequence of the gene region encoding a secretory signal peptide at low temperature shown in Table 2. Accordingly, DNA according to the present invention may be the full-length DNA consisting of the nucleotide sequence of the gene region encoding a secretory signal peptide shown in Table 1 or 2. As long as the amino acid sequence encoded by DNA according to the present invention exhibits secretion signal activity at 30°C, such DNA may be part of the DNA consisting of the nucleotide sequence of the gene region encoding a secretory signal peptide at moderate temperature shown in Table 1. Alternatively, such DNA may be part of the DNA consisting of the nucleotide sequence of the gene region encoding a secretory signal peptide at low temperature shown in Table 2, provided that the amino acid sequence encoded by such DNA exhibits secretory signal activity at 15°C.

DNA according to the present invention includes a DNA that hybridizes under stringent conditions with DNA consisting of a nucleotide sequence complementary to DNA consisting of a nucleotide sequence of a gene region encoding a secretory signal peptide shown in Table 1 or 2 and that encodes a secretory signal peptide exhibiting secretory signal activity at 30°C regarding a gene region encoding a secretory signal peptide at moderate temperature shown in Table 1 or at 15°C regarding a gene region encoding a secretory signal peptide at low temperature shown in Table 2.

Under stringent conditions, for example, hybridization with phosphorus-32-labeled probe DNA is carried out in a hybridization solution comprising 5x SSC (0.75 M NaCl, 0.75 M sodium citrate), 5x Denhart's reagent (0.1% ficoll, 0.1% polyvinylpyrrolidone, 0.1% bovine serum albumin), and 0.1% sodium dodecyl sulfate (SDS) at 45°C to 65°C, and preferably at 55°C to 65°C. The step of washing is carried out in a washing solution comprising 2x SSC and 0.1% SDS at 45°C to 55°C, and preferably in a washing solution comprising 0.1x SSC and 0.1% SDS at 45°C to 55°C. When probe DNA labeled with an enzyme using the AlkPhos direct labeling module kit (Amersham Bioscience) is used, hybridization is carried out in a hybridization solution (containing 0.5 M NaCI and a 4% blocking reagent) having the- composition described in the manufacturer's instructions of the kit at 55°C to 75°C. The step of washing is carried out in a primary washing solution (containing 2M urea) described in the manufacturer's instructions of the kit at 55°C to 75°C and in a secondary washing solution at room temperature.- Another detection method may be employed. In such a case, such detection method may be carried out under its standard conditions.

Once the nucleotide sequence of DNA according to the present invention has been determined, DNA of the present invention can be obtained by chemical synthesis, by PCR using the cloned probe as a template, or by hybridization using a DNA fragment having the aforementioned nucleotide sequence as a probe. Further, DNA that is a mutant of the DNA of the present invention and has functions equivalent to those before mutation can be synthesized via, for example, site-directed mutagenesis.

Mutation can be introduced into DNA of the present invention by conventional techniques, such as the Kunkel method, the Gapped duplex method, or a method in accordance therewith. For example, a mutagenesis kit (e.g., Mutant-K that utilizes site-directed mutagenesis, TaKaRa) or the LA PCR in vitro Mutagenesis Series kit (TaKaRa) may be used to introduce mutation. Other mutagenesis techniques may also be employed.

The secretory signal peptide of the present invention is encoded by DNA of the present invention. The secretory signal peptide of the present invention can be obtained as a fusion protein with a protein to be expressed (e.g., a membrane or secretory protein).

An expression vector comprising DNA of the present invention and a foreign gene (hereafter referred to as the "expression vector of the present invention") can be obtained by inserting DNA of the present invention and a foreign gene into an adequate vector. The foreign gene may be located downstream or upstream of DNA of the present invention in the expression vector of the present invention. Alternatively, DNA of the present invention may be present in the foreign gene. Preferably, the foreign gene is located adjacent to a site downstream of DNA of the present invention. DNA of the present invention is inserted into the expression vector of the present invention in-frame with the foreign gene.

Any vector may be used without particular limitation, provided that the vector can be replicated in a host. Examples thereof include a plasmid, a shuttle vector, and a helper plasmid. When the vector is incapable of replication, a DNA fragment that becomes replicable upon insertion thereof into a host chromosome may be used.

Examples of plasmid DNAs include E. coli-derived plasmid DNAs (e.g., pBR322, pBR325, pUC118, pUC119, pUC18, pUC19, and pBluescript), Bacillus subtilis-derived plasmid DNAs (e.g., pUB110 and pTP5), and yeast-derived plasmid DNAs (e.g., a YEp plasmid such as YEp13 and a YCp plasmid such as YCp50). Examples of phage DNAs include λ phages, such as Charon4A, Charon21A, EMBL3, EMBL4, λgt10, λgt11, and λZAP. Animal virus vectors, such as retrovirus or vaccinia virus vectors, and insect virus vectors, such as baculovirus vectors, can also be used.

DNA of the present invention may be inserted into a vector by first cleaving the purified DNA with an adequate restriction enzyme, inserting the cleavage product into a restriction site or multicloning site of an adequate vector DNA, and ligating the product to a vector. With the provision of a homologous region at a part of the vector and at a part of DNA of the present invention, the vector may be ligated to DNA of the present invention via, for example, the in vitro method involving PCR or the in vivo method using yeast.

A foreign gene may be inserted into a vector at a site downstream or upstream of DNA of the present invention, or a foreign gene may be inserted into a vector so as to locate DNA of the present invention within the foreign gene in the same manner as with the insertion of DNA of the present invention into the vector.

In the expression vector according to the present invention, a foreign gene located downstream or upstream of DNA of the present invention or a foreign gene containing DNA of the present invention therein may be any protein or peptide. Examples thereof include a membrane protein and a secretory protein.

Further, a transformant can be obtained by introducing the expression vector of the present invention into a host. Any host can be used without particular limitation, provided that DNA of the present invention and a foreign gene can be expressed therein. An example thereof is yeast. Examples of yeast include Saccharomyces cerevisiae, experimental yeast, fermentation yeast, edible yeast, and industrial yeast.

The expression vector of the present invention may be introduced into yeast via any method without particular limitation, provided that DNA is introduced into yeast by such method. Examples thereof include electroporation, spheroplast, and lithium acetate methods. Also, yeast transformation involving substitution and/or insertion with a chromosome may be carried out using vectors such as a YIp vector or a DNA sequence homologous to an arbitrary region in the chromosome. Further, the expression vector of the present invention may be introduced into yeast by any method described in general experiment guidebooks or academic articles.

A transformant can also be obtained not only by introducing the expression vector of the present invention into the aforementioned yeast but also by introducing the expression vector into bacteria of Escherichia such as Escherichia coli, Bacillus such as Bacillus subtilis, or Pseudomonas such as Pseudomonas putida, animal cells such as COS cells, insect cells such as Sf9 cells, or plants belonging to genera such as Brassicaceae. When a bacterial host is used, it is preferable that the expression vector of the present invention may be capable of autonomous replication therein and that the vector may be composed of a promoter, a ribosome binding site, DNA of the present invention, a foreign gene, and a transcription termination sequence.

The expression vector of the present invention may be introduced into bacteria by any method without particular limitation, provided that DNA is to be introduced into bacteria by such method. Examples thereof include a method involving the use of calcium ions and electroporation.

When an animal cell host is used, for example, monkey cells (COS-7 cells or Vero cells), Chinese hamster ovarian cells (CHO cells), or mouse L cells may be used. The expression vector of the present invention may be introduced into animal cells by, for example, electroporation, the calcium phosphate method, or lipofection.

When an insect cell host is used, for example, Sf9 cells or the like may be used. The expression vector of the present invention may be introduced into insect cells by, for example, the calcium phosphate method, lipofection, or electroporation.

When a plant host is used, for example, the entire plant, a plant organ (e.g., a leaf, petal, stem, root, or seed), plant tissue (e.g., epidermis, phloem, parenchyma, xylem, or fibrovascular bundle), or cultured plant cells may be used. The expression vector of the present invention may be introduced into a plant host by, for example-, electroporation, the agrobacterium method, the particle gun method, or PEG.

Whether or not DNA of the present invention and a foreign gene were incorporated into a host can be examined by, for example, PCR, Southern hybridization, or Northern hybridization. For example, DNA is prepared from a transformant, a DNA-specific primer is designed, and PCR is then carried out. Thereafter, the amplification product is subjected to agarose gel electrophoresis, polyacrylamide gel electrophoresis, or capillary electrophoresis, the resultant is stained with ethidium bromide or an SYBR Green solution, and the amplification product is detected as a band to confirm the transformation. Also, PCR may be carried out using a primer labeled in advance with a fluorescent dye or the like and the amplification product may be detected. Further, the amplification product may be bound to a solid phase support, such as a microplate, to confirm the amplification product via, for example, fluorescence or enzyme reactions.

In the method for producing proteins according to the present invention, the aforementioned transformant obtained with the use of the expression vector of the present invention (hereafter referred to as the "transformant of the present invention") is cultured, and a fusion protein of the secretory signal peptide encoded by DNA according to the present invention and a protein encoded by a foreign gene is secreted and produced extracellularly or transported to and expressed in the intracellular organelles, including cell walls, cell membranes, endoplasmic reticulums, and Golgi bodies. When DNA of the present invention introduced into the transformant of the present invention is related to the gene region encoding a secretory signal peptide at moderate temperature, the transformant of the present invention is cultured at 20°C to 42°C, and preferably at 25°C to 37°C. When DNA of the present invention introduced into the transformant of the present invention is related to the gene region encoding a secretory signal peptide at low temperature, the transformant of the present invention is cultured at 0°C to 20°C, and preferably at 4°C to 15°C. Other culture conditions (e.g., the composition of the medium) for the transformant of the present invention can be adequately selected in accordance with the type of host to be used.

After the transformant of the present invention has been cultured, a protein encoded by a foreign gene can be isolated, extracted, and/or purified from the culture supernatant or culture product, as a fusion protein of the secretory signal peptide of the present invention with a protein encoded by a foreign gene. Alternatively, the secretory signal peptide of the present invention may be cleaved by, for example, signal peptidase in the transformant, and a protein encoded by a foreign gene can be selectively isolated, extracted, and/or purified from the culture supernatant or culture product. Proteins can be isolated, extracted, and purified in accordance with relevant general techniques.

According to the present invention, membrane proteins and secretory proteins can be expressed with higher efficiency than is possible with conventional membrane and secretory protein expression systems in yeast or the like with the use of secretory signal peptides. The expression system according to the present invention is superior to conventional membrane and secretory protein expression systems in terms of secretion efficiency. For example, the amount of protein production can be further increased in conventional expression of membrane proteins and secretory proteins in yeast.

### Examples

Hereafter, the present invention is described in greater detail with reference to the examples, although the technical scope of the present invention is not limited to these examples.

Concerning secretory signal peptides that are present in many of the genes encoding membrane proteins and secretory proteins in the Saccharomyces cerevisiae genome, the secretion abilities thereof were evaluated via the reporter assay system (see International Application- Number: PCT/JP2006/311597, claiming the priority right of JP Patent Application No. 2005-169768) involving the use of secreted Cypridina noctiluca luciferase (hereafter referred to as "CLuc;" nucleotide sequence: SEQ ID NO: 103; amino acid sequence: SEQ ID NO: 104) as a reporter protein. CLuc is used as a mature protein lacking a naturally occurring secretory signal peptide (hereafter referred to as "mature CLuc").

The secretory signal peptides exhibiting higher secretion efficiency than the secretory signal peptides used in conventional expression systems in yeast (see Non-Patent Documents 1 to 5) were identified in the following manner.

### [Example 1] Production of reporter vector pCLuRA-s

The reporter vector, pCLuRA-s, was produced utilizing a gene encoding mature CLuc as a reporter gene in the following manner.

The pUG35-MET25-EGFP3+MCS plasmid (see International Application Number: PCT/JP2006/311597, claiming the priority right of JP Patent Application No. 2005-169768) produced from pUG35 (http://mips.gsf.de/proj/yeast/info/tools/hegemann/gfp.html) was cleaved with HindIII and XbaI, the DNA fragment was fractionated via agarose gel electrophoresis, and a vector fragment of approximately 5.1 kbp was obtained. This vector fragment is hereafter referred to as "DNA fragment A."

The pCLuRA plasmid comprises a gene having, at the 5' end of DNA encoding mature CLuc, DNA encoding the α-factor-derived secretory signal peptide ligated thereto (see International Application Number: PCT/JP2006/311597, claiming the priority right of JP Patent Application No. 2005-169768). A gene encoding mature CLuc (i.e., a protein consisting of an amino acid sequence derived from the amino acid sequence of CLuc as shown in SEQ ID NO: 104 by deletion of a secretory signal peptide sequence consisting of amino acids 1 to 18) (hereafter referred to as the "mature CLuc gene") was amplified from the pCLuRA plasmid via PCR.

The following primers were used. cLuc ORF -Sig F +HindIII comprises at its 5' end the HindIII cleavage site, and downstream thereof, a sequence complementary to a 21-bp region encompassing the 5' end of the mature CLuc gene. cLuc ORF -Sig R +XbaI comprises at its 5' end the XbaI cleavage site, and downstream thereof, a sequence complementary to a 24-bp-sequence including a termination codon of the mature CLuc gene.

cLuc ORF -Sig F +HindIII: GCGC-AAGCTT-
CAGGACTGTCCTTACGAACCT (SEQ ID NO: 105)

cLuc ORF -Sig R +XbaI: GCGC-TCTAGA-
CTATTTGCATTCATCTGGTACTTC (SEQ ID NO: 106)

PCR was carried out using 50 µl of a reaction solution comprising 300 nM of each-primer, 1 mM MgSO₄, 200 µM dNTP (a mixed solution of 4 types of deoxynucleotide triphosphates), 1 ng of the pCLuRA plasmid, and 1x buffer and KOD-Plus-1U included in the KOD-Plus- (TOYOBO). The PCR cycle comprised: the first step at 94°C for 2 minutes; 35 cycles of the second step at 94°C for 15 seconds (denaturation), at 50°C for 30 seconds (annealing), and at 68°C for 2 minutes (elongation); and the third step at 68°C for 5 minutes.

The amplified DNA fragment was cleaved at the restriction enzyme cleavage sites at both of its ends with HindIII and XbaI, a DNA fragment was fractionated via agarose gel electrophoresis, and a DNA fragment containing approximately 1.6 kbp of the mature CLuc gene was obtained. This DNA fragment is hereafter referred to as "DNA fragment B."

Subsequently, DNA fragments A were ligated to DNA fragments B using the DNA Ligation Kit ver. 2.1 (TaKaRa), and the ligation products were then introduced into E. coli DH5α. The resulting transformants were cultured overnight, plasmids were extracted using the GenElute Plasmid Miniprep Kit (Sigma-Aldrich), and transformants having a plasmid of interest were identified based on the restriction enzyme cleavage patterns and via nucleotide sequence analysis. A plasmid was prepared from the transformant, the plasmid was cleaved with SpeI -and BamHI, a DNA fragment was fractionated via agarose gel electrophoresis, and a DNA fragment of approximately 6.7 kbp was obtained. This DNA fragment is hereafter referred to as "DNA fragment C."

A low-temperature-inducible promoter, i.e., an HSP12 gene promoter (hereafter referred to as "HSP12 promoter;" SEQ ID NO: 107), was amplified via PCR from the pLTex321s vector (see Patent Document 2).

The following primers were used. -610-HSP12 IGR F +SpeI comprises at its 5' end a SpeI cleavage site, and downstream thereof, a sequence complementary to a 19-bp region encompassing the 5' end of the HSP 12 promoter region. -610-HSP12 IGR R +BamHI comprises at its 5' end a BamHI cleavage site, and downstream thereof, a sequence complementary to a 28-bp region encompassing the 3' end of the HSP12 promoter region.

-610-HSP12 IGR F +SpeI: GG-ACTAGT-
GATCCCACTAACGGCCCAG (SEQ ID NO: 108)

-610-HSP12 IGR R +BamHI: CG-GGATCC-
TGTTGTATTTAGTTTTTTTTGTTTTGAG (SEQ ID NO: 109)

PCR was carried out using 50 µl of a reaction solution comprising 300 nM of each primer, 1 mM MgSO₄, 200 µM dNTP, 1 ng of the pLTex321s vector, and 1x buffer and KOD-Plus-1 U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 1 minute (elongation); and the third step at 68°C for 5 minutes.

The amplified DNA fragment was cleaved at restriction enzyme cleavage sites at both ends thereof with SpeI and BamHI, a DNA fragment was fractionated via agarose gel electrophoresis, and a fragment of approximately 600 bp of the HSP12 promoter region was obtained. This fragment of the HSP12 promoter region is hereafter referred to as "DNA fragment D."

DNA fragments C were ligated to DNA fragments D using the DNA Ligation Kit ver. 2.1, and the ligation products were introduced into E. coli DH5α. The resulting transformants were cultured overnight, plasmids were extracted using the GenElute Plasmid Miniprep Kit, and a transformant having a plasmid of interest was identified based on the restriction enzyme cleavage pattern and via nucleotide sequence analysis. The pCLuRA-s plasmid was prepared from the transformant. In the pCLuRA-s plasmid, the mature CLuc gene is inserted downstream of the HSP 12 promoter.

### [Example 2] Isolation of Saccharomyces cerevisiae-derived secretory signal peptide

The secretory signal peptides existing in membrane proteins and secretory proteins derived from the budding yeast Saccharomyces cerevisiae were extracted in the following manner.

Thousand and thirty seven genes included in the categories of the plasma membrane, the integral membrane, the cell periphery, the cell wall, the extracellular, the endoplasmic reticulum (ER), and Golgi from the subcellular localization table of MIPS CYGD (http://mips.gsf.de/genre/proj/yeast/), which is the Saccharomyces cerevisiae genomic database, were selected.

Based on the amino acid sequences encoded by the nucleotide sequences of the genes selected from the database, the transmembrane sites and the secretory signal peptides were predicted using the prediction programs for transmembrane sites and the prediction programs for the secretory signal peptides, such as TMHMM 2.0 (http://www.cbs.dtu.dk/services/TMHMM/), Phobius (http://phobius.cgb.ki.se/), SOSUI signal Beta (http://sosui.proteome.bio.tuat.ac.jp/∼sosui/proteome/sosuisignal/sosuisignal_s ubmit.html), and SignalP 3.0 (http://www.cbs.dtu.dk/services/SignalP/). Based on the results of this analysis, the gene regions encoding the predicted secretory signal peptides were extracted from the aforementioned database via several prediction programs, concerning genes encoding 440 types of proteins, for which the secretory signal peptides had been predicted (Table 3).

Table 3 below shows: the systematic and common names of the genes encoding 440 types of proteins relating to the secretory signal peptides extracted from the budding yeast genome information; the nucleotide sequences and the amino acid sequences of the gene regions encoding the predicted secretory signal peptide of the protein-encoding genes (all SEQ ID NOs of these nucleotide sequences and the amino acid sequences are indicated in the rightmost column in Table 3); and the primers used for amplifying the gene regions encoding secretory signal peptides (nucleotide sequences of the synthetic primers used for the amplifying the 1st PCR products; all SEQ ID NOs of such primers are indicated in the rightmost column in Table 3).

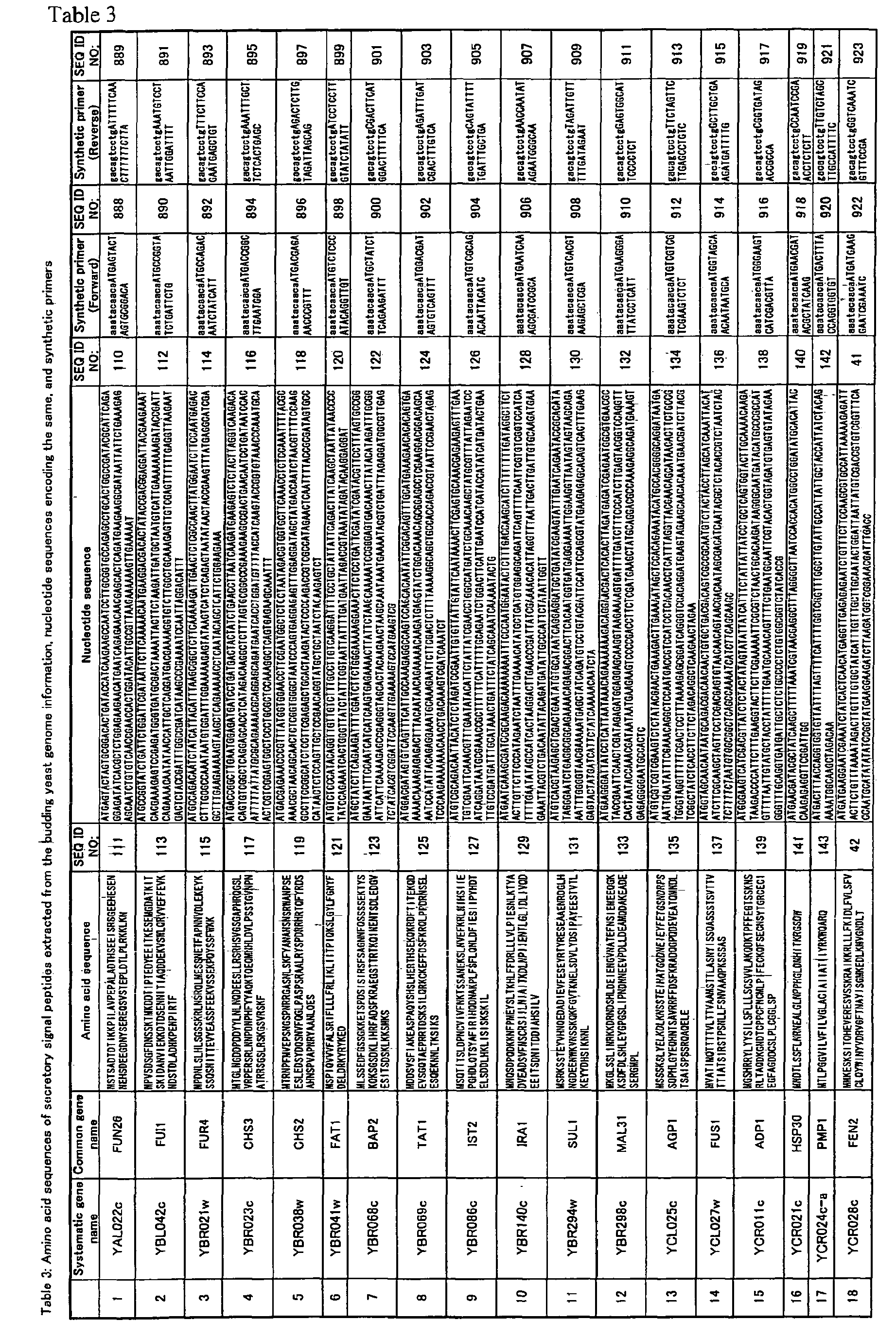

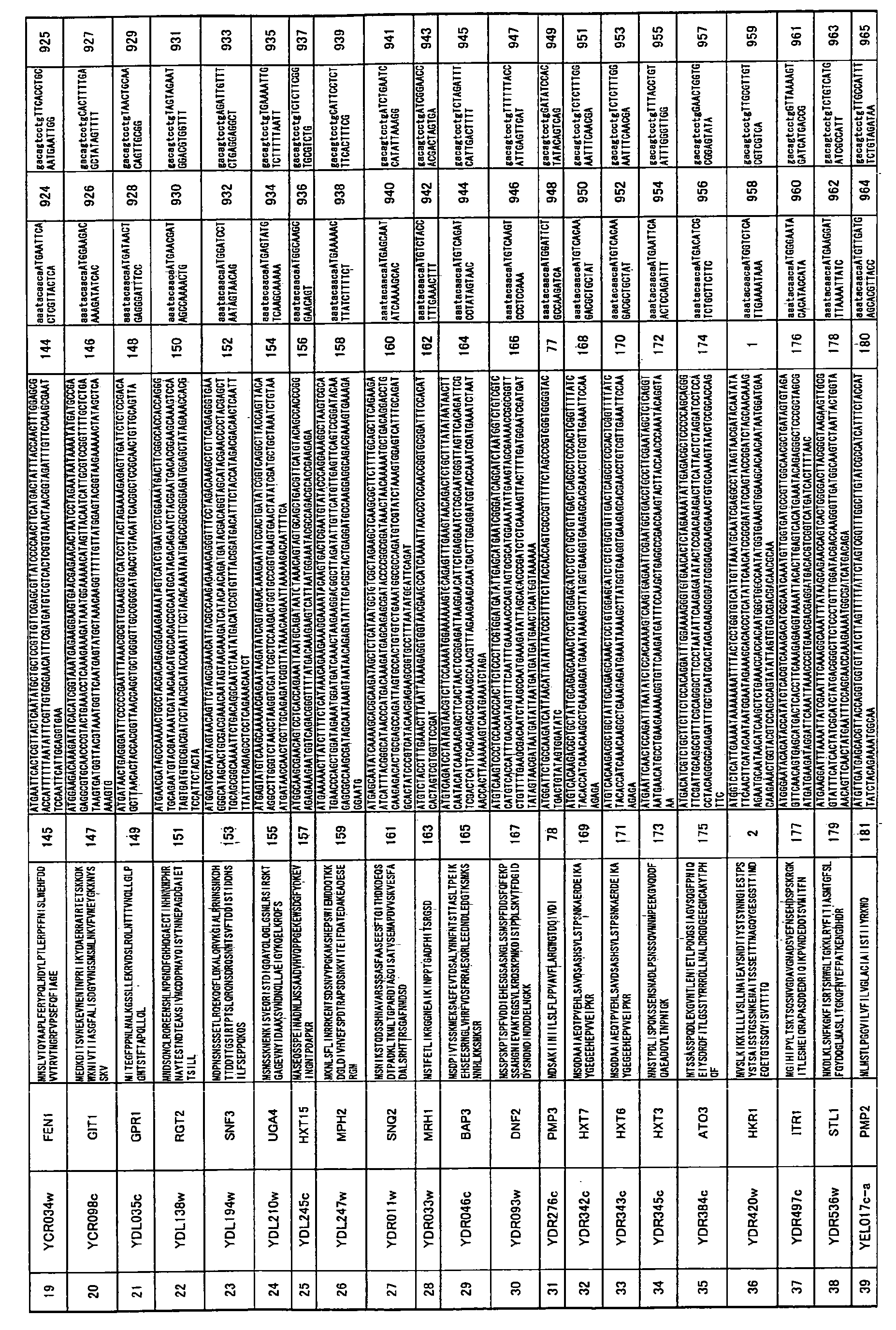

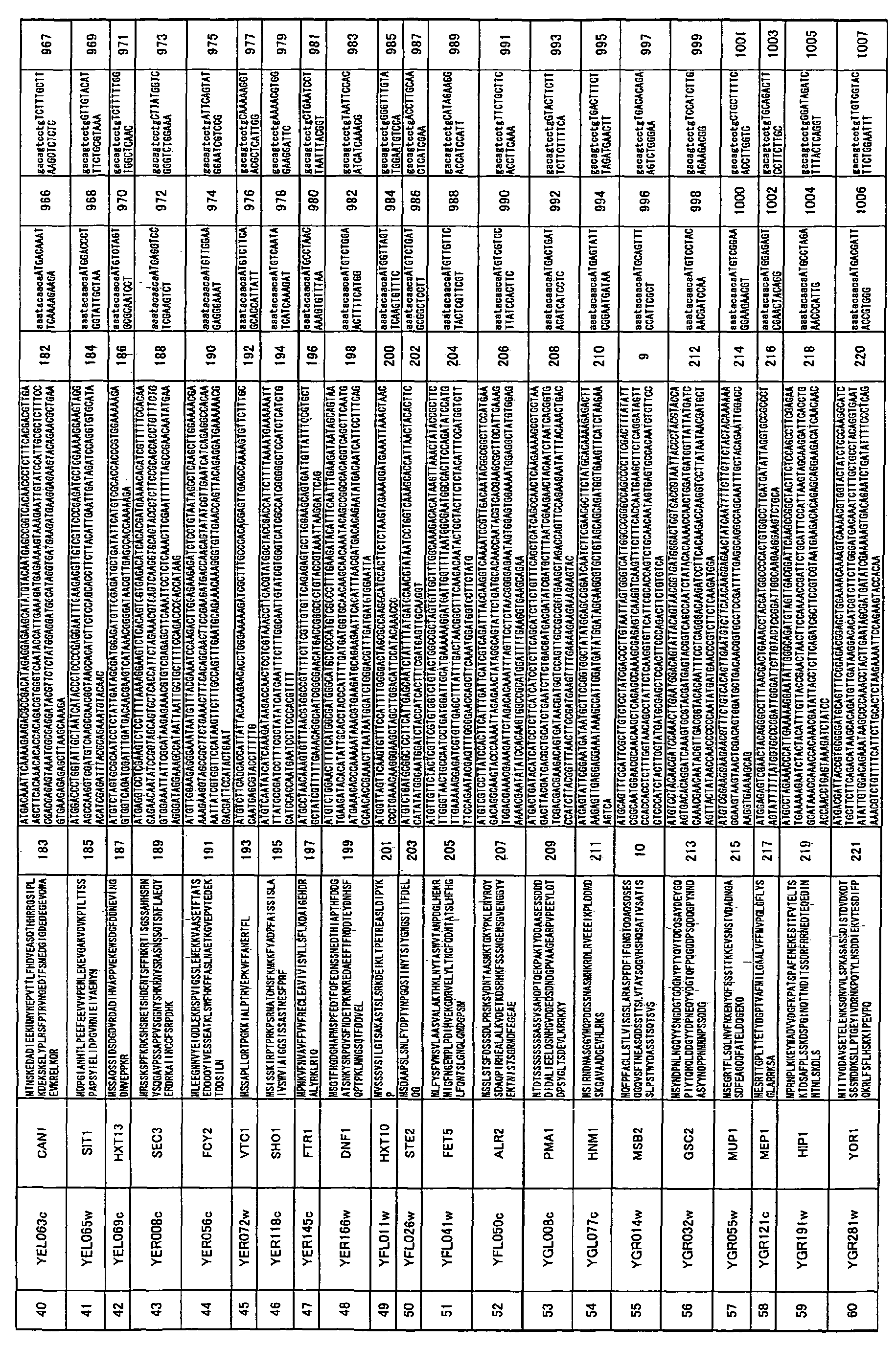

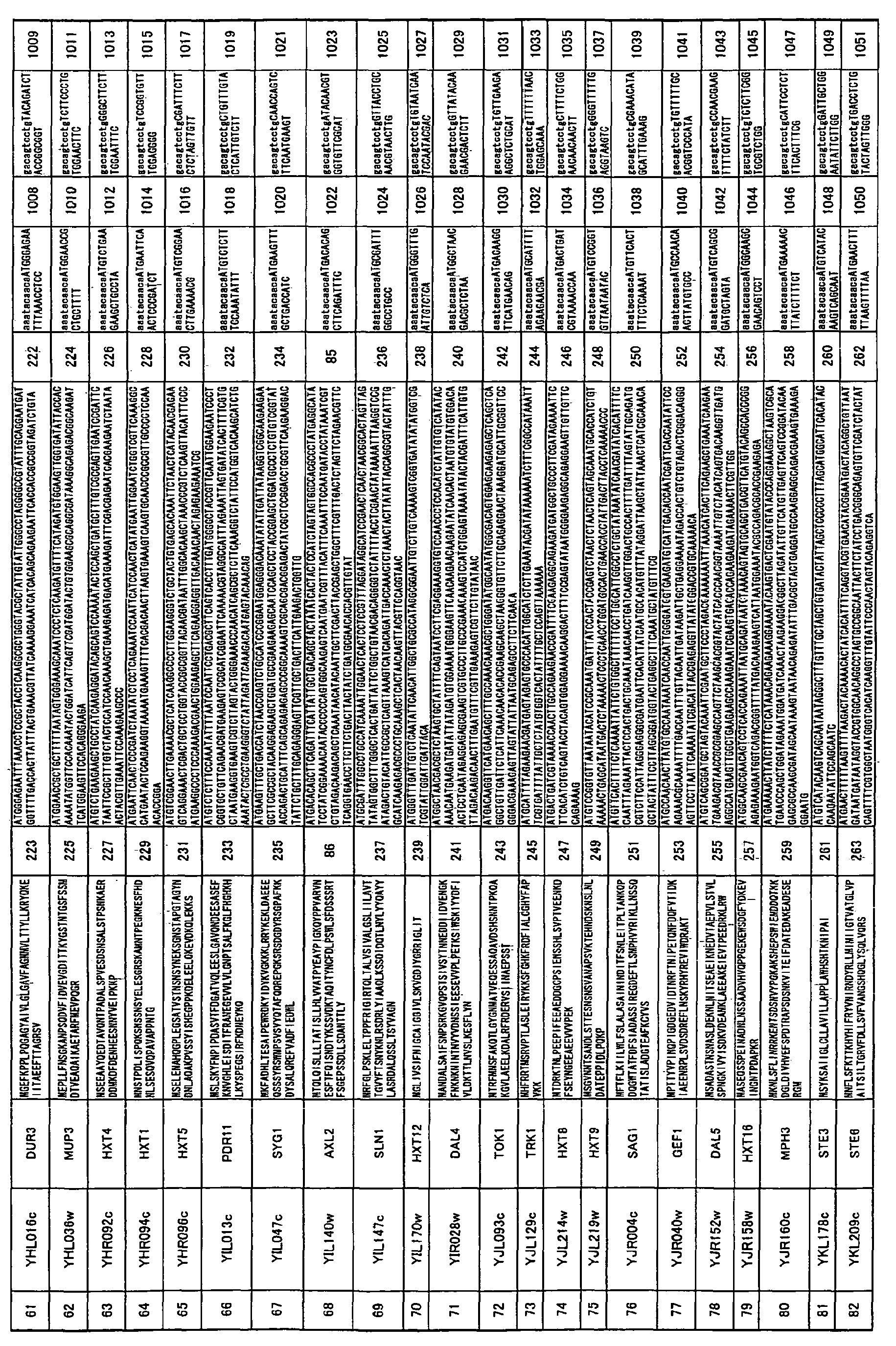

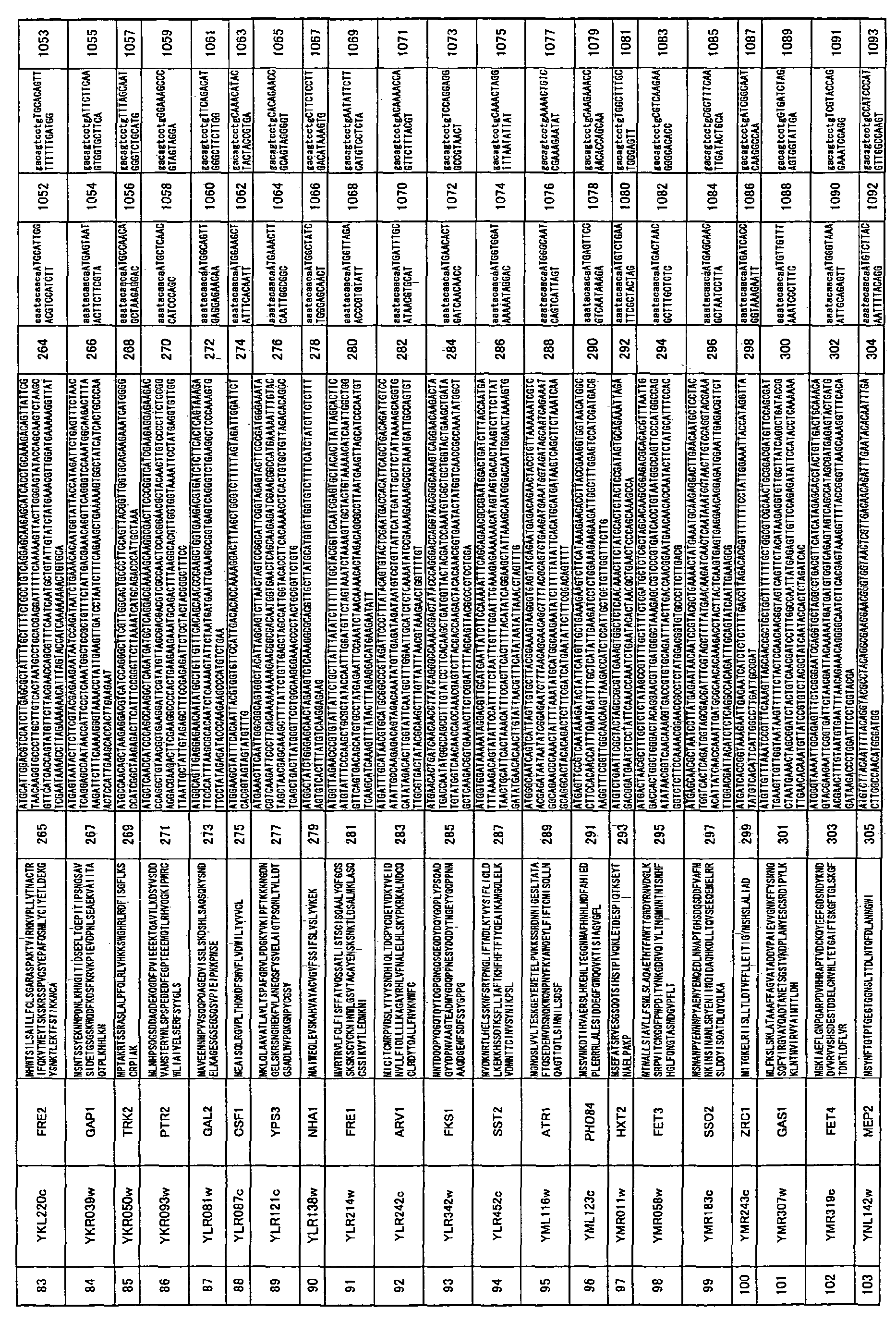

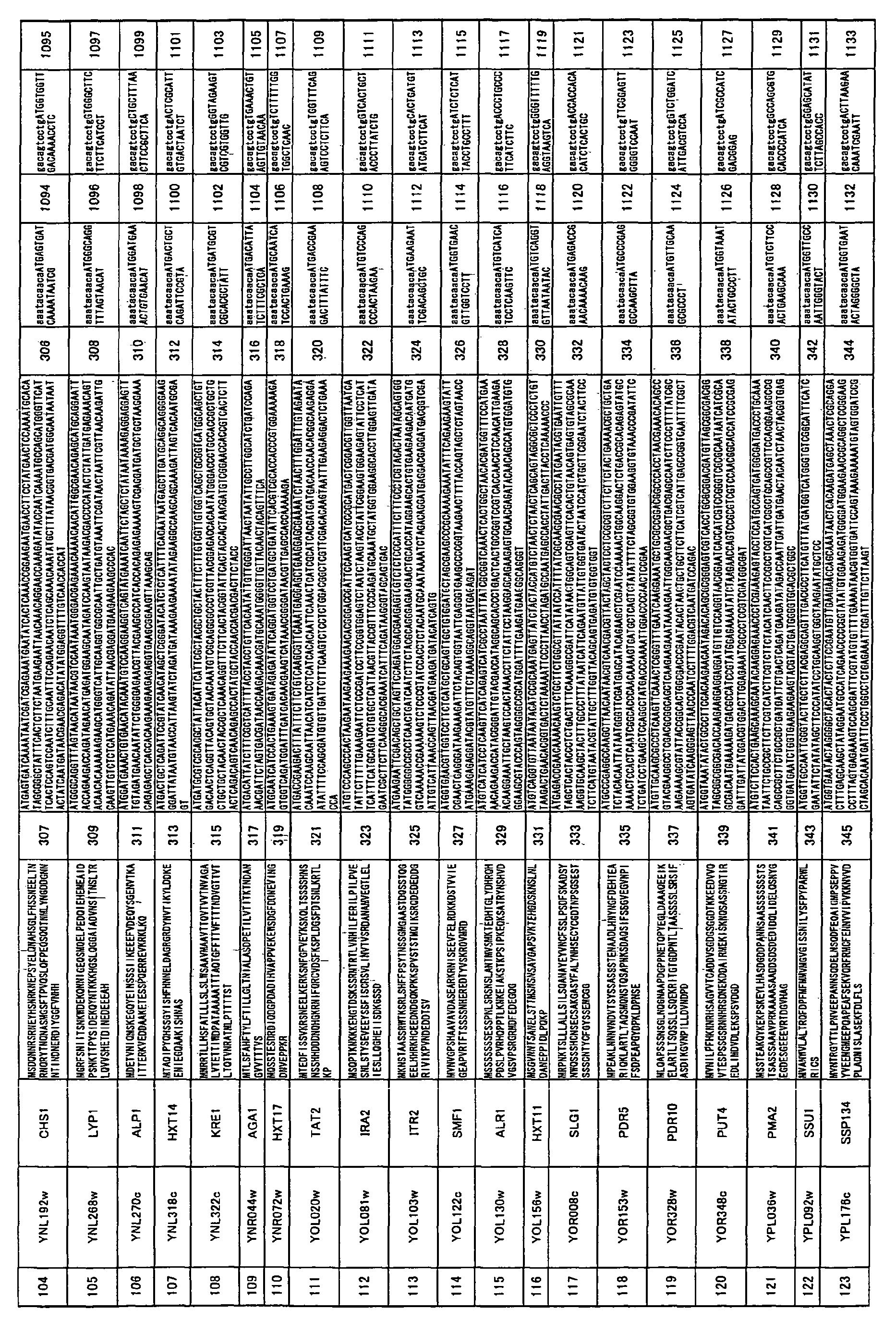

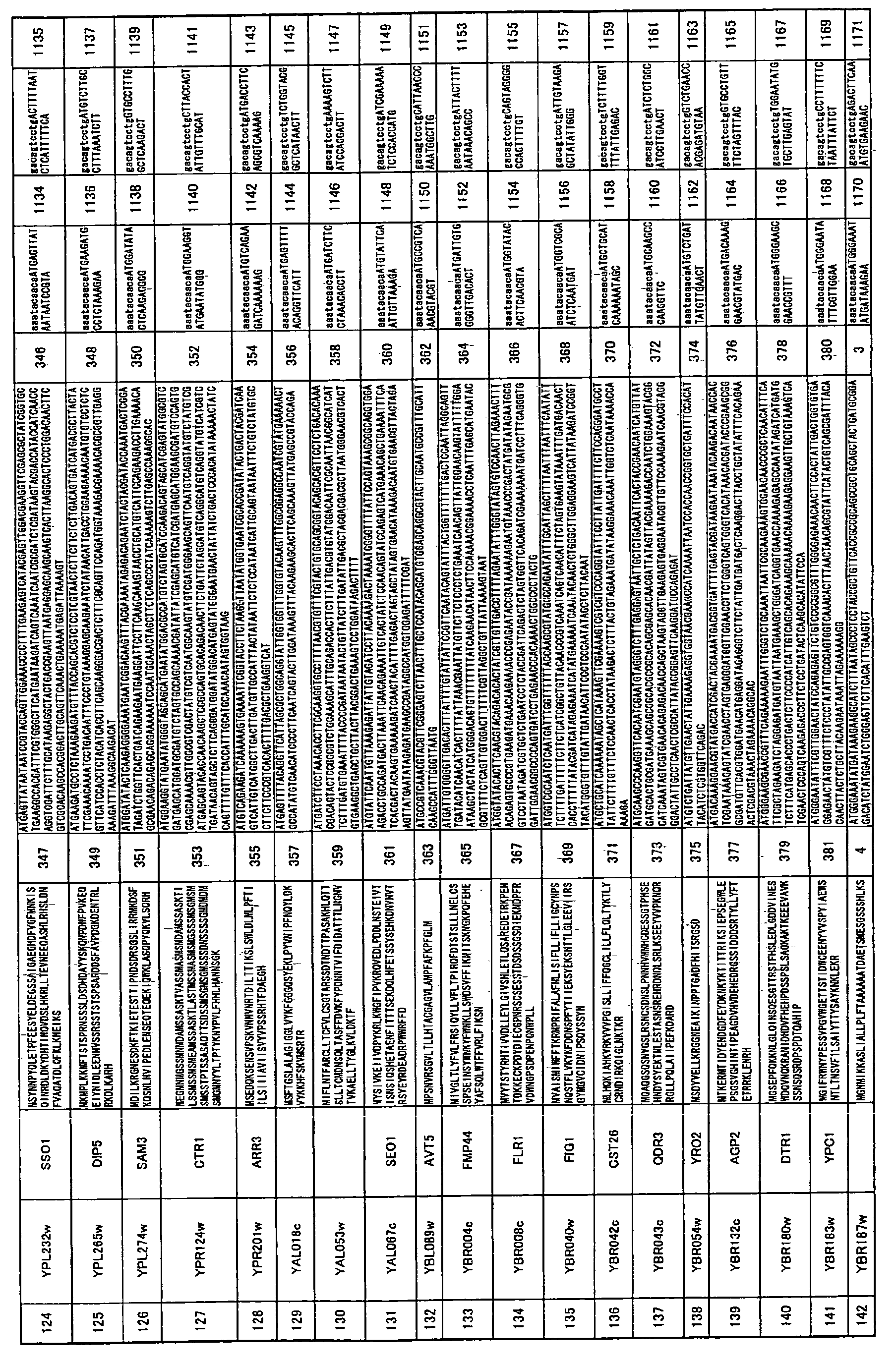

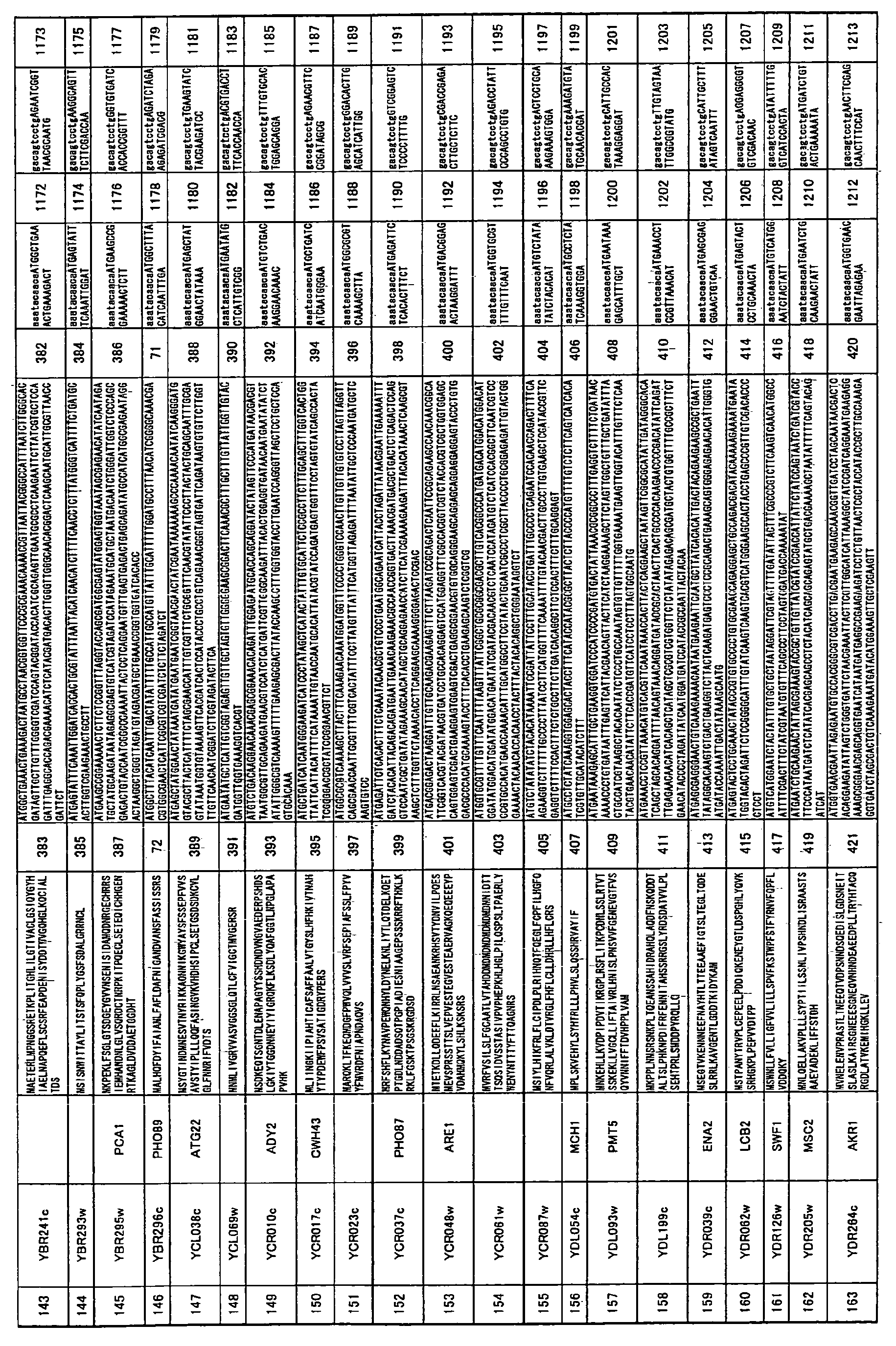

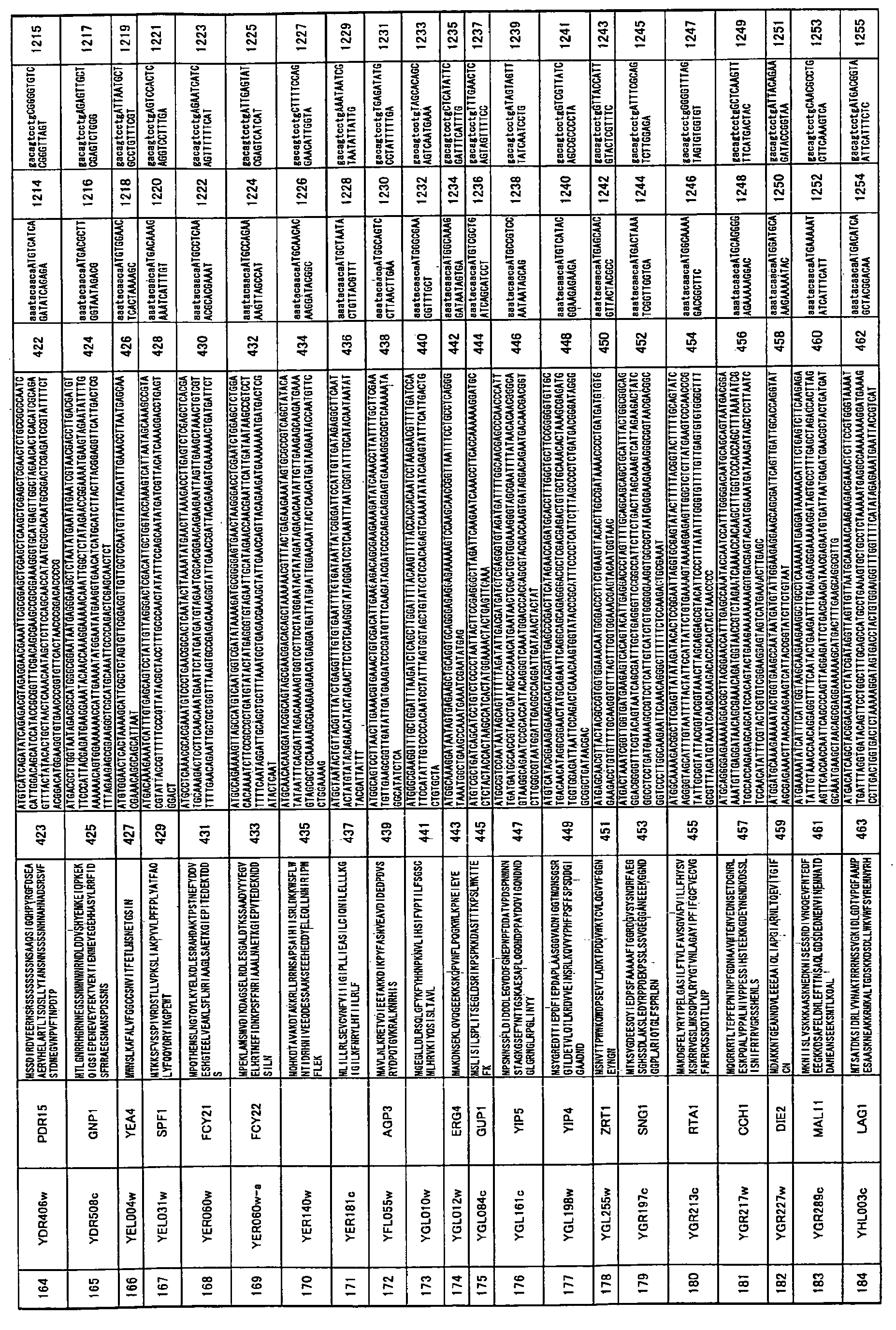

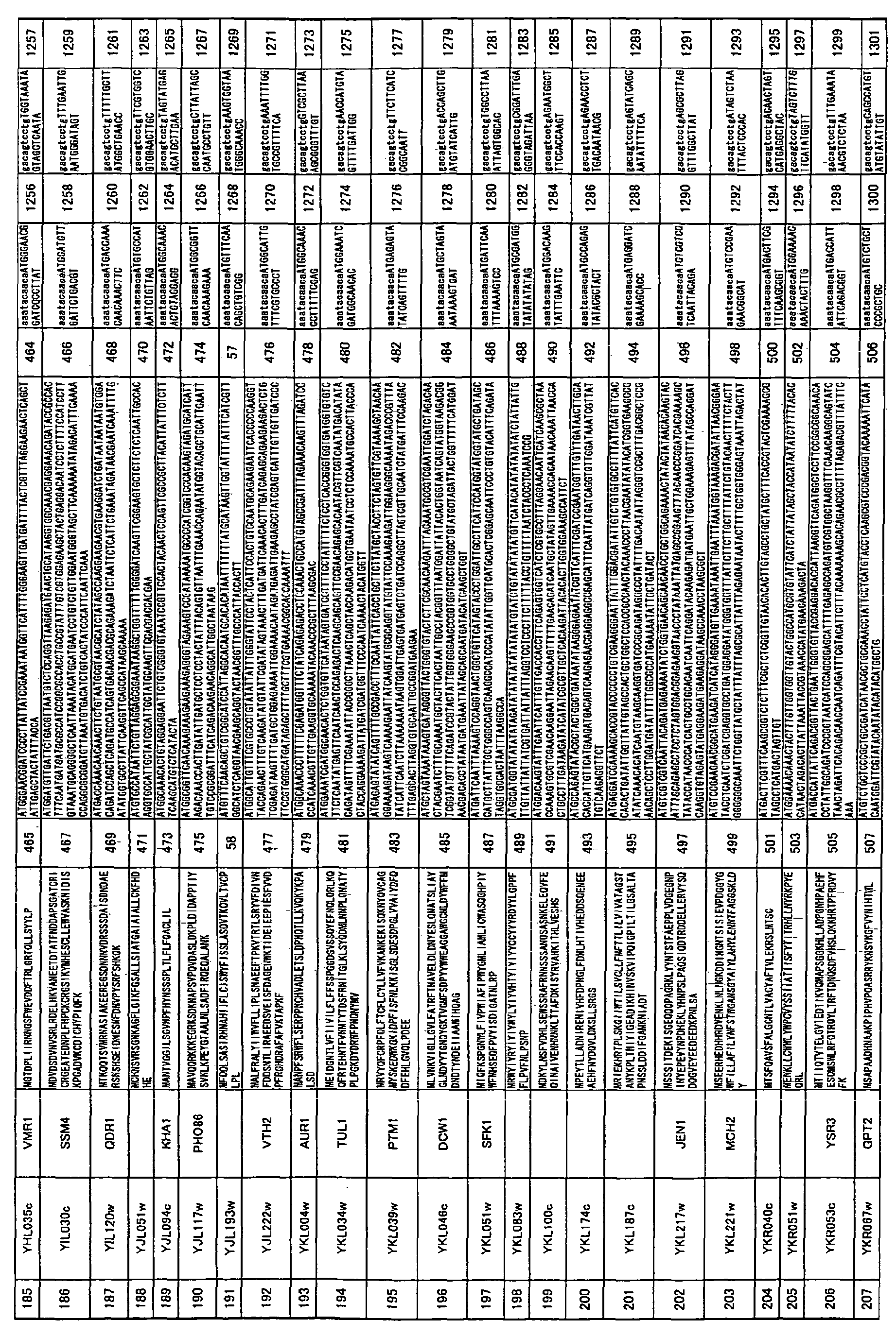

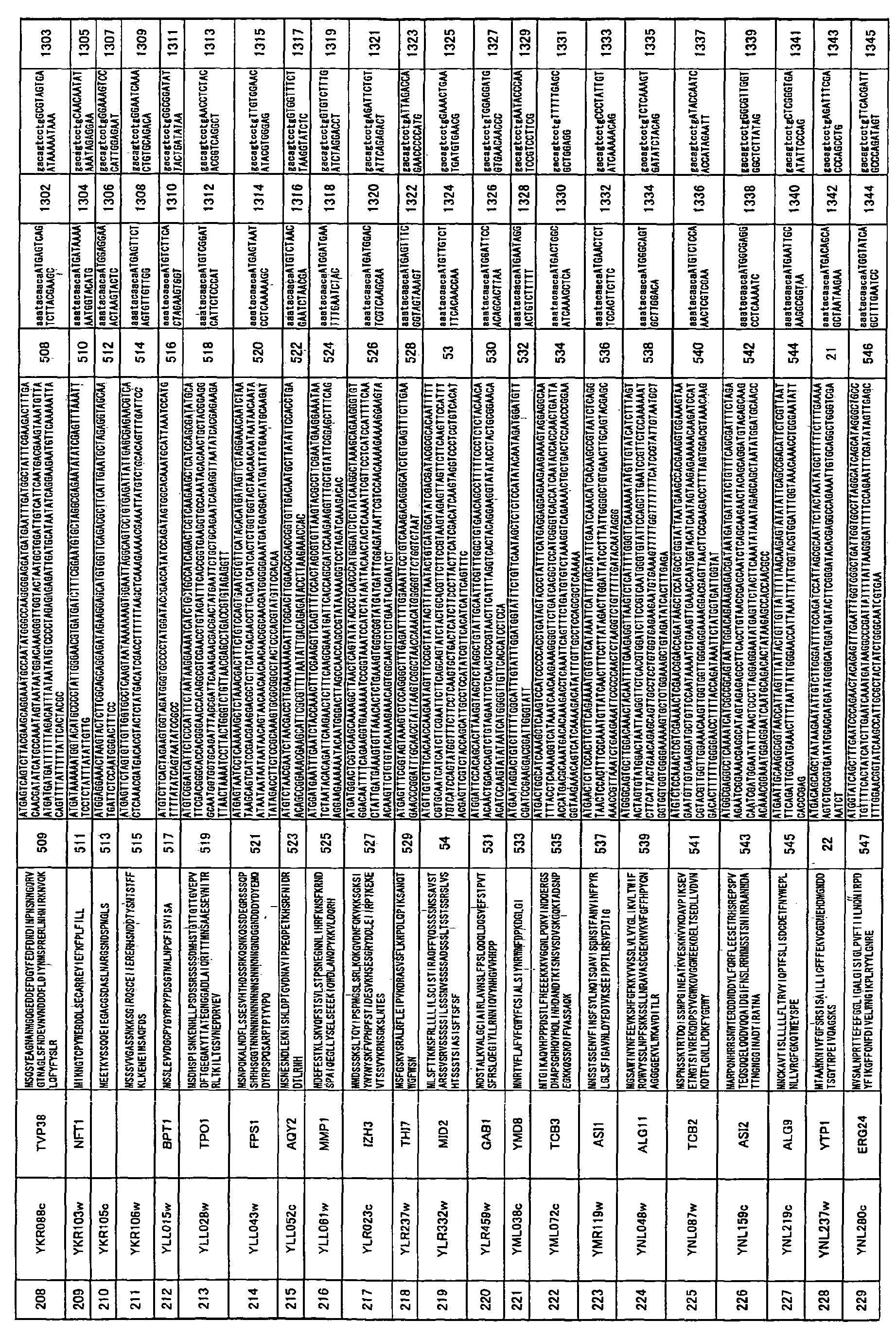

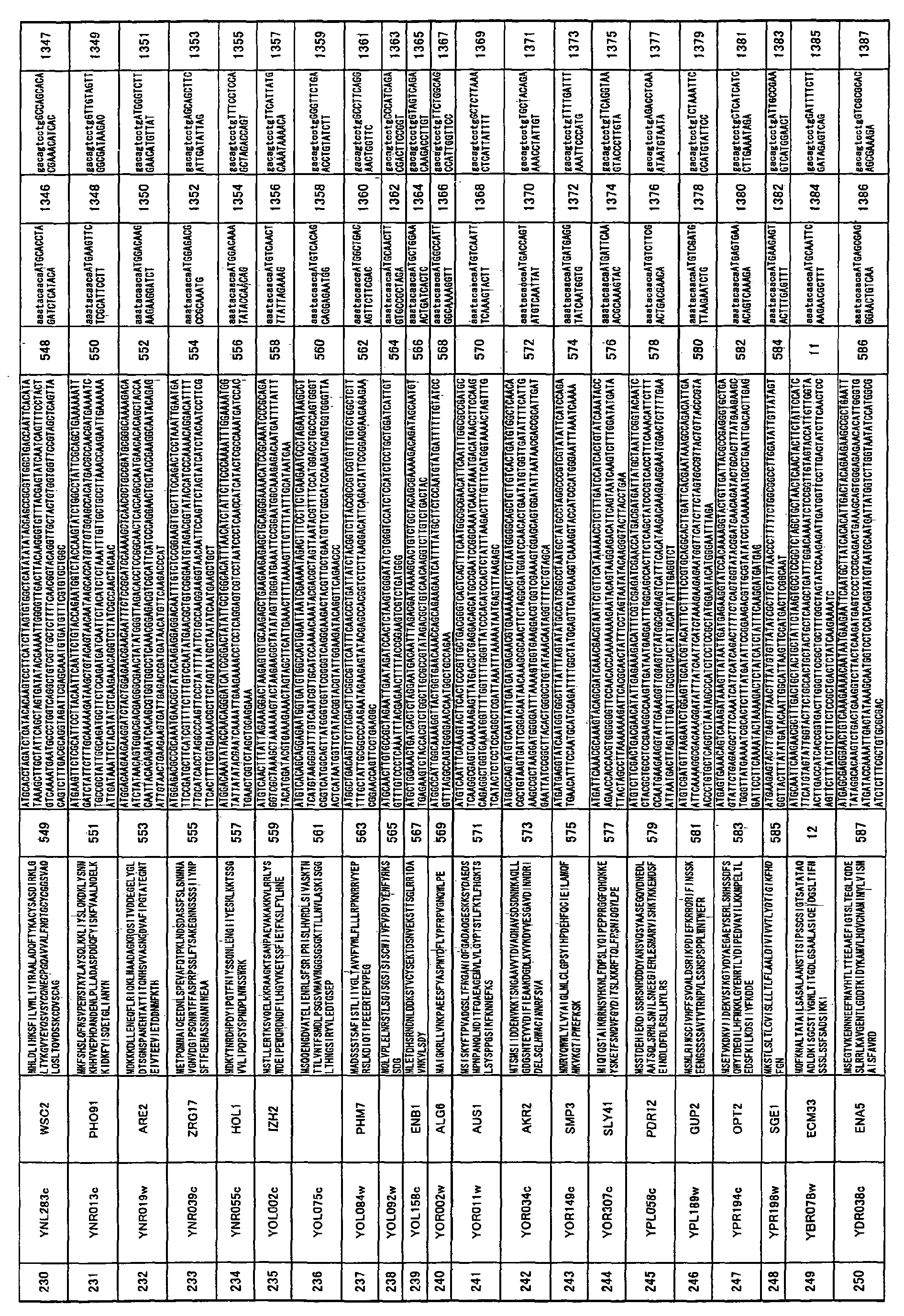

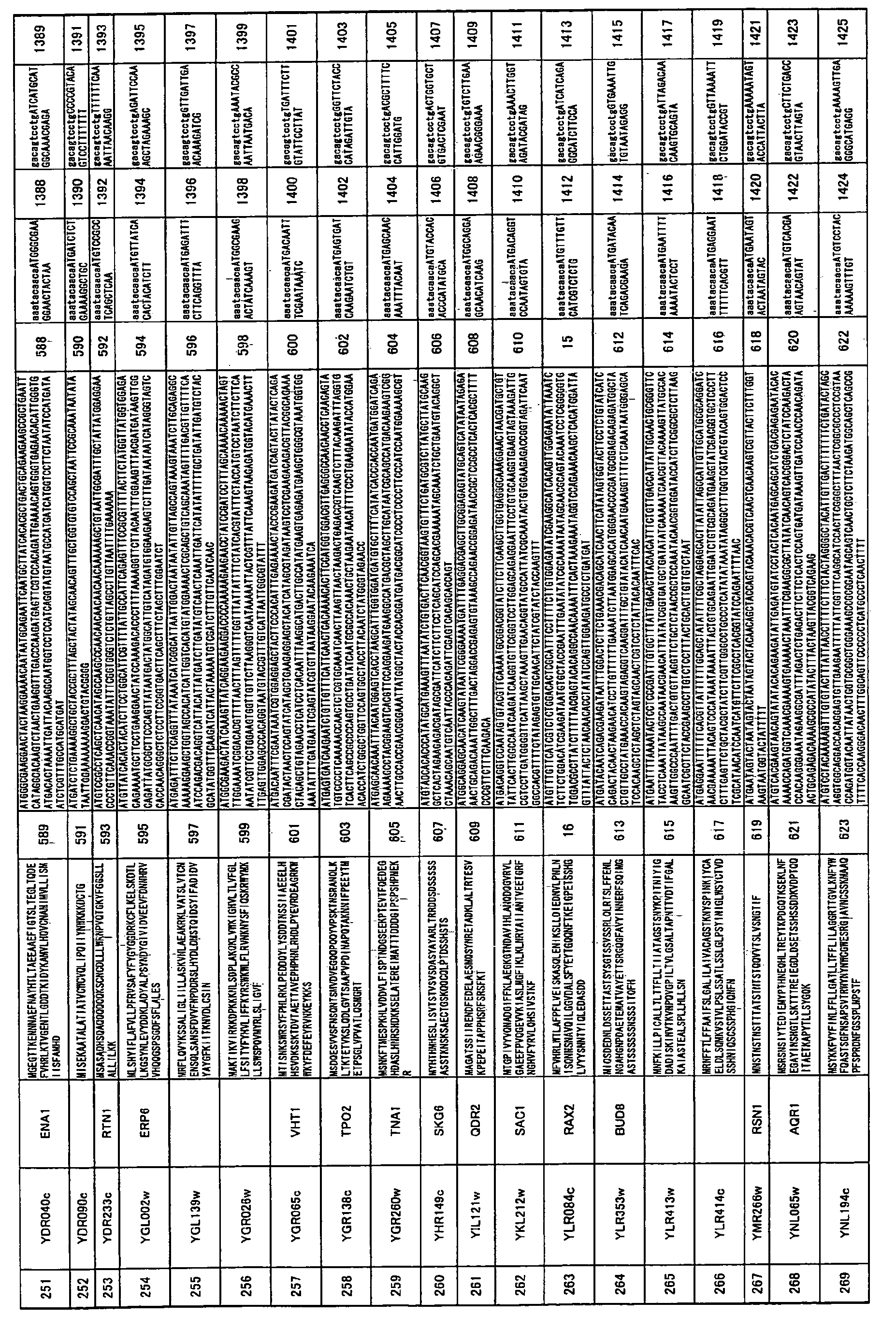

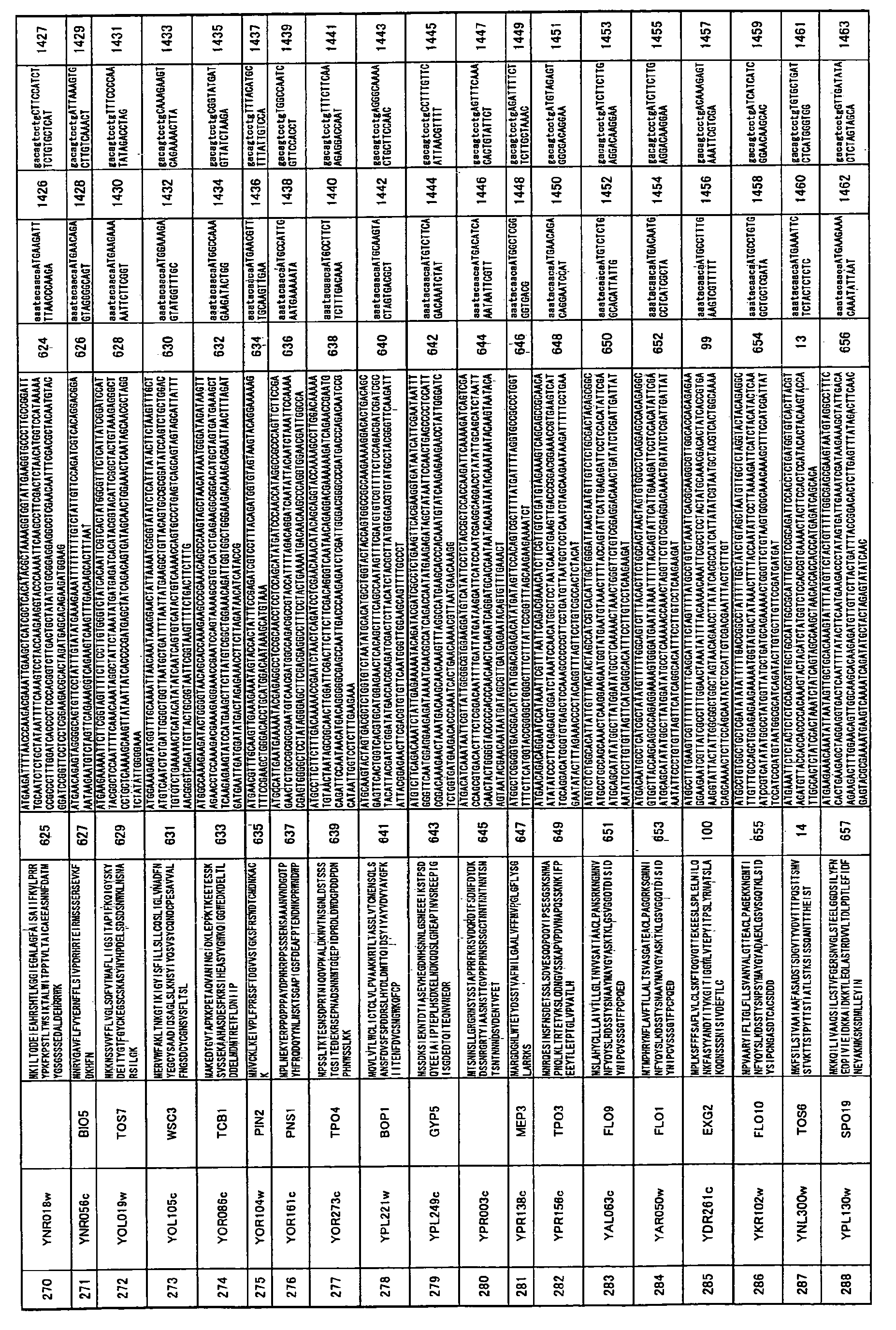

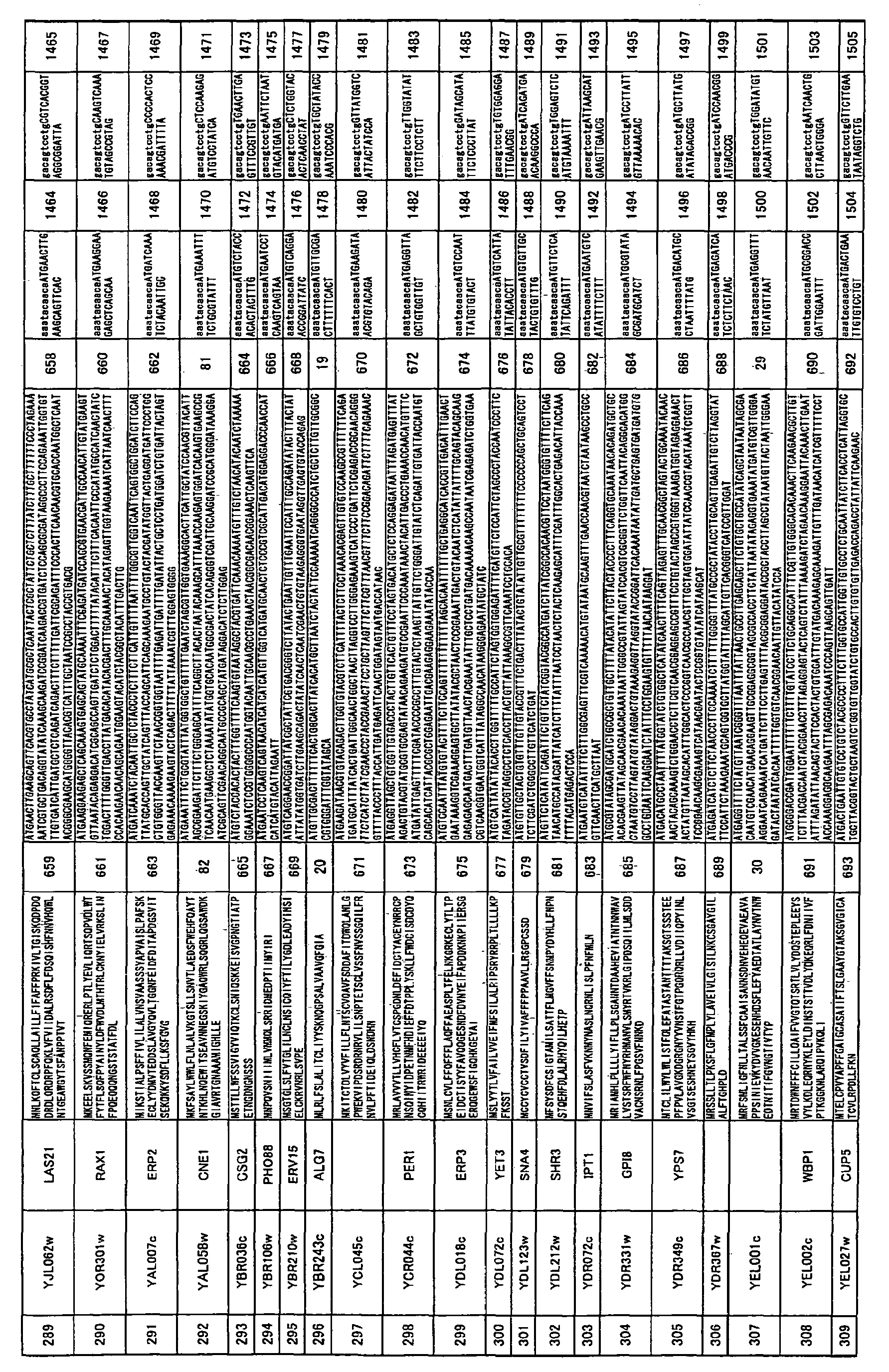

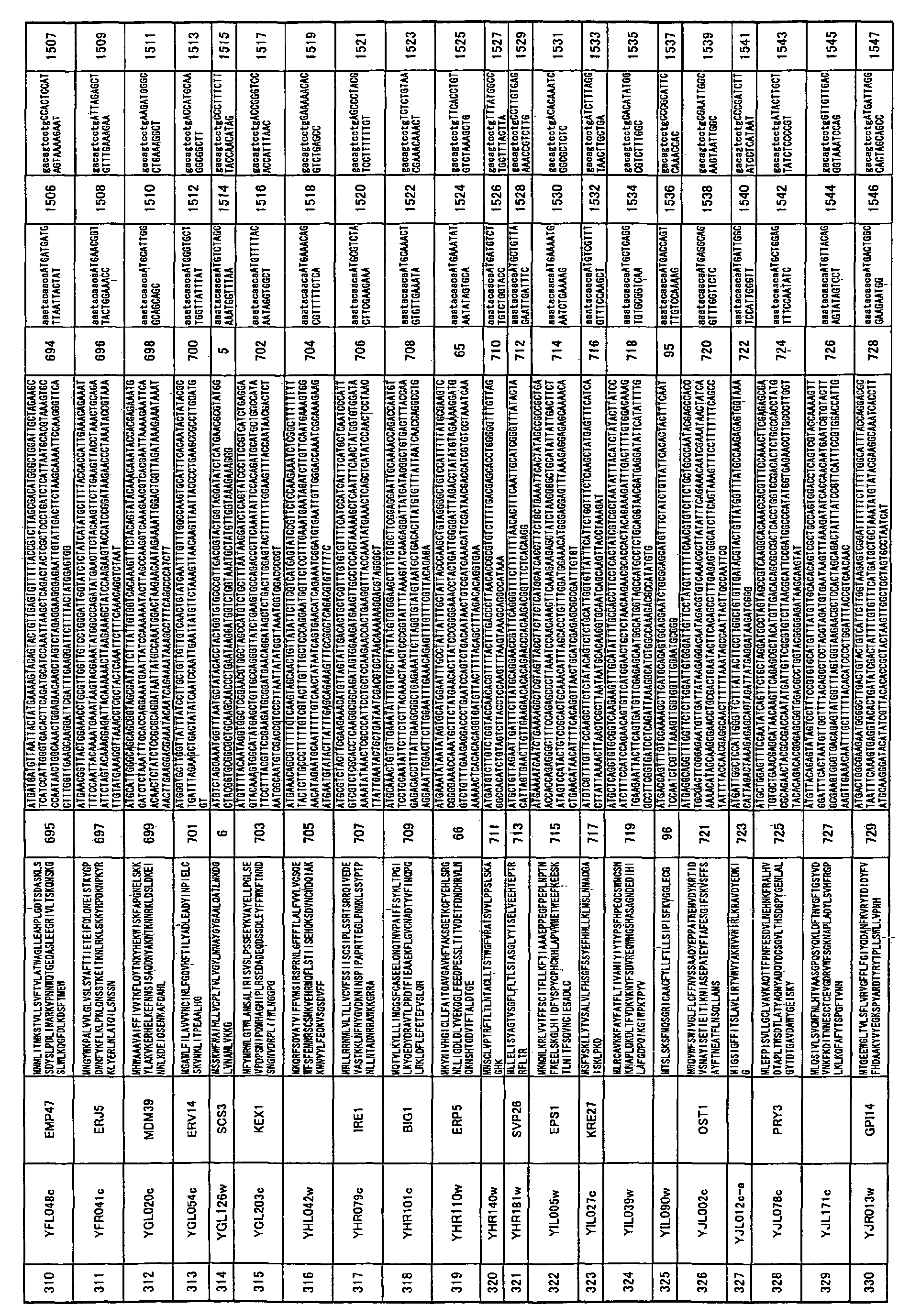

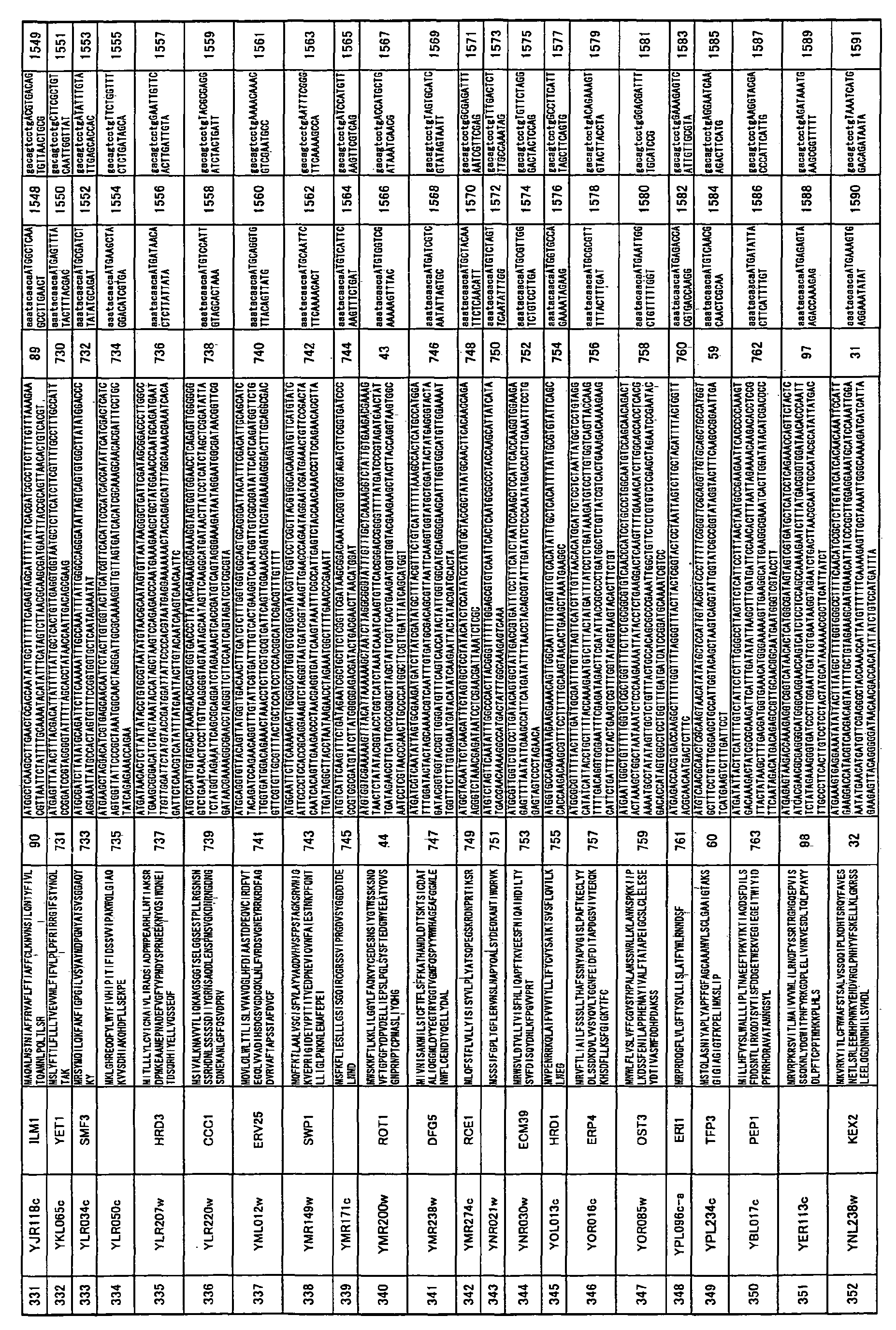

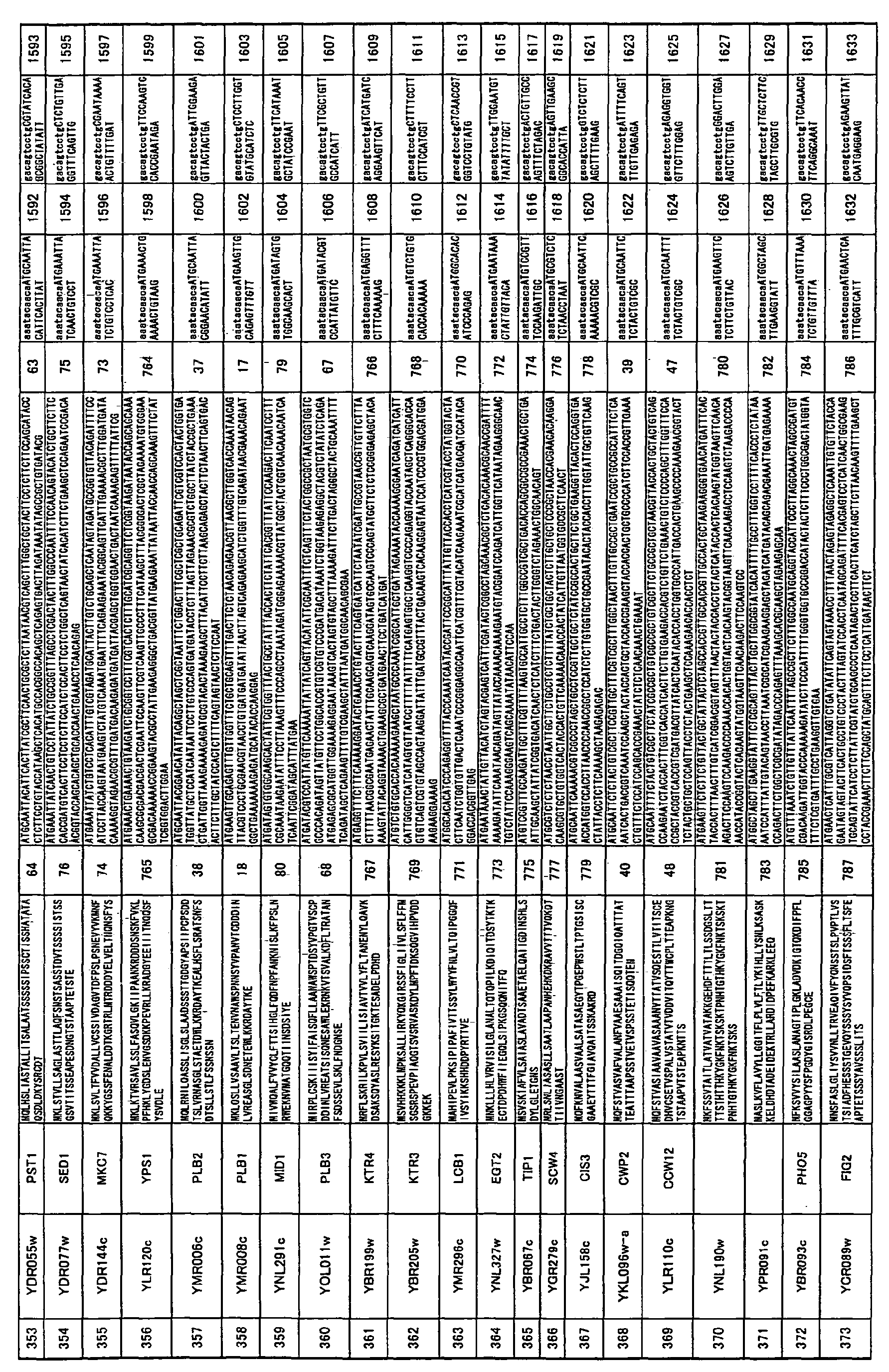

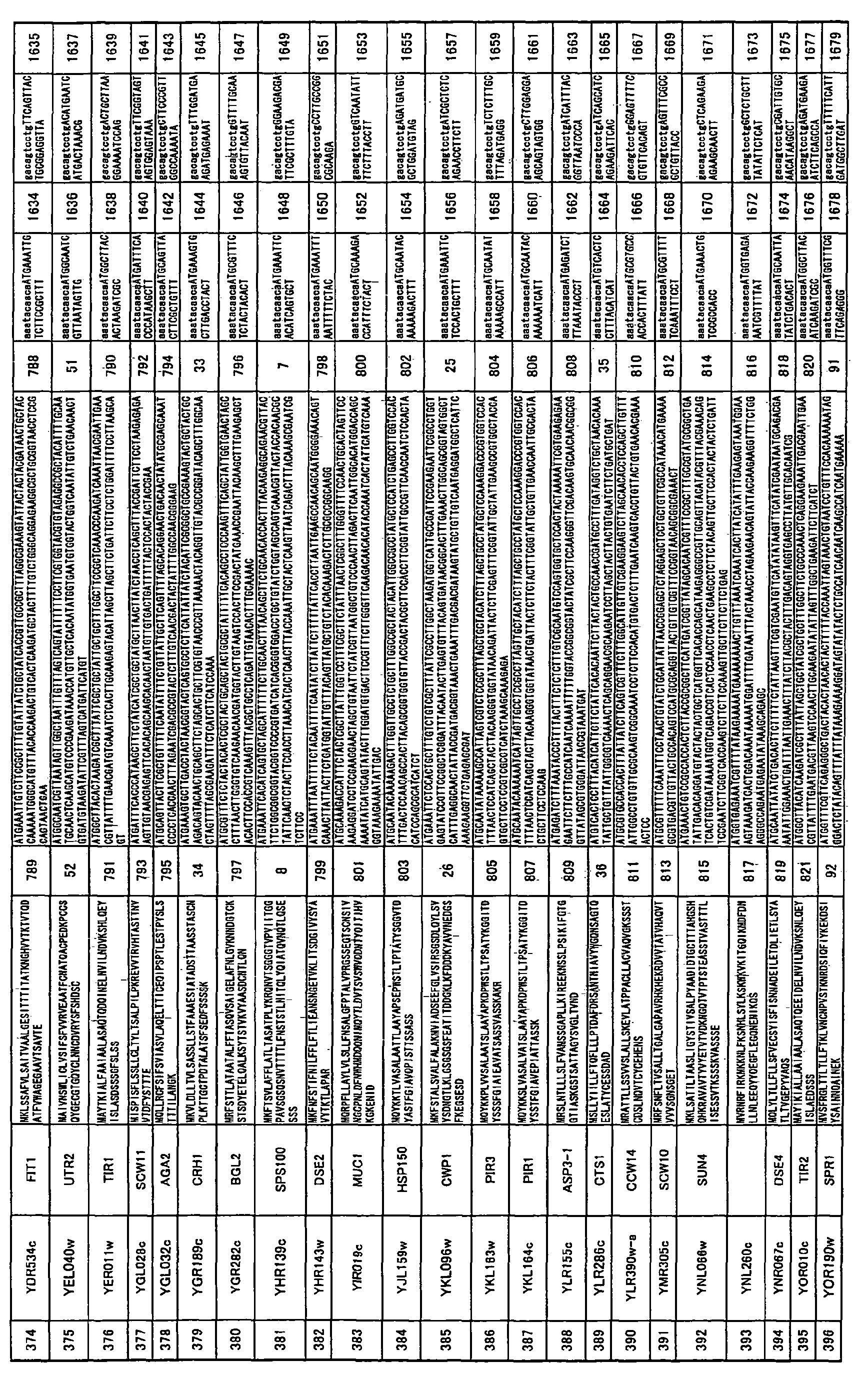

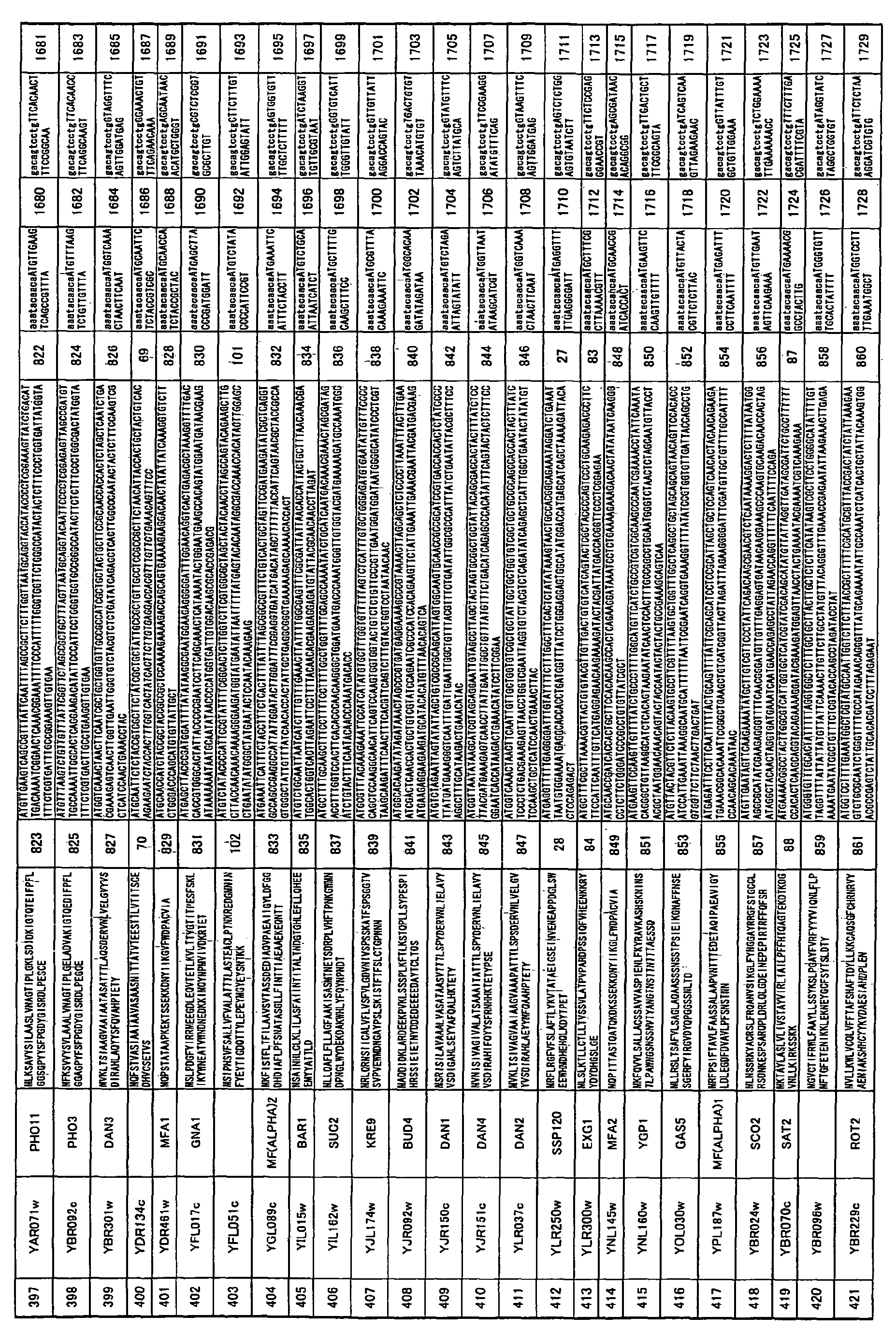

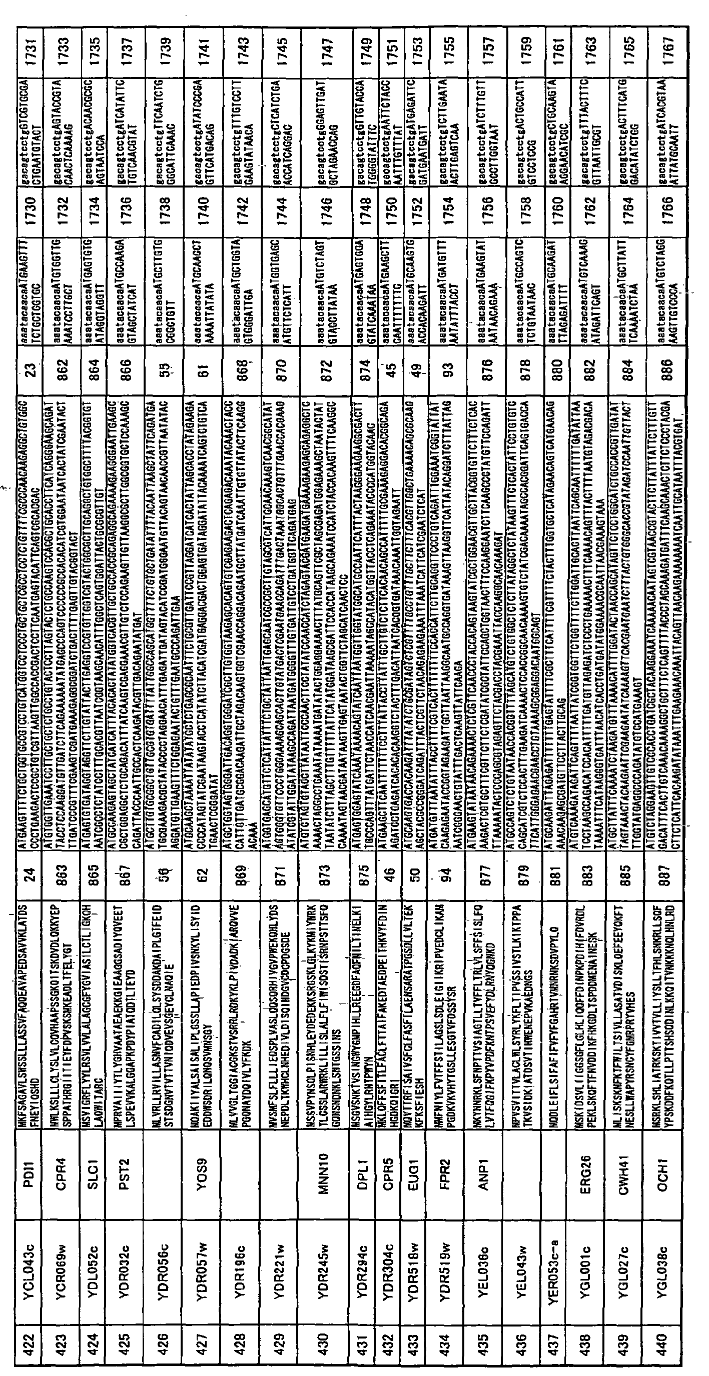

The gene regions encoding secretory signal peptides shown in Table 3 were amplified via PCR from the Saccharomyces cerevisiae genomic DNA. The sequences of the synthetic primers for the gene regions encoding secretory signal peptides used in PCR are as shown in Table 3. Each forward primer comprises at its 5' end a sequence complementary to a 10-bp region encompassing the 3' end of the HSP12 promoter contained in the pCLuRA-s plasmid. Each- reverse primer comprises at its 5' end a sequence complementary to a 10-bp region encompassing the 5' end of the mature. CLuc gene.

PCR was carried out using 50 µl of a reaction solution comprising 300 nM of each primer, 1 mM MgSO₄, 200 µM -dNTP, 1 ng of yeast genomic DNA (derived from the Saccharomyces cerevisiae S288C strain, Invitrogen), and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 1 minute (elongation); and the third step at 68°C for 5 minutes. Thus, DNA fragments independently comprising at both of its ends 10-bp regions complementary to the HSP12 promoter and the mature CLuc gene contained in the pCLuRA-s plasmid and gene regions encoding secretory signal peptides were obtained. Hereafter, these- DNA fragments are referred to as "1st PCR products."

As the controls, a DNA fragment having at both of its ends 10-bp regions complementary to the HSP 12 promoter and the mature CLuc gene and a gene region (nucleotide sequence: SEQ ID NO: 1768; amino acid sequence: SEQ ID NO: 1769) encoding the α-factor-derived secretory signal peptide (hereafter referred to as "DNA fragment E") or a gene region (nucleotide sequence: SEQ ID NO: 1770; amino acid sequence: SEQ ID NO: 1771) encoding secretory signal peptide derived from yeast virus (M28 virus) toxin (K28 prepro-toxin) (hereafter referred to as "DNA fragment F") used in conventional expression systems in yeast, as with the 1st PCR products, was prepared in the following manner.

DNA fragment E was amplified via PCR from the pCLuRA plasmid (see International Application Number: PCT/JP2006/311597, claiming the priority right of JP Patent Application No. 2005-169768).

The following synthetic primers were used. MF(ALPHA)1 Sig. F comprises at its 5' end a sequence complementary to a 10-bp region encompassing the 3' end of the HSP 12 promoter contained in the pCLuRA-s plasmid. Also, MF(ALPHA)1 Sig. R comprises at its 5' end a sequence complementary to a 10-bp region encompassing the 5' end of the mature CLuc gene.

MF(ALPHA)1 Sig. F: aaatacaaca-ATGAGATTTCCTTCAATTTT (SEQ
ID NO: 1772)

MF(ALPHA)1 Sig. R: gacagtcctg-AGCTTCAGCCTCTCTTTTCT (SEQ
ID NO: 1773)

PCR was carried out using 50 µl of a reaction solution comprising 300 nM of each primer, 1 mM MgSO₄, 200 µM dNTP, 1 ng of the pCLuRA plasmid, and 1x buffer and KOD-Plus-1U included in the KOD-Plus-. The PCR cycle comprised: the first -step- at 94°C for 2 minutes; 30 cycles of the second step at 94°C for 15 seconds- (denaturation), at 50°C for 30 seconds (annealing), and at 68°C for 1 minute (elongation); -and the third step at 68°C for 5 minutes. Thus, a DNA fragment E having at both of its ends 10-bp regions complementary to the HSP12 promoter and the mature CLuc gene contained in the pCLuRA-s plasmid and the gene regions encoding α-factor-derived secretory signal peptides, as with the 1st PCR product, was obtained.

DNA fragment F was prepared in the following manner.

A low-temperature-inducible expression vector, the aforementioned pLTex321s vector (see Patent Document 2), was cleaved with XhoI and SphI, a DNA fragment was fractionated via agarose gel electrophoresis, and a vector fragment of approximately 7.3 kbp was obtained. This vector fragment is hereafter referred to as "DNA fragment G."

Subsequently, the following synthetic DNAs were prepared in order to introduce tags for purifying the expressed proteins, i.e., the 6x His tag and the V5 antigen tag, into DNA fragment G.

V5-H tag F:
TCGAGGGTAAGCCTATCCCTAACCCTCTCCTCGGTCTCGATTCTACGCGT
-ACCGGTCATCATCACCATCACCATTGAGCATG (SEQ ID NO: 1774)

V5-H tag R:
CTCAATGGTGATGGTGATGATGACCGGTACGCGTAGAATCGAGACCGAG
GAGAGGGTTAGGGATAGGCTTACCC (SEQ ID NO: 1775)

These synthetic DNAs were annealed, the double-stranded synthetic DNA was ligated to DNA fragment G using the DNA Ligation Kit ver. 2.1, and the ligation product was introduced into E. coli DH5α.

The resulting transformant was cultured overnight, plasmids were extracted using the GenElute Plasmid Miniprep Kit, and a transformant having a plasmid of interest was identified based on the restriction enzyme cleavage pattern and via nucleotide sequence analysis. The pLTex321sV5H vector was prepared from the transformant.

The resulting vector, pLTex321sV5H, was cleaved with SmaI and EcoRI, a DNA fragment was fractionated via agarose gel electrophoresis, and a vector fragment of approximately 7.4 kbp was obtained. This vector fragment is hereafter referred to as "DNA fragment H."

Subsequently, the following synthetic DNAs were prepared in order to introduce the gene region encoding the preprotoxin-derived secretory signal peptide into DNA fragment H.

K28 PPT Sig. F:
ATGGAATCTGTTTCTTCTTTGTTTAATATTTTTCTACTATTATGGTTAATT
ATAAATCTTTGGTTTTGG (SEQ ID NO: 1776)

K28 PPT Sig. R:
GGAATTCCTGCAGCCCGGGCAAATTAGAAACAGACAACAAAGCCAAAAC
CAAAGATTTATAATTAACCAT (SEQ ID NO: 1777)

These synthetic DNAs were annealed, and the 3' ends of the strands of the annealed double-stranded synthetic DNA were subjected to elongation using the Klenow fragment (TOYOBO). Thereafter, the resulting DNA fragment was cleaved with EcoRI, the cleavage product was- ligated to DNA fragment H using the DNA Ligation Kit ver. 2.1, and the resultant was introduced into E. coli DH5α.

The resulting transformant was cultured overnight, plasmids were extracted using the GenElute Plasmid Miniprep Kit, and a transformant having a plasmid of interest was identified based on the restriction enzyme cleavage pattern and via nucleotide sequence analysis. The pLTex321sV5H K28 vector was prepared from the transformant.

Further, the 3' end of the gene region encoding the preprotoxin-derived secretory signal peptide, which had been introduced into the pLTex321sV5H K28 vector, was elongated via PCR.

The following synthetic primers were used. K28 Sig. F is a 24-bp region encompassing the 5' end of the gene region encoding the preprotoxin-derived secretory signal peptide in the pLTex321sV5H K28 vector. K28 Sig. R (36) comprises a DNA sequence complementary to 25-bp region encompassing the 3' end of the gene region encoding the preprotoxin-derived secretory signal peptide in the pLTex321sV5H K28 vector, a DNA sequence encoding 5 amino acid residues to be elongated, and a DNA sequence containing SmaI, PstI, and EcoRI restriction enzyme cleavage sites.

K28 Sig. F: ATGGAATCTGTTTCTTCTTTGTTT (SEQ ID NO: 1778)

K28 Sig. R (36): GGAATTCCTGCAGCCCGGG-
ACCTCTAGCATATTT-CAAATTAGAAACAGACAACAAAGCC (SEQ ID NO:
1779)

PCR was carried out using 50 µl of a reaction solution comprising 300 nM of each primer, 1 mM MgSO₄, 200 µM dNTP, 1 ng of the pLTex321sV5H K28 vector, and 1x buffer and KOD-Plus-1U included in the KOD-Plus-. The PCR cycle comprised: the first step at 94°C for 2 minutes; 30 cycles of the second step at 94°C for 15 seconds (denaturation), at 50°C for 30 seconds (annealing), and at 68°C for 30 seconds (elongation); and the third step at 68°C for 5 minutes.

The DNA fragment obtained via such PCR was treated with EcoRI, ligated to DNA fragment H using the DNA Ligation Kit ver. 2.1, and introduced into E. coli DH5α.

The resulting transformant was cultured overnight, plasmids were extracted using the GenElute Plasmid Miniprep Kit, and -a transformant having a plasmid of interest was identified based on the restriction enzyme cleavage pattern and via nucleotide sequence analysis. The pLTex321sV5H K28L vector was prepared from the transformant.

The pLTex321 sV5H K28L vector was used as a template, and a DNA fragment (DNA fragment F) containing a gene region encoding the preprotoxin-derived secretory signal peptide was amplified via PCR. The following synthetic primers were used. K28L Sig. F comprises at its 5' end- a sequence complementary to a 10-bp region encompassing the 3' end of the HSP12 promoter contained in the pCLuRA-s plasmid. Also, K28L Sig. R comprises at its 5' end a sequence complementary to a 10-bp region encompassing -the 5' end of the mature CLuc gene.
K28L Sig. F: aaatacaaca-ATGGAATCTGTTTCTTCTTT (SEQ ID NO: 1780)
K28L Sig. R: gacagtcctg-ACCTCTAGCATATTTCAAAT (SEQ ID NO: 1781)

PCR was carried out using 50 µl of a reaction solution comprising 300 nM of each primer, 1 mM MgSO₄, 200 µM dNTP, 1 ng of the pLTex321sV5H K28L vector, and 1x buffer and KOD-Plus-1U included in the KOD-Plus-. The PCR cycle comprised: the first step at 94°C for 15 seconds; 30 cycles of the second step at 94°C for 15 seconds (denaturation), at 50°C for 30 seconds (annealing), and at 68°C for 1 minute (elongation); and the third step at 68°C for 5 minutes. Thus, a DNA fragment (DNA fragment F) having at both of its ends 10-bp regions complementary to the HSP12 promoter and the mature CLuc gene contained in the pCLuRA-s plasmid and the gene region encoding the preprotoxin-derived secretory signal peptide, as with the 1st PCR product, was obtained.

In order to elongate the regions complementary to the pCLuRA-s plasmids at both ends of the DNA fragment containing the gene region encoding each secretory signal peptide, amplification was further carried out via PCR.

The following synthetic primers were used. 2nd PCR F comprises a sequence complementary to a 50-bp region encompassing the 3' end of the HSP12 promoter region. 2nd PCR R comprises a sequence complementary to a 50-bp region encompassing the 5' end of the mature CLuc gene.

2nd PCR F:
TTCGATAATCTCAAACAAACAACTCAAAACAAAAAAAACT-
AAATACAACA (SEQ ID NO: 1782)

2nd PCR R:
CAGGAAGTTGGAACTGTGTTTGGTGGATCAGGTTCGTAAG-
GACAGTCCTG (SEQ ID NO: 1783)

PCR was carried out using 50 µl of a reaction solution comprising 300 nM of each primer, 1 mM MgSO₄, 200 µM dNTP, a 10-fold diluted 1st PCR product, 1 µl of DNA fragment E or DNA fragment F, and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 1 minute (elongation); and the third step at 68°C for 5 minutes. Thus, a DNA fragment having at both of its ends 50-bp regions complementary to the HSP12 promoter and the mature CLuc gene contained in the pCLuRA-s plasmid and the gene region encoding each of 440 types of secretory signal peptides, a DNA fragment comprising a gene region encoding the α-factor-derived secretory signal peptide, or a DNA fragment comprising a gene region encoding the preprotoxin-derived secretory signal peptide was obtained. Hereafter, these DNA fragments are referred to as "2nd PCR products."

### [Example 3] Construction of secretory signal peptide library

Using the 2nd PCR products obtained in Example 2 and the reporter vector, pCLuRA-s, prepared in Example 1, the secretory signal peptide library using the Saccharomyces cerevisiae host was constructed in the following manner.

The pCLuRA-s plasmid was cleaved with BamHI and HindIII, a DNA fragment was fractionated via agarose gel electrophoresis, and a DNA fragment of approximately 7.3 kbp was obtained. Hereafter, this DNA fragment is referred to as "DNA fragment I."

As the host of this library the Saccharomyces cerevisiae BY4743 PEP4Δ PRB1Δ strain was used. The BY4743 PEP4Δ PRB1Δ strain was prepared by producing the BY4741 PEP4Δ PRB1Δ strain and the BY4742 PEP4Δ PRB1Δ strain in which the PEP4 and PRB1 genes encoding major protease in the Saccharomyces cerevisiae BY4741 strain (Invitrogen) and the BY4742 strain (Invitrogen) had been disrupted by the method of Hegemann et al. (http://mips.gsf.de/proj/yeast/info/tools/hegemann/loxp_kanmx.html), and coupling these strains.

Using, as a template, the pUG6 plasmid (http://mips.gsf.de/proj/yeast/info/tools/hegemann/loxp_kanmx.html), a DNA fragment that is necessary for disrupting the PEP4 gene was first amplified via PCR. The following synthetic primers were used. dPEP4 kan F has at its 5' end a sequence complementary to a 40-bp upstream region of the PEP4 gene and at its 3' end a sequence complementary to a 19-bp upstream region of the 10xP-kanMX-1oxP module region of pUG6. dPEP4 kan R has at its 5' end a sequence complementary to a 40-bp downstream region of the PEP4 gene and at its 3' end a sequence complementary to a 22-bp downstream region of the loxP-kanMX-loxP module region of pUG6.

dPEP4 kan F:
ATTTAATCCAAATAAAATTCAAACAAAAACCAAAACTAAC-
CAGCTGAAGCTTCGTACGC (SEQ ID NO: 1784)

dPEP4 kan R:
GGCAGAAAAGGATAGGGCGGAGAAGTAAGAAAAGTTTAGC-
GCATAGGCCACTAGTGGATCTG (SEQ ID NO: 1785)

PCR was carried out using 50 µl of a reaction solution comprising 300 nM of each primer, 1 mM MgSO₄, 200 µM dNTP, 1 ng of the pUG6 plasmid, and 1x buffer and KOD-Plus-1U included in the KOD-Plus-. The PCR cycle comprised: the first step at 94°C for 15 seconds; 30 cycles of the second step at 94°C for 15 seconds (denaturation), at 50°C for 30 seconds (annealing), and at 68°C for 2 minutes (elongation); and the third step at 68°C for 5 minutes. Thus, a DNA fragment having at both of its ends sequences complementary to 40-bp upstream and downstream regi-ons of the PEP4 gene and containing the 1oxP-kanMX-1oxP module region was obtained. This DNA fragment was used to disrupt the PEP4 genes of the BY4741 strain and the BY4742 strain by the method of Hegemann et al. to obtain the BY4741 PEP4Δ strain and the BY4742 PEP4Δ strain.

Further, a DNA fragment, which is necessary for disrupting the PRB 1 gene, was prepared via PCR. The following synthetic primers were used. dPRB1 kan F has at its 5' end a sequence complementary to a 40-bp upstream region of the PRB1 gene and at its 3' end a sequence complementary to a 19-bp upstream region of the loxP-kanMX-loxP module region of pUG6. dPRB1 kan R has at its 5' end a sequence complementary to a 40-bp downstream region of the PRB1 gene and at its 3' end a sequence complementary to a 22-bp downstream region of the loxP-kanMX-loxP module region of pUG6.

dPRB1 kan F:
CAATAAAAAAACAAACTAAACCTAATTCTAACAAGCAAAG-
CAGCTGAAGCTTCGTACGC (SEQ ID NO: 1786)

dPRB1 kan R:
AAGAAAAAAAAAAGCAGCTGAAATTTTTCTAAATGAAGAA-
GCATAGGCCACTAGTGGATCTG (SEQ ID NO: 1787)

PCR was carried out using 50 µl of a reaction solution comprising 300 nM of each primer, 1 mM MgSO₄, 200 µM dNTP, 1 ng of the pUG6 plasmid, and 1x buffer and KOD-Plus-1U included in the KOD-Plus-. The PCR cycle comprised: the first step at 94°C for 15 seconds; 30 cycles of the second step at 94°C for 15 seconds (denaturation), at 50°C for 30 seconds (annealing), and at 68°C for 2 minutes (elongation); and the third step at 68°C for 5 minutes. Thus, a DNA fragment having at both of its ends sequences complementary to 40-bp upstream and downstream regions of the PRB1 gene and the loxP-kanMX-loxP module region was obtained.

This DNA fragment was used to disrupt the PRB1 genes of the BY4741 PEP4Δ strain and the BY4742 PEP4Δ strain by the method of Hegemann et al. to obtain the BY4741 PEP4Δ PRB1Δ strain and the BY4742 PEP4Δ PRB1Δ strain.

The BY4741 PEP4Δ PRB1Δ strain was mated to the BY4742 PEP4Δ PRB1Δ strain by a conventional technique to obtain the BY4743 PEP4Δ PRB1Δ diploid strain.

Transformation was carried out using the 2nd PCR products of the BY4743 PEP4Δ PRB1Δ strain and DNA fragment I in the following manner.

The BY4743 PEP4Δ PRB1Δ strain was cultured in a YPD medium (1% yeast extract, 2% peptone, and 2% glucose) at 30°C to a stationary phase, the strain was recovered from 250 µl of the culture solution via centrifugation, the strain was washed with 100 µl of sterile water, and the strain was further recovered via centrifugation.

The obtained strain was suspended in 60 µl of a yeast transformation solution (33.3% polyethylene glycol (PEG) 4,000, 100 mM of lithium acetate, 250 µg/ml of carrier DNA (Clontech), 10 ng of DNA fragment I, and 2 µl of each 10 fold diluted 2nd PCR product) and then cultured at 30°C for 30 minutes. Thereafter, 6 µl of dimethyl sulfoxide (DMSO) was added, and culture was further ca-rried out at 42°C for an additional 1 hour. Thereafter, 1 ml of the SD+HL medium (0.67% yeast nitrogen base without amino acid, 2% glucose, 0.002% L-histidine HC1, and 0.01% L-leucine) was inoculated with 10 µl of the culture solution, and culture was carried out at 30°C for 3 days. Thus, homologous recombination of DNA fragment I and each 2nd PCR product took place in the host BY4743 PEP4Δ PRB1Δ strain. Under the control (downstream) of the HSP12 promoter, the BY4743 PEP4Δ PRB1Δ strain sustaining a reporter plasmid in which the gene region encoding each secretory signal peptide has been fused to the 5' terminus of the mature CLuc gene was selectively allowed to grow by introducing a uracil requirement. Thus, the secretory signal peptide library utilizing the Saccharomyces cerevisiae host was constructed.

Some of the resulting strains were subjected to sequence analysis of reporter plasmids, and the generation of the plasmid of interest was confirmed in the yeast cell.

### [Example 4] Evaluation of the secretion ability of the secretory signal peptide

With the use of the secretory signal peptide library using the Saccharomyces cerevisiae host constructed in Example 3, the secretion ability of the secretory signal peptide was evaluated with the use of mature CLuc (secreted luciferase) in the following manner.

One ml of SD+HL+PPB medium (0.67% yeast nitrogen base without amino acid, 2% glucose, 0.002% L-histidine HCI, 0.01% L-leucine, and 200 mM potassium phosphate buffer (pH 6.0)) was inoculated with the Saccharomyces cerevisiae (the secretory signal peptide library) sustaining a reporter plasmid containing the gene region encoding each secretory signal peptide prepared in Example 3, and culture was conducted at 30°C for 3 days. This culture solution was designated as the preculture, and part thereof was used in the following culture.

One ml of SD+HL+PPB medium was inoculated with the culture solution (10 µl) obtained via the preculture and culture was conducted at 30°C to the logarithmic growth phase.

Using the culture solution that had been cultured until the logarithmic growth phase, activity of the mature CLuc (secreted luciferase) secreted in the culture was assayed to evaluate the secretion ability of each secretory signal peptide.

Activity of luciferase was assayed in the following manner.

To the culture solution (20 µl), 80 µl of a 2.5 µM luciferin solution was added, and, 2 seconds thereafter, the luminescence level was assayed for 5 seconds. Simultaneously, 200 µl of the culture solution was used to assay the absorbance thereof at 600 nm, and the luminescence level was divided by the obtained value to normalize the luciferase activity value based on the absorbance.

The results are shown in Table 4. Table 4 shows the relative secretion efficiency of the secretory signal peptides (indicated in terms of systematic and common names of the genes from which these secretory signal peptides have been derived) at moderate culture temperature (30°C) in relation to that of the α-factor-derived secretory signal peptide. The secretory signal peptides used in conventional secretory expression systems are shaded. The result of assaying the secretion efficiency of the α-factor-derived secretory signal peptide is shown in the column indicated as "α-factor" in terms of common names. The result of assaying the secretion efficiency of the preprotoxin-derived secretory signal peptide is shown in the column indicated as "K28L" in terms of common names.

### Table 4

**Table 4: Secretion efficiency of each of secretory signal peptides at moderate culture temperature (30° C)**

| Systematic gene name | Common gene name | Secretion efficiency relative to α-factor-derived secretory signal peptide |
|---|---|---|
| YDR420w | HKR1 | 5.64 |
| YBR187w | | 3.37 |
| YGL126w | SCS3 | 3.27 |
| YHR139c | SPS100 | 2.69 |
| YGR014w | MSB2 | 2.66 |
| YBR078w | ECM33 | 2.48 |
| YNL300w | TOS6 | 2.46 |
| YLR084c | RAX2 | 2.27 |
| YMR008c | PLB1 | 2.04 |
| YCR061w | | 2.00 |
| YDR055w | PST1 | 1.91 |
| YLR110c | CCW12 | 1.85 |
| YNL160w | YGP1 | 1.77 |
| YLR250w | SSP120 | 1.68 |
| YBR042c | CST26 | 1.65 |
| YDR134c | | 1.64 |
| YBR296c | PHO89 | 1.54 |
| YLR034c | SMF3 | 1.51 |
| YBR243c | ALG7 | 1.49 |
| YLR332w | MID2 | 1.44 |
| YDR077w | SED1 | 1.41 |
| YPL234c | TFP3 | 1.34 |
| YDR261c | EXG2 | 1.32 |
| YBR070c | SAT2 | 1.31 |
| YGR189c | CRH1 | 1.27 |
| YKL096w-a | CWP2 | 1.25 |
| YLR300w | EXG1 | 1.25 |
| YIL140w | AXL2 | 1.25 |
| YHR181w | SVP26 | 1.24 |
| YEL001c | | 1.23 |
| YCL043c | PDI1 | 1.19 |
| YGL032c | AGA2 | 1.19 |
| YNL237w | YTP1 | 1.17 |
| YIL090w | | 1.07 |
| YDR518w | EUG1 | 1.07 |
| YEL040w | UTR2 | 1.02 |
| YOR190w | SPR1 | 1.02 |
| | α-factor | 1.00 |
| YHR079c | IRE1 | 0.98 |
| YBR293w | | 0.96 |
| YNR044w | AGA1 | 0.92 |
| YFL041w | FET5 | 0.92 |
| YNL066w | SUN4 | 0.89 |
| YCR028c | FEN2 | 0.87 |
| YNL327w | EGT2 | 0.86 |
| YNR067c | DSE4 | 0.85 |
| YKL209c | STE6 | 0.78 |
| YNL190w | | 0.78 |
| YAL058w | CNE1 | 0.77 |
| YPR124w | CTR1 | 0.77 |
| YCR017c | CWH43 | 0.76 |
| YDR205w | MSC2 | 0.76 |
| YJL078c | PRY3 | 0.74 |
| YCR011c | ADP1 | 0.71 |
| YNL322c | KRE1 | 0.71 |
| YKL163w | PIR3 | 0.69 |
| YER145c | FTR1 | 0.68 |
| YER113c | | 0.65 |
| YDR056c | | 0.64 |
| YDR304c | CPR5 | 0.63 |
| YIL162w | SUC2 | 0.62 |
| YLR286c | CTS1 | 0.62 |
| YER118c | SHO1 | 0.61 |
| YMR006c | PLB2 | 0.61 |
| YOL011w | PLB3 | 0.61 |
| YPL189w | GUP2 | 0.60 |
| YDR057w | YOS9 | 0.59 |
| YDR144c | MKC7 | 0.58 |
| YNL219c | ALG9 | 0.56 |
| YLR050c | | 0.54 |
| YKL096w | CWP1 | 0.53 |
| YMR307w | GAS1 | 0.53 |
| YCL027w | FUS1 | 0.53 |
| YNL238w | KEX2 | 0.52 |
| YGL038c | OCH1 | 0.52 |
| YMR058w | FET3 | 0.51 |
| YDR349c | YPS7 | 0.51 |
| YLR207w | HRD3 | 0.50 |
| YFL051c | | 0.50 |
| YJL159w | HSP150 | 0.50 |
| YAR071w | PH011 | 0.47 |
| | K28L | 0.47 |
| YDL072c | YET3 | 0.43 |
| YBR093c | PHO5 | 0.41 |
| YDR276c | PMP3 | 0.40 |
| YAL053w | | 0.40 |
| YHR110w | ERP5 | 0.40 |
| YIL039w | | 0.39 |
| YMR200w | ROT1 | 0.38 |
| YDR519w | FPR2 | 0.38 |
| YCR021c | HSP30 | 0.37 |
| YFL026w | STE2 | 0.37 |
| YOR008c | SLG1 | 0.37 |
| YGL089c | MF(ALPHA)2 | 0.37 |
| YPL187w | MF(ALPHA)1 | 0.36 |
| YJR118c | ILM1 | 0.36 |
| YNL291c | MID1 | 0.35 |
| YDR367w | | 0.35 |
| YEL004w | YEA4 | 0.34 |
| YNL194c | | 0.34 |
| YAL063c | FLO9 | 0.32 |
| YHR143w | DSE2 | 0.32 |
| YNR030w | ECM39 | 0.31 |
| YBR183w | YPC1 | 0.29 |
| YGL020c | MDM39 | 0.28 |
| YIL027c | KRE27 | 0.28 |
| YDR536w | STL1 | 0.27 |
| YGR279c | SCW4 | 0.26 |
| YGL027c | CWH41 | 0.26 |
| YKL164c | PIR1 | 0.26 |
| YBR092c | PHO3 | 0.26 |
| YFR041c | ERJ5 | 0.25 |
| YKL221w | MCH2 | 0.25 |
| YPL130w | SPO19 | 0.25 |
| YAL007c | ERP2 | 0.25 |
| YBR229c | ROT2 | 0.25 |
| YJL193w | | 0.24 |
| YLR155c | ASP3-1 | 0.24 |
| YDR221w | | 0.24 |
| YDL212w | SHR3 | 0:21 |
| YEL036c | ANP1 | 0.19 |
| YLR413w | | 0.17 |
| YLR120c | YPS1 | 0.17 |
| YAR050w | FLO1 | 0.17 |
| YJL174w | KRE9 | 0.16 |
| YMR171c | | 0.16 |
| YDL093w | PMT5 | 0.16 |
| YKL187c | | 0.16 |
| YGR282c | BGL2 | 0.15 |
| YKL051w | SFK1 | 0.15 |
| YBR067c | TIP1 | 0.15 |
| YMR119w | ASI1 | 0.15 |
| YJL158c | CIS3 | 0.15 |
| YJL051w | | 0.15 |
| YLR037c | DAN2 | 0.15 |
| YLR390w-a | CCW14 | 0.14 |
| YGL028c | SCW11 | 0.14 |
| YDR534c | FIT1 | 0.14 |
| YBR301w | DAN3 | 0.14 |
| YNR018w | | 0.14 |
| YEL002c | WBP1 | 0.14 |
| YPR091c | | 0.13 |
| YLR121c | YPS3 | 0.13 |
| YIL147c | SLN1 | 0.12 |
| YOL105c | WSC3 | 0.12 |
| YMR149w | SWP1 | 0.12 |
| YCL045c | | 0.11 |
| YMR305c | SCW10 | 0.11 |
| YBL017c | PEP1 | 0.10 |
| YBR210w | ERV15 | 0.09 |
| YLR214w | FRE1 | 0.09 |
| YML116w | ATR1 | 0.09 |
| YML012w | ERV25 | 0.09 |
| YNL283c | WSC2 | 0.09 |
| YOR010c | TIR2 | 0.09 |
| YJL222w | VTH2 | 0.09 |
| YJR150c | DAN1 | 0.08 |
| YBR205w | KTR3 | 0.08 |
| YNL142w | MEP2 | 0.08 |
| YKL174c | | 0.07 |
| YGR213c | RTA1 | 0.07 |
| YMR243c | ZRC1 | 0.07 |
| YLR414c | | 0.07 |
| YCR069w | CPR4 | 0.07 |
| YBR036c | CSG2 | 0.07 |
| YER011w | TIR1 | 0.07 |
| YKL220c | FRE2 | 0.06 |
| YOL019w | TOS7 | 0.06 |
| YKL178c | STE3 | 0.06 |
| YLR087c | CSF1 | 0.06 |
| YJL002c | OST1 | 0.06 |
| YEL027w | CUP5 | 0.05 |
| YCR034w | FEN1 | 0.05 |
| YKL039w | PTM1 | 0.05 |
| YHR140w | | 0.05 |
| YBR054w | YRO2 | 0.05 |
| YKL034w | TUL1 | 0.05 |
| YOR149c | SMP3 | 0.05 |
| YPR201w | ARR3 | 0.05 |
| YBR241c | | 0.05 |
| YLR242c | ARV1 | 0.05 |
| YNR055c | HOL1 | 0.05 |
| YMR266w | RSN1 | 0.05 |
| YOR011w | AUS1 | 0.04 |
| YIL005w | EPS1 | 0.04 |
| YJR004c | SAG1 | 0.04 |
| YOL030w | GAS5 | 0.04 |
| YDR038c | ENA5 | 0.04 |
| YDR245w | MNN10 | 0.04 |
| YBR004c | FMP44 | 0.04 |
| YPL096c-a | ERI1 | 0.04 |
| YJL012c-a | | 0.04 |
| YHL016c | DUR3 | 0.04 |
| YLR459w | GAB1 | 0.04 |
| YDR384c | ATO3 | 0.03 |
| YDR040c | ENA1 | 0.03 |
| YAL067c | SEO1 | 0.03 |
| YEL017c-a | PMP2 | 0.03 |
| YGL255w | ZRT1 | 0.03 |
| YIL015w | BAR1 | 0.03 |
| YKR102w | FL010 | 0.03 |
| YOR104w | PIN2 | 0.02 |
| YJL171c | | 0.02 |
| YDL052c | SLC1 | 0.02 |
| YIR028w | DAL4 | 0.02 |
| YHL042w | | 0.02 |
| YBR040w | FIG1 | 0.02 |
| YHR101c | BIG1 | 0.02 |
| YIL170w | HXT12 | 0.02 |
| YDL018c | ERP3 | 0.02 |
| YOR085w | OST3 | 0.02 |
| YBR021w | FUR4 | 0.02 |
| YJR013w | GPI14 | 0.02 |
| YER053c-a | | 0.02 |
| YLR220w | CCC1 | 0.02 |
| YHL035c | VMR1 | 0.02 |
| YDR033w | MRH1 | 0.02 |
| YIR019c | MUC1 | 0.02 |
| YDL210w | UGA4 | 0.02 |
| YER072w | VTC1 | 0.02 |
| YEL065w | SIT1 | 0.02 |
| YCR024c-a | PMP1 | 0.02 |
| YHL036w | MUP3 | 0.02 |
| YGL203c | KEX1 | 0.02 |
| YJL062w | LAS21 | 0.02 |
| YIL013c | PDR11 | 0.02 |
| YDL035c | GPR1 | 0.01 |
| YCR044c | PER1 | 0.01 |
| YMR238w | DFG5 | 0.01 |
| YAL018c | | 0.01 |
| YML123c | PHO84 | 0.01 |
| YOL013c | HRD1 | 0.01 |
| YBR199w | KTR4 | 0.01 |
| YKL046c | DCW1 | 0.01 |
| YEL043w | | 0.01 |
| YKR039w | GAP1 | 0.01 |
| YKL004w | AUR1 | 0.01 |
| YDR046c | BAP3- | 0.01 |
| YDR072c | IPT1 | 0.01 |
| YJR040w | GEF1 | 0.01 |
| YCR037c | PHO87 | 0.01- |
| YLL061w | MMP1 | 0.01 |
| YJR151c | DAN4 | 0.01 |
| YKR040c | | 0.01 |
| YLL015w | BPT1 | 0.01 |
| YML038c | YMD8 | 0.01 |
| YGR121c | MEP1 | 0.01 |
| YPL232w | SSO1 | 0.01 |
| YDR331w | GPI8 | 0.01 |
| YPL265w | DIP5 | 0.01 |
| YGL008c | PMA1 | 0.01 |
| YBR069c | TAT1 | 0.01 |
| YDR090c | | 0.01 |
| YGR191w | HIP1 | 0.01 |
| YGR055w | MUP1 | 0.01 |
| YOL084w | PHM7 | 0.01 |
| YPR138c | MEP3 | 0.01 |
| YEL063c | CAN1 | 0.01 |
| YCR098c | GIT1 | 0.01 |
| YJR158w | HXT16 | 0.01 |
| YFL011w | HXT10 | 0.01 |
| YKL065c | YET1 | 0.01 |
| YGL002w | ERP6 | 0.01 |
| YGL139w | | 0.01 |
| YCL025c | AGP1 | 0.01 |
| YDL199c | | 0.01 |
| YBL089w | AVT5 | 0.01 |
| YDL245c | HXT15 | 0.01 |
| YCR023c | | 0.01 |
| YBL042c | FUI1 | 0.01 |
| YGR065c | VHT1 | 0.01 |
| YMR319c | FET4 | 0.01 |
| YNL318c | HXT14 | 0.01 |
| YHR096c | HXT5 | 0.01 |
| YFL050c | ALR2 | 0.01 |
| YMR183c | SSO2 | 0.01 |
| YDR093w | DNF2 | 0.01 |
| YDR011w | SNQ2 | 0.01 |
| YLR023c | IZH3 | 0.01 |
| YOL103w | ITR2 | 0.01 |
| YDR342c | HXT7 | 0.01 |
| YHR094c | HXT1 | 0.01 |
| YJR152w | DAL5 | 0.01 |
| YBR068c | BAP2 | 0.01 |
| YBR096w | | 0.01 |
| YJL214w | HXT8 | 0.01 |
| YNL280c | ERG24 | 0.01 |
| YJL219w | HXT9 | 0.01 |
| YKR093w | PTR2 | 0.01 |
| YLR237w | TH17 | 0.01 |
| YHR092c | HXT4 | 0.01 |
| YKR050w | TRK2 | 0.01 |
| YJR160c | MPH3 | 0.01 |
| YDR343c | HXT6 | 0.01 |
| YDL247w | MPH2 | 0.01 |
| YER060w | FCY21 | 0.01 |
| YOR328w | PDR10 | 0.01 |
| YNL048w | ALG11 | 0.01 |
| YJL129c | TRK1 | 0.01 |
| YBR038w | CHS2 | 0.01 |
| YDR345c | HXT3 | 0.01 |
| YGR260w | TNA1 | 0.01 |
| YBR086c | IST2 | 0.01 |
| YOR153w | PDR5 | 0.01 |
| YGR032w | GSC2 | 0.01 |
| YBR140c | IRA1 | 0.00 |
| YGL054c | ERV14 | 0.00 |
| YER056c | FCY2 | 0.00 |
| YJL093c | TOK1 | 0.00 |
| YKR053c | YSR3 | 0.00 |
| YEL069c | HXT13 | 0.00 |
| YGR281w | YOR1 | 0.00 |
| YIL047c | SYG1 | 0.00 |
| YBR023-c | CHS3 | 0.00 |
| YCL038c | AT.G22 | 0.00 |
| YDL138w | RGT2 | 0.00 |
| YDR497c | ITR1 | 0.00 |
| YKL100c | | 0.00 |
| YEL031w | SPF1 | 0.00 |
| YBR041w | FAT1 | 0.00 |
| YER166w | DNF1 | 0.00 |
| YDR032c | PST2 | 0.00 |
| YLR081w | GAL2 | 0.00 |
| YFL017c | GNA1 | 0.00 |
| YGR026w | | 0.00 |
| YKL083w | | 0.00 |
| YBR294w | SUL1 | 0.00 |
| YGL077c | HNM1 | 0.00 |
| YDR461w | MFA1 | 0.00 |
| YER008c | SEC3 | 0.00 |
| YPL221w | BOP1 | 0.00 |
| YNR039c | ZRG17 | 0.00 |
| YHR149c | SKG6 | 0.00 |
| YOL156w | HXT11 | 0.00 |
| YGL001c | ERG26 | 0.00 |
| YDL194w | SNF3 | 0.00 |
| YLR138w | NHA1 | 0.00 |
| YJL094c | KHA1 | 0.00 |
| YNL087w | TCB2 | 0.00 |
| YER060w-a | FCY22 | 0.00 |
| YOR016c | ERP4 | 0.00 |
| YNR013c | PHO91 | 0.00 |
| YPL092w | SSU1 | 0.00 |
| YAL022c | FUN26 | 0.00 |
| YNL268w | LYP1 | 0.00 |
| YPR003c | | 0.00 |
| YBR298c | MAL31 | 0.00 |
| YOL075c | | 0.00 |
| YMR011w | HXT2 | 0.00 |
| YCR048w | ARE1 | 0.00 |
| YNL270c | ALP1 | 0.00 |
| YGR138c | TPO2 | 0.00 |
| YOL122c | SMF1 | 0.00 |
| YDR294c | DPL1 | 0.00 |
| YMR274c | RCE1 | 0.00 |
| YOL158c | ENB1 | 0.00 |
| YDR126w | SWF1 | 0.00 |
| YOR002w | ALG6 | 0.00 |
| YLL052c | AQY2 | 0.00 |
| YOL020w | TAT2 | 0.00 |
| YNL192w | CHS1 | 0.00 |
| YGL084c | GUP1 | 0.00 |
| YDR264c | AKR1 | 0.00 |
| YKR106w | | 0.00 |
| YCR089w | FIG2 | 0.00 |
| YPR194c | OPT2 | 0.00 |
| YDR062w | LCB2 | 0.00 |
| YPL274w | SAM3 | 0.00 |
| YIL120w | QDR1 | 0.00 |
| YIL121w | QDR2 | 0.00 |
| YOR273c | TPO4 | 0.00 |
| YKL217w | JEN1 | 0.00 |
| YBR295w | PCA1 | 0.00 |
| YLL028w | TPO1 | 0.00 |
| YOL130w | ALR1 | 0.00 |
| YPR156c | TPO3 | 0.00 |
| YGR197c | SNG1 | 0.00 |
| YGL161c | YIP5 | 0.00 |
| YGL012w | ERG4 | 0.00 |
| YKR105c | | 0.00 |
| YPL249c | GYP5 | 0.00 |
| YDL123w | SNA4 | 0.00 |
| YNL145w | MFA2 | 0.00 |
| YBR106w | PH088 | 0.00 |
| YOL092w | | 0.00 |
| YLR353w | BUD8 | 0.00 |
| YDR508c | GNP1 | 0.00 |
| YBR043c | QDR3 | 0.00 |
| YFL055w | AGP3 | 0.00 |
| YJL117w | PHO86 | 0.00 |
| YDR039c | ENA2 | 0.00 |
| YBR132c | AGP2 | 0.00 |
| YNL065w | AQR1 | 0.00 |
| YBR180w | DTR1 | 0.00 |
| YNR072w | HXT17 | 0.00 |
| YOR348c | PUT4 | 0.00 |
| YGR289c | MAL11 | 0.00 |
| YGL198w | YIP4 | 0.00 |
| YPL036w | PMA2 | 0.00 |
| YGR227w | DIE2 | 0.00 |
| YCR010c | ADY2 | 0.00 |
| YGR217w | CCH1 | 0.00 |
| YJR092w | BUD4 | 0.00 |
| YGL010w | | 0.00 |
| YDR233c | RTN1 | 0.00 |
| YLL043w | FPS1 | 0.00 |
| YOR034c | AKR2 | 0.00 |
| YOR161c | PNS1 | 0.00 |
| YNL159c | AS12 | 0.00 |
| YMR296c | LCB1 | 0.00 |
| YCL069w | | 0.00 |
| Y-PL176c | SSP134 | 0.00 |
| YPL058c | PDR12 | 0.00 |
| YNR019w | ARE2 | 0.00 |
| YML072c | TCB3 | 0.00 |
| YOR307c | SLY41 | 0.00 |
| YDR406w | PDR15 | 0.00 |
| YBR008c | FLR1 | 0.00 |
| YKR051w | | 0.00 |
| YIL030c | SSM4 | 0.00 |
| YLR342w | FKS1 | 0.00 |
| YHL003c | LAG1 | 0.00 |
| YER181c | | 0.00 |
| YOL081w | IRA2 | 0.00 |
| YKR067w | GPT2 | 0.00 |
| YOR301w | RAX1 | 0.00 |
| YBR024w | SCO2 | 0.00 |
| YER140w | | 0.00 |
| YOR086c | TCB1 | 0.00 |
| YOL002c | IZH2 | 0.00 |
| YKR103w | NFT1 | 0.00 |
| YKL212w | SAC1 | 0.00 |
| YCR087w | | 0.00 |
| YKR088c | TVP38 | 0.00 |
| YNL260c | | 0.00 |
| YNR056c | BIO5 | 0.00 |
| YDL054c | MCH1 | 0.00 |
| YPR198w | SGE1 | 0.00 |
| YDR196c | | 0:00 |
| YNR021w | | 0.00 |
| YFL048c | EMP47 | 0.00 |
| YLR452c | SST2 | 0.00 |

In order to evaluate the secretion abilities of the secretory signal peptides at low temperature, the culture temperature for the culture solution was lowered to 15°C, and culture was further continued at 15°C for 72 hours. Thereafter, luminescence and absorbance were assayed by the luciferase activity assay technique, and the normalized activity values were also obtained.

The results are shown in Table 5. Table 5 shows the relative secretion efficiency of secretory signal peptides (indicated in terms of systematic and common names of the genes from which these secretory signal peptides have been derived) at low culture temperature (15°C) in relation to the α-factor-derived secretory signal peptide. The secretory signal peptides used in conventional secretory expression systems are shaded. The result of assaying the secretion efficiency of the α-factor-derived secretory signal peptide is shown in the column indicated as "α-factor" in terms of common names. The result of assaying the secretion efficiency of the preprotoxin-derived secretory signal peptide is shown in the column indicated as "K28L" in terms of common gene name.

### Table 5

**Table 5: Secretion efficiency of each of secretory signal peptides at low culture temperature (15° C)**

| Systematic gene name | Common gene name | Secretion efficiency relative to α-factor-derived secretory signal peptide |
|---|---|---|
| YBR243c | ALG7 | 5.76 |
| YNL237w | YTP1 | 5.67 |
| YCL043c | PD11 | 5.61 |
| YKL096w | CWP1 | 5.55 |
| YBR078w | ECM33 | 5.17 |
| YLR250w | SSP120 | 5.05 |
| YEL001c | | 4.76 |
| YMR008c | PLB1 | 4.71 |
| YNL238w | KEX2 | 4.70 |
| YLR084c | RAX2 | 4.61 |
| YGR189c | CRH1 | 4.01 |
| YLR286c | CTS1 | 3.95 |
| YMR006c | PLB2 | 3.78 |
| YKL096w-a | CWP2 | 3.70 |
| YCR028c | FEN2 | 3.64 |
| YGL126w | SCS3 | 3.54 |
| YMR200w | ROT1 | 3.46 |
| YDR304c | CPR5 | 3.44 |
| YLR110c | CCW12 | 3.33 |
| YDR518w | EUG1 | 3.21 |
| YEL040w | UTR2 | 3.16 |
| YLR332w | MID2 | 3.00 |
| YDR056c | | 2.97 |
| YJL193w | | 2.83 |
| YHR139c | SPS100 | 2.76 |
| YGR014w | MSB2 | 2.69 |
| YPL234c | TFP3 | 2.59 |
| YGL032c | AGA2 | 2.58 |
| YDR057w | YOS9 | 2.56 |
| YDR055w | PST1 | 2.54 |
| YHR110w | ERP5 | 2.50 |
| YOL011w | PLB3 | 2.48 |
| YDR134c | | 2.47 |
| YBR296c | PHO89 | 2.42 |
| YDR144c | MKC7 | 2.37 |
| YDR077w | SED1 | 2.37 |
| YDR276c | PMP3 | 2.35 |
| YNL291c | MID1 | 2.35 |
| YAL058w | CNE1 | 2.32 |
| YLR300w | EXG1 | 2.31 |
| YIL140w | AXL2 | 2.30 |
| YBR187w | | 2.26 |
| YBR070c | SAT2 | 2.19 |
| YJR118c | ILM1 | 2.16 |
| YOR190w | SPR1 | 2.14 |
| YDR519w | FPR2 | 2.13 |
| YIL090w | | 2.13 |
| YER113c | | 2.06 |
| YDR261c | EXG2 | 2.05 |
| YDR420w | HKR1 | 2.04 |
| YFL051c | | 2.03 |
| YNL300w | TOS6 | 2.02 |
| YGL038c | OCH1 | 1.96 |
| YJL078c | PRY3 | 1.95 |
| YNL219c | ALG9 | 1.88 |
| YLR034c | SMF3 | 1.86 |
| YBR229c | ROT2 | 1.84 |
| YBR093c | PHO5 | 1.81 |
| YFL041w | FET5 | 1.77 |
| YJL174w | KRE9 | 1.70 |
| YHR143w | DSE2 | 1.67 |
| YNL327w | EGT2 | 1.66 |
| YOR008c | SLG1 | 1.66 |
| YER145c | FTR1 | 1.63 |
| YNL160w | YGP1 | 1.61 |
| YLR050c | | 1.57 |
| YGL089c | MF(ALPHA)2 | 1.57 |
| YER118c | SHO1 | 1.53 |
| YAR071w | PHO11 | 1.53 |
| YCR061w | | 1.53 |
| YKL187c | | 1.51 |
| YFL026w | STE2 | 1.48 |
| YNR067c | DSE4 | 1.47 |
| YIL039w | | 1.44 |
| YDR349c | YPS7 | 1.42 |
| YKL221w | MCH2 | 1.41 |
| YIL027c | KRE27 | 1.40 |
| YCL045c | | 1.38 |
| YOL105c | WSC3 | 1.37 |
| YEL027w | CUP5 | 1.35 |
| YNR044w | AGA1 | 1.35 |
| YMR119w | ASI1 | 1.34 |
| YLR390w-a | CCW14 | 1.32 |
| YLR207w | HRD3- | 1.31 |
| YCL027w | FUS1 | 1.30 |
| YNL283c | WSC2 | 1.24 |
| YLR120c | YPS1 | 1.23 |
| YLR413w | | 1.23 |
| YDR367w | | 1.22 |
| YAL007-c | ERP2 | 1.22 |
| YKL209c | STE6 | 1.20 |
| YCR011c | ADP1 | 1.16 |
| YNL194c | | 1.14 |
| YHR181w | SVP26 | 1.14 |
| YPL130w | SPO19 | 1.14 |
| YEL004w | YEA4 | 1.13 |
| YNL190w | | 1.10 |
| YDR205w | MSC2 | 1.09 |
| YIL162w | SUC2 | 1.09 |
| YDR221w | | 1.04 |
| | α-factor | 100 |
| YJL051w | | 0.96 |
| YBR205w | KTR3 | 0.95 |
| YGL027c | CWH41 | 0.95 |
| YGR282c | BGL2 | 0.90 |
| YAL053w | | 0.86 |
| YBR092c | PHO3 | 0.85 |
| YPL189w | GUP2 | 0.85 |
| YCR017c | CWH43 | 0.81 |
| YJL222w | VTH2 | 0.80 |
| YIL015w | BAR1 | 0.79 |
| YJL159w | HSP150 | 0.78 |
| YPR124w | CTR1 | 0.76 |
| YBR042c | CST26 | 0.74 |
| YKL163w | PIR3 | 0.73 |
| YNR030w | ECM39 | 0:71 |
| YNL322c | KRE1 | 0.70 |
| YKR102w | FL010 | 0.70 |
| YLR414c | | 0.70 |
| YEL002c- | WBP1 | 0.70 |
| | K28L | |
| YAR050w | FLO1 | 0.67 |
| YCR021c | HSP30 | 0.64 |
| YAL063c | FLO9⁻ | 0.63 |
| YMR171c | | 0.63 |
| YBR210w | ERV15 | 0.63 |
| YPR091c | | 0.62 |
| YML012w | ERV25 | 0.61 |
| YLR087c | CSF1 | 0.61 |
| YDR534c | FIT1 | 0.61 |
| YFR041c | ERJ5 | 0.59 |
| YBR183w | YPC1 | 0.59 |
| YBR293w | | 0.59 |
| YJL002c | OST1 | 0.56 |
| YMR307w | GAS1 | 0.56 |
| YOR149c | SMP3 | 0.55 |
| YDL072c | YET3 | 0.54 |
| YLR121c | YPS3 | 0.53 |
| YIL147c | SLN1 | 0.51 |
| YLR214w | FRE1 | 0.51 |
| YKL051w | SFK1 | 0:50 |
| YGL020c | MDM39 | 0.48 |
| YLR155c | ASP3-1 | 0.48 |
| YDR536w | STL1 | 0.46 |
| YNL066w | SUN4 | 0.46 |
| YKL034w | TUL1 | 0.45 |
| YDL212w | SHR3 | 0.45 |
| YKL039w | PTM1 | 0.44 |
| YCR069w | CPR4 | 0.43 |
| YPL187w | MF(ALPHA)1 | 0.42 |
| YMR305c | SCW10 | 0.39 |
| YMR149w | SWP1 | 0.39 |
| YHR079c | IRE1 | 0.38 |
| YKL174c | | 0.36 |
| YGR213c | RTA1 | 0.35 |
| YML116w | ATR1 | 0.33 |
| YBR241c | | 0.33 |
| YJR004c | SAG1 | 0.33 |
| YCR034w | FEN1 | 0.32 |
| YGR279c | SCW4 | 0.32 |
| YKL220c | FRE2 | 0.32 |
| YKL164c | PIR1 | 0.32 |
| YOL013c | HRD1 | 0.31 |
| YEL036c | ANP1 | 0.31 |
| YLR037c | DAN2 | 0.31 |
| YDR245w | MNN10 | 0.31 |
| YJR150c | DAN1 | 0.29 |
| YOL019w | TOS7 | 0.28 |
| YGL028c | SCW11 | 0.27 |
| YLR459w | GAB1 | 0.26 |
| YDL093w | PMT5 | 0.26 |
| YCR044c | PER1 | 0.26 |
| YJL171c | | 0.26 |
| YBR301w | DAN3 | 0.26 |
| YNR018w | | 0.24 |
| YHR101c | BIG1 | 0.24 |
| YBR067c | TIP1 | 0.24 |
| YBR036c- | CSG2 | 0.23 |
| YNL142w | MEP2 | 0.22 |
| YMR238w | DFG5 | 0.22 |
| YHL042w | | 0.22 |
| YGL139w | | 0.21 |
| YML038c | YMDB | 0.21 |
| YJL158c | CIS3 | 0.20 |
| YGL255w | ZRT1 | 0.20 |
| YAL067c | SEO1 | 0.19 |
| YLR242c | ARV1 | 0.19 |
| YDR038c | ENA5 | 0.19 |
| YIR019c | MUC1 | 0.18 |
| YMR243c | ZRC1 | 0:18 |
| YBL017c | PEP1 | 0.18 |
| YOR011w | AUS1 | 0.17 |
| YBR199w | KTR4 | 0.17 |
| YOL030w | GAS5 | 0.16 |
| YER011w | TIR1 | 0.16 |
| YGL002w | ERP6 | 0.16 |
| YJR013w | GPI14 | 0.16 |
| YHR140w | | 0.16 |
| YMR058w | FET3 | 0:15 |
| YKL178c | STE3 | 0.15 |
| YOR085w | OST3 | 0.15 |
| YIL005w | EPS1 | 0.14 |
| YOR010c | TIR2 | 0.14 |
| YGL054c | ERV14 | 0.14 |
| YNR055c | HOL1 | 0.13 |
| YKL046c | DCW1 | 0.13 |
| YIL170w | HXT12 | 0.12 |
| YPR201w | ARR3 | 0.12 |
| YDR040c | ENA1 | 0.12 |
| YDL052c | SLC1 | 0.11 |
| YOR104w | PIN2 | 0.11 |
| YLL015w | BPT1 | 0.11 |
| YIR028w | DAL4 | 0.11 |
| YDL018c | ERP3 | 0.11 |
| YJL012c-a | | 0.10 |
| YKR040c | | 0.09 |
| YBR054w | YRO2 | 0.09 |
| YGL203c | KEX1 | 0.09 |
| YKL100c | | 0.08 |
| YHL035c | VMR1 | 0.08 |
| YJL094c | KHA1 | 0.08 |
| YBL089w | AVT5 | 0.07 |
| YIL013c | PDR11 | 0.07 |
| YHL036w | MUP3 | 0.07 |
| YLR237w | THI7 | 0.07 |
| YKR039w | GAP1 | 0.06 |
| YKL083w | | 0.06 |
| YDR072c | IPT1 | 0.06 |
| YJR040w | GEF1 | 0.06 |
| YDR384c | ATO3 | 0.06 |
| YEL043w | | 0.06 |
| YPL096c-a | ERI1 | 0.06 |
| YDR331w | GPI8 | 0.05 |
| YML123c | PHO84 | 0.05 |
| YDR033w | MRH1 | 0.05 |
| YEL065w | SIT1 | 0.05 |
| YHL016c | DUR3 | 0.05 |
| YER072w | VTC1 | 0:05 |
| YBR096w | | 0.04 |
| YDR046c | BAP3 | 0.04 |
| YKL004w | AUR1 | 0.04 |
| YBR021w | FUR4 | 0.04 |
| YDL210w | UGA4 | 0.04 |
| YLR220w | CCC1 | 0.04 |
| YBR004c | FMP44 | 0.04 |
| YKL065c | YET1 | 0.04 |
| YBR040w | FIG1 | 0.04 |
| YCL025c | AGP1 | 0.04 |
| YER053c-a | | 0.03 |
| YOR002w | ALG6 | 0.03 |
| YMR266w | RSN1 | 0.03 |
| YOL084w | PHM7 | 0.03 |
| YDR345c | HXT3 | 0.03 |
| YLL061w | MMP1 | 0.03 |
| YPL221w | BOP1 | 0.03 |
| YJR151c | DAN4 | 0.03 |
| YDR090c | | 0.03 |
| YJL062w | LAS21 | 0.03 |
| YPR138c | MEP3 | 0.03 |
| YAL018c | | 0.03 |
| YCL038c | ATG22 | 0:03 |
| YGR065c | VHT1 | 0.03 |
| YDL035c | GPR1 | 0.03 |
| YHR092c | HXT4 | 0.03 |
| YNL280c | ERG24 | 0.02 |
| YGL001c | ERG26 | 0.02 |
| YCR037c | PH087 | 0.02 |
| YMR183c | SSO2 | 0.02 |
| YER056c | FCY2 | 0.02- |
| YLR138w | NHA1 | 0.02 |
| YGR197c | SNG1 | 0.02 |
| YDR011w | SNQ2 | 0.02 |
| YOR153w | PDR5 | 0.02 |
| YMR319c | FET4 | 0.02 |
| YBR069c | TAT1 | 0.02 |
| YKR105c | | 0.02 |
| YER060w-a | FCY22 | 0.02 |
| YGR026w | | 0.02 |
| YOR016c | ERP4 | 0.02 |
| YDR032c | PST2 | 0.02 |
| YDR343c | HXT6 | 0.02 |
| YER060w | FCY21 | 0.02 |
| YDL194w | SNF3 | 0.02 |
| YGL008c | PMA1 | 0.02 |
| YDR093w | DNF2 | 0.02 |
| YPL265w | DIP5 | 0.02 |
| YNR039c | ZRG17 | 0.02 |
| YDR342c | HXT7 | 0.02 |
| YNL318c | HXT14 | 0.02 |
| YDL245c | HXT15 | 0.02 |
| YHR096c | HXT5 | 0.02 |
| YNL145w | MFA2 | 0.02 |
| YFL011w | HXT10 | 0.02 |
| YJR158w | HXT16 | 0.02 |
| YBL042c | FUI1 | 0.02 |
| YHR094c | HXT1 | 0.02 |
| YNR013c | PHO91 | 0.02 |
| YDR126w | SWF1 | 0.02 |
| YNL268w | LYP1 | 0.02 |
| YPL092w | SSU1 | 0.01 |
| YBR298c | MAL31 | 0.01 |
| YBR038w | CHS2 | 0.01 |
| YKR053c | YSR3 | 0.01 |
| YDL247w | MPH2 | 0.01 |
| YBR068c | BAP2 | 0.01 |
| YTL120w | QDR1 | 0.01 |
| YNR019w | ARE2 | 0.01 |
| YHR149c | SKG6 | 0.01 |
| YEL017c-a | PMP2 | 0.01 |
| YMR011w | HXT2 | 0.01 |
| YCR023c | | 0.01 |
| YCL069w | | 0.01 |
| YLR081w | GAL2 | 0.01 |
| YER166w. | DNF1 | 0.01 |
| YGL077c | HNM1 | 0.01 |
| YNL048w | ALG11 | 0.01 |
| YGR121c | MEP1 | 0.01 |
| YJL219w | HXT9 | 0.01 |
| YBR180w | DTR1 | 0:01 |
| YOL156w | HXT11 | 0.01 |
| YDR508c | GNP1 | 0.01 |
| YCR098c | GIT1 | 0.01 |
| YOL075c | | 0.01 |
| YPL232w | SS01 | 0.01 |
| YGR260w | TNA1 | 0.01 |
| YLL052c | AQY2 | 0.01 |
| YNR072w | HXT17 | 0.01 |
| YGR055w | MUP1 | 0.01 |
| YOL092w | | 0.01 |
| YBR294w | SUL1 | 0.01 |
| YLR023c | IZH3 | 0.01 |
| YGL012w | ERG4 | 0.01 |
| YDR062w | LCB2 | 0.01 |
| YMR296c | LCB1 | 0.01 |
| YDL138w | RGT2 | 0.01 |
| YGR191w | HIP1 | 0.01 |
| YEL031w | SPF1 | 0.01 |
| YJL214w | HXT8 | 0.01 |
| YKR050w | TRK2 | 0.01 |
| YFL017c | GNA1 | 0.01 |
| YJR152w | DAL5 | 0.01 |
| YDL199c | | 0.01 |
| YKR106w | | 0.01 |
| YCR024c-a | PMP1 | 0.01 |
| YOL122c | SMF1 | 0.01 |
| YPL036w | PMA2 | 0.01 |
| YOR328w | PDR10 | 0.01 |
| YEL069c | HXT13 | 0.01 |
| YGR032w | GSC2 | 0.01 |
| YGL198w | YIP4 | 0.01 |
| YOL103w | ITR2 | 0.01 |
| YJL117w | PHO86 | 0.01 |
| YDR039c | ENA2 | 0:01 |
| YNL087w | TCB2 | 0.01 |
| YBR043c | QDR3 | 0.01 |
| YBR106w | PHO88 | 0.01 |
| YCR048w | ARE1 | 0.01 |
| YDR497c | ITR1 | 0.01 |
| YNL270c | ALP1 | 0,01 |
| YCR089w | FIG2 | 0.01 |
| YJR160c | MPH3 | 0.01 |
| YGR138c | TPO2 | 0.01 |
| YAL022c | FUN26 | 0.01 |
| YPL249c | GYP5 | 0.01 |
| YOL158c | ENB1 | 0.01 |
| YLR353w | BUD8 | 0.01 |
| YNL192w | CHS1 | 0.01 |
| YMR274c | RCE1 | 0.01 |
| YPR194c | OPT2 | 0.01 |
| YEL063c | CAN1 | 0.01 |
| YJL129c | TRK1 | 0.01 |
| YKL217w | JEN1 | 0.01 |
| YJR092w | BUD4 | 0:01 |
| YOR161c | PNS1 | 0.01 |
| YFL050c | ALR2 | 0.01 |
| YPR156c | TPO3 | 0.00 |
| YOL020w | TAT2 | 0.00 |
| YOR273c | TPO4 | 0.00 |
| YOL130w | ALR1 | 0.00 |
| YOR034c | AKR2 | 0.00 |
| YDR264c | AKR1 | 0.00 |
| YGR227w | DIE2 | 0.00 |
| YKR093w | PTR2 | 0.00 |
| YLL043w | FPS1 | 0.00 |
| YBR295w | PCA1 | 0.00 |
| YOR348c | PUT4 | 0.00 |
| YOR307c | SLY41 | 0.00 |
| YKR051w | | 0.00 |
| YDL123w | SNA4 | 0.00 |
| YGL084c | GUP1 | 0.00 |
| YGR281w | YOR1 | 0.00 |
| YBR041w | FAT1 | 0.00 |
| YPL274w | SAM3 | 0.00 |
| YLR342w | FKS1 | 0.00 |
| YDR294c | DPL1 | 0.00 |
| YBR140c | IRA1 | 0.00 |
| YPR003c | | 0.00 |
| YGL161c | YIP5 | 0.00 |
| YGR217w | CCH1 | 0.00 |
| YBR086c | IST2 | 0.00 |
| YOR086c | TCB1 | 0.00 |
| YHL003c | LAG1 | 0.00 |
| YFL055w | AGP3 | 0.00 |
| YER008c | SEC3 | 0.00 |
| YNL159c | ASI2 | 0.00 |
| YCR010c | ADY2 | 0.00 |
| YIL121w | QDR2 | 0.00 |
| YDR461w | MFA1 | 0.00 |
| YGL010w | | 0.00 |
| YEL058c | PDR12 | 0.00 |
| YPL176c | SSP134 | 0.00 |
| YML072c | TCB3 | 0.00 |
| YIL030c | SSM4 | 0.00 |
| YNL065w | AQR1 | 0.00 |
| YDR406w | PDR15 | 0.00 |
| YKR067w | GPT2 | 0.00 |
| YKR103w | NFT1 | 0.00 |
| YBR008c | FLR1 | 0.00 |
| YER181c | | 0.00 |
| YLL028w | TP01 | 0.00 |
| YNL260c | | 0.00 |
| YKL212w | SAC1 | 0.00 |
| YNR056c | BI05 | 0.00 |
| YBR132c | AGP2 | 0.00 |
| YJL093c | TOK1 | 0.00 |
| YBR023c | CHS3 | 0.00 |
| YIL047c | SYG1 | 0.00 |
| YOR301w | RAX1 | 0.00 |
| YKR088c | TVP38 | 0.00 |
| YDL054c | MCH1 | 0.00 |
| YOL002c | IZH2 | 0:00 |
| YER140w | | 0.00 |
| YOL081w | IRA2 | 0.00 |
| YDR196c | | 0.00 |
| YDR233c | RTN1 | 0.00 |
| YBR024w | SCO2 | 0.00 |
| YCR087w | | 0.00 |
| YLR452c | SST2 | 0.00 |
| YFL048c | EMP47 | 0.00 |
| YPR198w | SGE1 | 0.00 |
| YGR289c | MAL11 | 0.00 |
| YNR021w | | 0.00 |

As shown in Table 4 and in Table 5, the secretion abilities of the secretory signal peptides at each culture temperature were evaluated by assaying the activities of the secreted luciferase secreted outside the yeast cells. As a result, -all the secretory signal peptides were found to exhibit luciferase activity in the yeast culture solution. Among the 440 types of secretory signal peptides, 9 types of secretory signal peptides and 51 types of secretory signal peptides, which would exhibit the secretory ability more than 2 times higher than that of the α-factor derived secretory signal peptide used in conventional effective secretory protein expression systems in yeast were newly identified at moderate culture temperature (30°C) and at low culture temperature (15°C), respectively. These identified secretory signal peptides included those derived from membrane proteins as well as those derived from secretory proteins.

### [Example 5] Evaluation of secretion ability of secretory signal peptide in relation to other proteins

In Example 4, the secretion ability of the secretory signal peptide was evaluated in terms of the secreted luciferase (mature CLuc). In Example 5, the secretion abilities of the 16 types of secretory signal -peptides shown in Table 6 among the secretory signal peptides shown in Table 3 were -evaluated in terms of secretory proteins, i.e., human pancreatic amylase (cDNA: SEQ ID NO: 1788; amino acid sequence: SEQ ID NO: 1789), for which a simple method for activity assay has been established, in order to inspect the efficacy of the secretory signal peptides in secretory expression of other proteins. Saccharomyces cerevisiae was used as a host.

Table 6 shows the secretory signal peptides used in Example 5 in terms of systematic and common names of the genes from which the secretory signal peptides have been derived. Table 6 also shows the nucleotide sequence of a synthetic primer (SEQ ID NO: shown in the right column) used for amplifying the gene region encoding each secretory signal peptide from the genomic DNA of Saccharomyces cerevisiae.

### Table 6

**Table 6: Secretory signal peptides used in Example 5**

| | Systematic gene name | Common gene name | Synthetic primer (Forward) | SEG ID NO: | Synthetic primer (Reverse) | SEQ ID NO: |
|---|---|---|---|---|---|---|
| 1 | YCR028c | FEN2 | ATGATGAAGGAATCGAAATCTATCAC | 1790 | gggGGTCAAATCGTTTCCGACCAT | 1791 |
| 2 | YDR420w | HKR1 | ATGGTCTCATTGAAAATAAAAAAAATTT | 1792 | gggTTGCGTTGTCGTCGTCACA | 1793 |
| 3 | YGR014w | MSB2 | ATGCAGTTTCCATTCGCTTG | 1794 | gggTGACACAGAAGTCTGGGAAGTGG | 1795 |
| 4 | YBR187w | | ATGGGAAATATGATAAAGAAGGCA | 1796 | gggAGACTTCAAATGTGAAGAACTCCCAG | 1797 |
| 5 | YBR296c | PHO89 | ATGGCTTTACATCAATTTGACTATATTT | 1798 | gggAGATCTAGAAGAGATCGACGACGC | 1799 |
| 6 | YCR061w | | ATGGTGCGTTTTGTTTCAATTT | 1800 | gggAGACCTATTCOCAGCCTGTGTA | 1801 |
| 7 | YNL237w | YTP1 | ATGACAGCAGCTAATAAGAATATTGTCT | 1802 | gggAGATTTCGACCCAGCCTGCA | 1803 |
| 8 | YBR078w | ECM33 | ATGCAATTCAAGAACGCTTTG | 1804 | gggGATTTTCTTGATAGAGTCAGCGG | 1805 |
| 9 | YLR084c | RAX2 | ATGTTTGTTCATCGTCTCTGGAC | 1806 | gggATCATCAGAGGCATCTTCCAAC | 1807 |
| 10 | YNL300w | TOS6 | ATGAAATTCTCTACTCTCTCCACCG | 1808 | gggTGTGCTGATCTCATGGGTGGT | 1809 |
| 11 | YBR243c | ALG7 | ATGTTGCGACTTTTTTCACTGG | 1810 | gggTGCTATACCAAATCCCACGG | 1811 |
| 12 | YGL126w | SCS3 | ATGTCTAGCAAATGGTTTAATGCTATAC | 1812 | gggCCCTTTCTTTACCAACATAGCATTT | 1813 |
| 13 | YMR008c | PLB1 | ATGAAGTTGCAGAGTTTGTTGGTT | 1814 | gggCTCCTTGGTGTATGCATCTCTTTT | 1815 |
| 14 | YHR139c | SPS100 | ATGAAATTCACATCAGTGCT | 1816 | gggGGAAGACGATTCGCTTTGTA | 1817 |
| 15 | YKL096w | CWP1 | ATGAAATTCTCCACTGCTTTGTC | 1818 | gggATCGCTCTCAGAACCTTCTTTG | 1819 |
| 16 | YCL043c | PDI1 | ATGAAGTTTTCTQCTGGTGCC | 1820 | gggGTCGTGCGACTGAATGTACTCATT | 1821 |

In order to introduce the gene regions encoding the aforementioned 16 types of secretory signal peptides into the pLTex321sV5H low-temperature-inducible expression vector prepared in Example 2, the gene regions were first amplified via PCR from the genomic DNA of Saccharomyces cerevisiae. The synthetic primers for the gene regions are shown in Table 6. Each reverse primer comprises at its 5' end a 3-nucleotide (GGG) sequence of the SmaI cleavage site. Inclusion of such sequence in each reverse primer results in the generation of each DNA fragment- encoding secretory signal peptides obtained by PCR described below. Introduction of the resulting DNA fragment into the SmaI-cleaved pLTex321sV5H vector results in the regeneration of the SmaI cleavage site.

PCR was carried out using 50 µl of a reaction solution comprising 300 nM of each primer, 1 mM MgSO₄, 200 µM dNTP, 1 ng of the yeast genomic DNA, and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 1 minute (elongation); and the third step at 68°C for 5 minutes. In order to add a phosphate group to the 5' ends of the DNA fragments obtained via PCR, DNA fragments were phosphorylated using 100 µl of a reaction solution comprising 1x buffer included in T4 Polynucleotide Kinase (TOYOBO), 1 mM of ATp, and 20 U of T4 Polynucleotide Kinase at 37°C for 1 hour.

Thereafter, the DNA fragments were fractionated via agarose gel electrophoresis, and DNA fragments each having a chain length of interest were obtained. Among the 16 obtained types of DNA fragments, DNA fragments encoding secretory signal peptides derived from the YGR014w gene and the YBR078w gene contained the SmaI cleavage site and the XhoI cleavage site. Thus, the other 14 types of DNA fragments were designated as DNA fragments J, and DNA fragments encoding 2 types of secretory signal peptides derived from the YGR014w gene and the YBR078w gene were designated as DNA fragments K. These DNA fragments were used to construct expression plasmids containing the genes encoding fusion proteins of secretory signal peptides with mature α-amylase (a protein consisting of an amino acid sequence derived from the amino acid sequence of human pancreatic α-amylase as shown in SEQ ID NO: 1789 by deletion of the secretory signal peptide consisting of amino acids 1 to 15) in the following manner.

The pLTex321sV5H low-temperature-inducible expression vector was cleaved with SmaI, the resulting DNA fragments were subjected to the reaction using 100 µl of a reaction solution containing 1x buffer and 2 U of E. coli Alkaline Phosphatase included in E. coli Alkaline Phosphatase (TOYOBO) at 60°C for 30 minutes, and a DNA fragment lacking a phosphate group at its 5' end was obtained. The resultant is referred to as DNA fragment L.

Subsequently, DNA fragments J were ligated to DNA fragments L using the DNA Ligation Kit ver. 2.1, and the ligation products were introduced into E. coli D-H5α. The resulting transformants were cultured overnight, plasmids were extracted using the GenElute Plasmid Miniprep Kit, and transformants having plasmids of interest (expression vectors) were identified based on the restriction enzyme cleavage patterns and via nucleotide sequence analysis. Expression vectors into which the gene regions encoding 14 types of secretory signal peptides have been introduced were prepared from these transformants. Further, the resulting expression vectors were cleaved with SmaI and with XhoI, the DNA fragments were fractionated via agarose gel electrophoresis, and the DNA fragments of interest were obtained. These fragments are designated as DNA fragments M.

The gene region encoding mature α-amylase that contains no secretory signal peptide was amplified via PCR from a plasmid containing the gene (AMY2A, cDNA: SEQ ID NO: 1788) encoding human pancreatic α-amylase.

The following synthetic primers were used. hAMY2A ORF F -Sig consists of a 25-bp region encompassing the 5' end of the gene region encoding mature α-amylase. hAMY2A ORF R +XhoI comprises at its 5' end the XhoI cleavage site, and downstream thereof, a sequence complementary to a 27-bp region including the termination cordon of the gene region encoding human pancreatic α-amylase.
hAMY2A ORF F -SIg: CAGTATTCCCCAAATACACAACAAG(SEQ ID NO: 1822)

hAMY2A ORF R +XhoI: GCGC-CTCGAG-
TTACAATTTAGATTCAGCATGAATTGC (SEQ ID NO: 1823)

PCR was carried out using 50 µl of a reaction solution comprising 300 nM of each primer, 1 mM MgSO₄, 200 µM dNTP, 1 ng of plasmid containing the gene encoding human pancreatic α-amylase (AMY2A), and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 3-0 seconds (annealing), and at 68°C for 2 minutes (elongation); and the third step at 6-8°C for 5 minutes.

The resulting PCR product was cleaved with XhoI, -the DNA fragments were fractionated via agarose gel electrophoresis, and the DNA fragment of interest (approximately 1.5 kbp) was obtained. This fragment is referred to as DNA fragment N.

Subsequently, DNA fragments M were ligated to DNA fragments N using the DNA Ligation Kit ver. 2.1, and the ligation products were introduced into E. coli DH5α. The resulting transformants were cultured overnight, plasmids were extracted using the GenElute Plasmid Miniprep Kit, and transformants having plasmids (expression vectors) containing the genes encoding the fusion proteins of the secretory signal peptides with mature α-amylase were identified based on the restriction enzyme cleavage patterns and via nucleotide sequence analysis. Expression vectors for 14 types of fusion proteins (of secretory signal peptides with mature α-amylase) were prepared from the resulting transformants.

The expression vector for a fusion protein (a fusion protein of a secretory signal peptide with mature α-amylase) was prepared using the secretory signal peptides derived from the YGR014w gene and the YBR078w gene in the following manner.

The gene region encoding mature α-amylase was amplified via PCR using synthetic primers, hAMY2A ORF F -Sig +SmaI and the aforementioned hAMY2A ORF R +XhoI (SEQ ID NO: 1823).

The -sequence of the synthetic primer used, hAMY2A ORF F -Sig +SmaI, is as shown below. hAMY2A ORF F -Sig +SmaI comprises at its 5' end a 3-nucleotide (GGG) sequence of the SmaI cleavage site, and downstream thereof, a 25-bp region encompassing the 5' end of the gene region encoding mature α-amylase.

hAMY2A ORF F -Sig +SmaI: GGG-
CAGTATTCCCCAAATACACAACAAG (SEQ ID NO: 1824)

PCR was carried out using 50 µl of a reaction solution comprising 300 nM of each primer, 1 mM MgSO₄, 200 µM dNTP, 1 ng of plasmid containing the gene encoding human pancreatic α-amylase (AMY2A), and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 2 minutes (elongation); and the third step at 68°C for 5 minutes. The resulting PCR product- was cleaved with XhoI, the DNA fragments were fractionated via agarose gel electrophoresis, and the DNA fragments of interest (approximately 1.5 kbp) were obtained. These fragments are referred to as DNA fragments O.

The pLTex321sV5H low-temperature-inducible expression vector was cleaved with SmaI and XhoI, the DNA fragments were fractionated via agarose gel electrophoresis, and the DNA fragments of interest (approximately 7.4 kbp) were obtained. These fragments are referred to as DNA fragments P.

Subsequently, DNA fragments O were ligated to DNA fragments P using the DNA Ligation Kit ver. 2.1, and the ligation products were introduced into E. coli DH5α. The resulting transformants were cultured overnight, plasmids were extracted using the GenElute Plasmid Miniprep Kit, and transformants having plasmids of interest were identified based on the restriction enzyme cleavage patterns and via nucleotide sequence analysis. Plasmids were prepared from the transformants. The resulting plasmids were cleaved with SmaI, and the DNA fragments were -subjected to a reaction using 100 µl of a reaction solution containing 1x buffer and 2 U of E. coli Alkaline Phosphatase included in E. coli Alkaline Phosphatase at 60°C for 30 minutes. Thus, a DNA fragment from which a _phosphate group at its 5' end had been removed was obtained. Such a fragment is referred to as DNA fragment Q.

Further, DNA fragments K were ligated to DNA fragments Q using the DNA Ligation Kit ver. 2.1, and the ligation products were introduced into E. coli DH5α. The resulting transformants were cultured overnight, plasmids were extracted using the GenElute Plasmid Miniprep Kit, and transformants having plasmids (expression vectors) containing the genes encoding the fusion proteins of the secretory signal- peptides with mature α-amylase were identified based on the restri-ction enzyme cleavage patterns and via nucleotide sequence analysis. Expression vectors for 2 types of fusion proteins (of secretory signal peptides with mature α-amylase) were prepared from the resulting transformants.

As the controls, expression vectors for the fusion proteins (a fusion protein of a secretory signal peptide with mature α-amylase) utilizing the α-factor-derived secretory signal peptide or preprotoxin (K28 prepro-toxin)-derived secretory signal peptide described in Example 3 were prepared in the following manner.

The gene region encoding the α-factor-derived secretory signal peptide was amplified via PCR from the pCLuRA plasmid (see International Patent Application No. PCT/JP2006/311597, claiming the priority right of JP Patent Application No. 2005-169768).

The following synthetic primers were used. MF(ALPHA) 1 Sig. 89aa F comprises a 26-bp region encompassing the 5' end of the gene region encoding the α-factor-derived secretory signal peptide. MF(ALPHA)1 Sig. 89aa R +SmaI comprises at its 5' end a 3-nucleotide (GGG) sequence of the SmaI cleavage site, and downstream thereof, a sequence complementary to a 22-bp region encompassing the 3' end of the gene region encoding the α-factor-d-erived secretory signal peptide.
MF(ALPHA)1 Sig. 89aa F: ATGAGATTTCCTTCAATTTTTACTGC (SEQ ID NO: 1825)

MF(ALPHA)1 Sig. 89aa R +SmaI: GGG-
AGCTTCAGCCTCTCTTTTCTCG (SEQ ID NO: 1826)

PCR was carried out using 50 µl of a reaction solution comprising 300 nM of each primer, 1 mM MgSO₄, 200 µM dNTP, 1 ng of the pCLuRA plasmid, and 1x buffer and KOD-Plus-1U included in the KOD-Plus-. The PCR cycle comprised: the first step at 94°C for 2 minutes; 35 cycles of the second step at 94°C for 15 seconds (denaturation), at 50°C for 30 seconds (annealing), and at 68°C for 1 minute (elongation); and the third step at 68°C for 5 minutes. The resulting DNA fragments were subjected to a reaction using 100 µl of a reaction solution comprising 1x buffer, 1 mM of ATP, and 20 U of T4 polynucleotide kinase included in T4 polynucleotide kinase at_37°C for 1 hour. Thus, a phosphate group was added to the 5' end of the DNA fragment.

Thereafter, the DNA fragments were fractionated via agarose- gel electrophoresis, and the DNA fragments of interest (270 bp) were obtained. These fragments are referred to as DNA fragments R.

Subsequently, DNA fragments R were ligated to DNA fragments L using the DNA Ligation Kit ver. 2.1, and the ligation products were introduced into E. coli DH5α. The resulting transformants were cultured overnight, plasmids were extracted using the GenElute Plasmid Miniprep Kit, and transformants having plasmids of interest (expression vectors) were identified based on the restriction enzyme cleavage patterns and via nucleotide sequence analysis. The expression vector (pLTex321sV5Hα) into which the gene region encoding the α-factor-derived secretory signal peptide had been introduced was prepared from the transformant.

The gene regions encoding mature α-amylase were introduced into the pLTex321sV5Hα expression vector comprising the gene region encoding the α-factor-derived secretory signal peptide and the pLTex321sV5H K28L expression vector comprising the gene region encoding the preprotoxin-derived secretory signal peptide prepared in Example 2 in the following manner.

These expression vectors were each cleaved with SmaI and XhoI, the DNA fragments were fractionated via agarose gel electrophoresis, and the DNA fragments of interest were obtained. The resulting DNA fragments were ligated to DNA fragments N using the DNA Ligation Kit ver. 2.1, and the ligation products were introduced into E. coli DH5α. The resulting transformants were cultured overnight, plasmids were extracted using the GenElute Plasmid Miniprep Kit, and transformants having plasmids (expression vectors) containing the genes encoding the fusion proteins- of the secretory signal peptides with mature α-amylase were identified based on the restriction enzyme cleavage patterns and via nucleotide sequence analysis. Expression vectors for 2 types of fusion proteins (of secretory signal peptides with mature α-amylase) were prepared from the resulting transformants.

Using the expression vector containing the gene encoding each of the 16 types- of fusion proteins of secretory signal peptides with mature α-amylase obtained- via the above procedure shown in Table 6 and the control vectors, i.e., an expression vector comprising a gene encoding the fusion protein of the α-factor-derived secretory signal peptide with mature α-amylase and an expression vector containing a gene encoding the fusion protein of preprotoxin (K28 prepro-toxin)-derived secretory signal peptide with mature α-amylase, the Saccharomyces cerevisiae BY4743 PEP4Δ PRB1Δ strain was transformed using the EZ yeast transformation kit (Qbiogene), and transformants each sustaining a relevant expression vector were obtained using SD+HL plate medium (0.67% of yeast nitrogen base without amino acid, 2% of glucose, 0.002% of L-histidine HCl, 0.01% of L-leucine, and 2% of agar).

In order to evaluate the levels of secretory expression of human pancreatic α-amylase in the resulting transformants, the following experiment was carried out.

One ml of SD+HL+PPB medium was inoculated with the resulting transformants, and preculture was conducted at 30°C for 3 days. Part of the culture preculture solution was used in the following culture.

Subsequently, one ml of SD+HL+PPB medium was inoculated with the culture so-lution (10 µl) obtained via the preculture and culture was conducted at 30°C to the logarithmic growth phase. Thereafter, the culture temperature for the culture solution was lowered to 10°C, and culture was further continued at 10°C for 168 hours.

After the culture had been conducted at a low temperature for 168 hours, the activity of human pancreatic α-amylase secreted in the culture solution was assayed to evaluate the secretion levels of the transformants. The human pancreatic α-amylase activity was assayed using 5 µl of the supernatant- of the culture solution and the amylase assay reagent (Diacolor Liquid AMY, Ono Pharmaceutical Co., Ltd.). Simultaneously, 200 µl of the culture solution was used to assay the absorbance thereof at 600 nm, and the activity value was- divided by the assayed value to normalize the human pancreatic α-amylase activity value based on the absorbance.

The results are shown in Table 7. Table 7 shows the secretory expression levels of human pancreatic α-amylase when using secretory signal peptides used in conventional expression systems in yeast (α-factor-derived secretory signal peptide and preprotoxin (K28 prepro-toxin)-derived secretory signal peptide) and the 16 types of secretory signal peptides found in Example 1 in terms of the secretion efficiency, in relation to the secretory expression levels resulting from the use of the α-factor-derived secretory signal peptide. In Table 7, each secretory signal peptide is indicated in terms of the systematic and common names of the genes from which the secretory signal peptides are derived. The result of the α-factor-derived secretory signal peptide is shown in the column indicated as "α-factor" in terms of common name. The result of preprotoxin-derived secretory signal peptide is shown in the column indicated as "K28L" in terms of common name.

### Table 7

**Table 7: Results of secretory expression of human pancreatic α-amylase using each of secretory signal peptides**

| | Systematic gene name | Common gene name | Secretion efficiency relative to α-factor-derived secretory signal peptide |
|---|---|---|---|
| 1 | YCR028c | FEN2 | 9.46 |
| 2 | YDR420w | HKR1 | 28.25 |
| 3 | YGR014w | MSB2 | 8.31 |
| 4 | YBR187w | | 29.98 |
| 5 | YBR296c | PHO89 | 8.08 |
| 6 | YCR061w | | 5.90 |
| 7 | YNL237w | YTP1 | 29.89 |
| 8 | YBR078w | ECM33 | 16.68 |
| 9 | YLR084c | RAX2 | 6.22 |
| 10 | YNL300w | TOS6 | 49.16 |
| 11 | YBR243c | ALG7 | 11.30 |
| 12 | YGL126w | SCS3 | 8.93 |
| 13 | YMR008c | PLB1 | 39.68 |
| 14 | YHR139c | SPS100 | 27.42 |
| 15 | YKL096w | CWP1 | 11.73 |
| 16 | YCL043c | PDI1 | 8.45 |
| 17 | | α-factor | 1.00 |
| 18 | | K28L | 0.02 |

As shown in Table 7, 16 types of secretory signal peptides were used to inspect the secretory expression of human pancreatic α-amylase. As a result, with the use of any secretory signal peptide, these secretory signal peptides were found to exhibit higher secretory expression levels than α-factor-derived secretory signal peptide and preprotoxin-derived secretory signal peptide that had been used in the highly efficient secretory protein expression in conventional expression systems in yeast.

### Effects of the Invention

The present invention provides secretory signal peptides exhibiting higher ability for transportation to cell organelles, including cell membranes, endoplasmic reticulum, and Golgi bodies, and higher efficiency of extracellular secretion, than conventional secretory signal peptides that can be used for membrane and secretory protein expression systems, for example.

All publications, patents, and patent applications cited herein are incorporated herein by reference in their entirety.

## Claims

1. DNA encoding the following secretory signal peptide (a) or (b):
(a) a secretory signal peptide consisting of the amino acid sequence as shown in any one of SEQ ID NO: 2, 4, 6, 8, 10, 12, 14, 16, and 18; or
(b) a secretory signal peptide consisting of an amino acid sequence derived from the amino acid sequence of the _secretory signal peptide (a) by deletion, substitution, or addition of one or several amino acids and- having secretory signal activity at 30°C.

2. DNA according to claim 1, wherein said DNA is any one of the following DNA (a) to (c) encoding a secretory signal peptide:
(a) DNA consisting of the nucleotide sequence as shown in any one of SEQ ID NO: 1, 3, 5, 7, 9, 11, 13, 15, and 17;
(b) DNA consisting of a nucleotide sequence derived from DNA (a) by deletion, substitution, or addition of one or several nucleotides and encoding a secretory signal peptide having secretory signal activity at 30°C; and
(c) DNA hybridizing under stringent conditions with DNA consisting of a nucleotide sequence complementary to DNA (a) and encoding a secretory signal peptide having secretory signal activity at 30°C.

3. A secretory-signal peptide encoded by DNA according to claim 1 or 2.

4. An expression vector comprising DNA according to claim 1 or 2 and a foreign gene.

5. A transformant transformed by the expression vector according to claim 4.

6. The transformant according to claim 5, wherein the host is yeast.

7. The transformant according to claim 6, wherein the yeast is Saccharomyces cerevisiae.

8. A method for producing a protein, wherein the transformant according to any one of claims 5 to 7 is cultured at 20°C to 42°C.

9. DNA encoding the following secretory signal peptide (a) or (b):
(a) a secretory signal peptide- consisting of the amino acid sequence as shown in any one of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, and 102; or
(b) a secretory signal peptide consisting of an- amino acid sequence derived from the amino acid sequence of the secretory signal peptide (a) by deletion, substitution, or addition of one or several amino acids and having secretory signal activity at 15°C.

10. DNA according to claim 9, wherein said DN-A- is any one of the following DNA (a) to (c) encoding a secretory signal peptide:
(a) DNA consisting of the nucleotide sequence as shown in any one of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, and 101;
(b) DNA consisting of a nucleotide sequence derived from DNA (a) by deletion, substitution, or addition of one or several nucleotides and encoding a secretory signal peptide having secretory signal activity at 15°C; and
(c) DNA hybridizing under stringent conditions with DNA consisting of a nucleotide sequence complementary to DNA (a) and encoding a secretory signal peptide having secretory signal activity at 15°C.

11. A secretory signal peptide encoded by DNA according to claim 9 or 10.

12. An expression vector comprising DNA according- to claim 9 or 10 and a foreign gene.

13. A transformant transformed by the expression vector according to claim 12.

14. The transformant according to claim 13, wherein the host is yeast.

15. The transformant according to claim 14, wherein the yeast is Saccharomyces cerevisiae.

16. A method for producing a protein, wherein the transformant according to any one of claims 13 to 15 is cultured at 0°C to 20°C.
